(19) Europäisches Patentamt
European Patent Office
Office européen des brevets

(11) Publication number: **0 143 081**
**B1**

(12) **EUROPEAN PATENT SPECIFICATION**

(45) Date of publication of patent specification: **23.08.89**

(51) Int. Cl.$^4$: **C 12 N 15/00**, C 12 N 9/60, C 12 N 9/64, C 12 N 1/18, C 07 H 21/04, A 61 K 37/54

(21) Application number: **84810564.9**

(22) Date of filing: **19.11.84**

(54) **Synthesis of tissue plasminogen activator(TPA) by yeast.**

(30) Priority: **21.11.83 GB 8331045**
**13.09.84 GB 8423112**

(43) Date of publication of application:
**29.05.85 Bulletin 85/22**

(45) Publication of the grant of the patent:
**23.08.89 Bulletin 89/34**

(84) Designated Contracting States:
**AT BE CH DE FR GB IT LI LU NL SE**

(56) References cited:
**EP-A-0 088 632**
**EP-A-0 093 619**
**EP-A-0 100 561**
**EP-A-0 174 835**
**EP-A-0 178 105**

**PROCEEDINGS OF THE NATIONAL ACADEMY OF SCIENCES OF THE USA, vol. 80, no. 1, January 1983, pages 1-5, Washington, US; A. MIYANOHARA et al.: "Expression of hepatitis B surface antigen gene in yeast"**

(73) Proprietor: **CIBA-GEIGY AG**
**Klybeckstrasse 141**
**CH-4002 Basel (CH)**

(72) Inventor: **Meyhack, Bernd, Dr.**
**Höhenweg 9**
**CH-4312 Magden (CH)**
Inventor: **Hinnen, Albert, Dr.**
**Offenburgerstrasse 20**
**CH-4057 Basle (CH)**

(56) References cited:

**NUCLEIC ACIDS RESEARCH, vol. 11, no. 6, 25th March 1983, pages 1657-1672, IRL Press Ltd., Oxford, GB; K. ARIMA et al.: "The nucleotide sequence of the yeast PHO5 gene: a putative precursor of repressible acid phosphatase contains a signal peptide"**

**BIOLOGICAL ABSTRACTS/RRM, abstract no. 25012848, Philadelphia, US; W.D. SCHLEUNING et al.: "Hela cell plasminogen activator isolation characterization and biosynthesis in cell-free systems and xenopus-laevis oocytes", & HAEMOSTASIS (SWITZERLAND) 1982, vol. 11, no. SUPPL. 1, p64**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 143 081 B1

(56) References cited:

**CHEMICAL ABSTRACTS, vol. 100, no. 1, January 1984, page 134, abstract no. 1512p, Columbus, Ohio, US; P.P. STEPIEN et al.: "Synthesis of a human insulin gene. VI. Expression of the synthetic proinsulin gene in yeast", & GENE 1983, 24(2-3), 289-97**

**PROCEEDINGS OF THE NATIONAL ACADEMY OF SCIENCES OF THE USA, vol. 80, no. 2, January 1983, pages 349-352, Washington, US; T. EDLUND et al.: "Isolation of cDNA sequences coding for a part of human tissue plasminogen activator"**

**EXPERIENTIA, vol. 38, no. 6, June 1982, page 745, Birkhäuser Verlag, Basel, CH; B. MEYHACK et al.: "The promoter for the regulated acid phosphatase in yeast"**

**The file contains technical information submitted after the application was filed and not included in this specification**

**Description**

Field of the invention

The invention relates to the production, via recombinant DNA technology, of tissue plasminogen activator in yeast, and to means and methods of such production. More specifically, the invention relates to yeast hybrid vectors containing a DNA sequence encoding a tissue plasminogen activator, operably linked to the PHO5 promoter, to yeast cells transformed with said hybrid vectors or containing a chromosomally integrated gene for tissue plasminogen activator, and to processes for the preparation of said hybrid vectors, said yeast cells and tissue plasminogen activator.

Background of the invention

Blood clots are the main cause of morbidity and of mortality of humans in the developed world. Blood clots are composed of fibrin which has been formed from its soluble precusor fibrinogen under the action of the enzyme thrombin. An array of enzymes and other substances ensure that clots normally form only when and where they are required to prevent loss of blood.

Mammalian plasma contains an enzymatic system capable of dissolving the fibrin in blood clots. One component of the fibrinolytic system is a group of enzymes, plasminogen activators, which convert plasminogen (an inactive proenzyme form of plasmin) to the proteolytic enzyme plasmin. Plasmin then degrades the fibrin network of the clots to form soluble products. In cases where the thrombolytic potential of the body is insufficient to remove intravascular thrombi formed, for example in patients suffering from thromboembolisms or post-surgical complications, it may be indispensable to use exogeneously administered thrombolytic agents.

Two activators of human plasminogen are commercially available for thrombolytic therapy: urokinase, a serine protease isolated from human urine or cultured kidney cells, and streptokinase, a bacterial protein obtainable from streptococci. Since neither enzyme has a specific affinity for fibrin, thrombolysis with these substances is associated with systemic activation of plasminogen which can produce indiscriminate digestion of coagulation proteins, and significantly increase the risk of internal bleeding (haemorrhage) during treatment. Furthermore, streptokinase is a protein foreign to man, and therefore stimulates the production of neutralizing antibodies which block its action, and lead to allergic reactions which are harmful and potentially fatal.

Another group of plasminogen activators, called tissue plasminogen activators (hereinafter referred to as "TPAs") are known to exist in most human tissues. TPAs originating from different tissues possibly differ from each other with respect to their molecular properties but are immunologically similar. They differ from urokinase with respect to their chemical and immunological properties, in their greatly enhanced fibrinolytic action in the presence of fibrin, as well as in their high affinity for fibrin [(1), (2)]. Due to their high affinity for fibrin, the action of TPAs is confined to the locality of the clot thereby reducing significantly the danger of uncontrolled haemorrhage.

TPA exists in two molecular forms: the active two chain form [referred to as TPA] and an inactive one-chain form [precursor TPA or "pro-TPA"; for reference, c.f. (2), (3), (4)]. Pro-TPA can be converted to active TPA by incubation with catalytic amounts of plasmin preferably in the presence of fibrin. Thus, plasmin triggers its own synthesis.

Sources of human TPA include, for example, extracts of various human tissues (which are not available for commercial exploitation) and various human tumor cells which have been shown to release TPAs to a varying extent [(5), (6)].

In a recently filed patent application (EP 41766, inventors D. Collen, D. C. Rijken and O. Matsuo) a TPA is disclosed which has a molecular weight of 72000 and has been isolated from a cultured human melanoma cell line (Bowes).

However, the use of transformed, cancerous cell lines (like melanoma cells) must necessarily limit the use of TPAs isolated therefrom as therapeutic agents. Furthermore, it has been proved to be difficult to grow mammalian cells on a large scale which is a prerequisite for the cheap and profitable manufacture of proteins secreted by these cells. The generation time of mammalian cells is considerably higher than that of microorganisms, thus requiring a prolonged fermentation period in order to obtain a sufficiently high cell density. The cell density, in turn, obtainable in the cultivation of mammalian cells is considerably lower than the cell density commonly reached in large-scale productions of microorganisms. Moreover, strain improvements are difficult to achieve as compared to microorganisms.

Thus, the production of TPA by microorganisms, making use of the well-developed technology of industrial microbiology and of the recent development of recombinant DNA technology, appears to be advantageous.

Just recently, cDNA for human tissue-type plasminogen activator derived from a human melanoma cell line, has been cloned and expressed in *Escherichia coli* [(7), (8)]. In one case (7), the expressed polypeptide is shown to have the fibrinolytic properties characteristic of authentic human TPA.

However, contaminating endotoxins are often found in protein preparations from *E. coli.* They have to be eliminated from an useful pharmaceutical product by expensive purification steps.

Since all eukaryotes share the mechanisms for the expression of genetic information, the expression of eukaryotic genes may proceed with greater efficiency in an eukaryotic host than in *E. coli.* Among the eukaryotic organisms suitable for exploitation yeast is the easiest to handle. The secretory pathway of yeast resembles that of higher animal cells and it is known that yeast cells can process proteins by cleaving the signal sequence (uncharged N-terminal part of a protein, usually split off during the secretory transport) (9). In addition it seems that proteins which enter and pass through the secretory pathway of an eukaryotic host are likely to form a high degree of tertiary structure as compared to proteins synthesized in the cytoplasm. It is interesting to note that prokaryotes such as *E. coli* do not seem to have large proteins with higher order structures. Associated with the secretory pathway is the glycosylation system. The basic steps leading to glycosylated proteins are similar in all eukaryotes and it is expected that yeast cells, contrary to prokaryotic cells like *E. coli*, can produce proteins which are glycosylated (although some final steps in the yeast glycosylation pathway differ from those in mammalian cells and might have to be modified in yeast).

Since yeast is a microorganism, yeast cells are easy to cultivate. The cell mass obtainable per volume of culture fluid is considerably higher for yeast than for *E. coli*. In addition, the fermentational behaviour of yeast is well understood and conditions for large scale fermentations are already established. Moreover, yeast cells are free of endotoxins.

Object of the invention

Several methods are known for the production of tissue plasminogen activators by means of cell culture technique or by recombinant DNA technology using *E. coli* as the host organism. The method of producing TPA by means of yeast which combines the advantage of a microorganism easily to be manipulated and to be cultivated, and the preference of an eukaroytic organism, has not been described so far. It is thus an object of the present invention to provide such a process. It is a further object of the present invention to provide yeast vector systems capable of effectively expressing an inserted TPA coding sequence.

Detailed description of the invention

1. Hybrid vectors containing a TPA coding sequence

The present invention relates to yeast hybrid vectors comprising the yeast PHO5 promoter operably linked to a DNA sequence consisting of a signal sequence linked in frame to the human tissue plasminogen activator (TPA) coding region, and a DNA sequence containing transcription termination signals of a yeast gene.

The TPA DNA which is part of the yeast hybrid vectors according to the present invention, may be derived from any human cell which is able to produce TPA. Such cells are of cancerous or non-cancerous origin and are preferably derived from an established cell line.

Examples of suitable cells include melanoma cells, such as Bowes or Malme cells, fibrosarcoma, epidermal carcinoma, bladder carcinoma, hypernephroma or liposarcoma cells, HeLa cells, fibroblasts, lymphocytes, keratocytes or breast epithelial or embryonic kidney cells. In a preferred embodiment of the present invention HeLa cells are used. The TPA DNA may be chromosomal DNA or cDNA. Chromosomal TPA DNA may be isolated from a gene library containing the TPA gene and TPA cDNA may be prepared via the mRNA route using methods known in the art. It is also possible to use fragments of the TPA coding sequence or mutated variants of the gene which may exhibit identical or changed proteolytic properties and/or activation behaviour during conversion of pro-TPA to active TPA. Some of these gene products may show new desirable properties.

The inducible PHO5 promoter can be repressed or derepressed at the will of the experimentator, solely by increasing or decreasing the concentration of inorganic phosphate in the medium. Thus, it can be repressed during the exponential growth phase of the yeast and may be turned on (derepressed) only during early stationary phase at maximal cell density allowing expression of the TPA gene.

The hybrid vectors according to the invention include a signal sequence. Suitable signal sequences are the PHO5 signal sequence or the TPA signal sequence. Other signal sequences, such as those of the yeast invertase or α-factor genes, may also be used. Alternatively, fused signal sequences may be constructed by ligating part of the PHO5 signal sequence with part of the TPA signal sequence. Those combinations are favoured which allow a precise cleavage between the signal sequence and the mature TPA amino acid sequence. Additional sequences, such as pro- or spacer-sequences which may or may not carry specific processing signals can also be included in the constructions to facilitate accurate processing of precursor molecules. Alternatively fused proteins can be generated containing internal processing signals which allow proper maturation *in vivo* or *in vitro*. Preferably, the processing signals contain a Lys-Arg residue, which is recognized by a yeast endopeptidase located in the Golgi membranes.

Upon expression of the TPA gene, the gene product enters the sectretory pathway and is transported to the periplasmic space. If further excretion through the cell wall into the culture medium can be achieved, a considerable increase in yields should be possible. Also, the recovery process can be simplified with no cells to be disrupted. Furthermore TPA can be recovered without an additional methionine at the N-terminus, because there is no need for an ATG as a translation start signal in front of the mature coding sequence. Since glycosylation is associated with the secretory pathway the produced TPA is expected to be glycosylated. There are several features which render glycosylated TPA advantageous over unglycosylated TPA: Glycosylated TPA resembles more closely the genuine TPA from mammalian cells than unglycosylated TPA does. Furthermore, the tertiary structure of proteins like TPA is probably depending to a certain degree on the presence of glycosyl residues. It is expected that carbohydrate residues present in the TPA molecules have a favourable influence on chemical stability and on pharmacological activity.

Apart from a yeast promoter and the TPA coding region, the hybrid vectors according to the invention contain additional DNA sequence(s) which are inessential or less important for the function of the promoter, i.e. for the expression of the TPA coding region, but which may perform important functions, for example, in the propagation of the yeast cells transformed with said hybrid vectors. The additional DNA sequence(s) may be derived from prokaryotic and/or eukaryotic cells and may include chromosomal and/or extrachromasomal DNA sequences. For example, the additional DNA sequences may stem from (or consist of) plasmid DNA, such as bacterial or eukaryotic plasmid DNA, viral DNA and/or chromosomal DNA, such as bacterial, yeast or higher eukaryotic chromosomal DNA. Preferred hybrid vectors contain additional DNA sequences derived from bacterial plasmids, especially *Escherichia coli* plasmid pBR322 or related plasmids, bacteriophage λ, yeast 2 μ plasmid, and/or yeast chromosomal DNA.

Preferably, the additional DNA sequences carry a yeast replication origin and a selective genetic marker for yeast. Hybrid vectors containing a yeast replication origin, e.g. a chromosomal autonomously replicating segment(ars), are extrachromosomally maintained within the yeast cell after transformation and are autonomously replicated upon mitosis. Hybrid vectors containing sequences homologous to yeast 2 μ plasmid DNA can be used as well. These hybrid vectors will get integrated by recombination into 2 μ plasmids already present within the cell or will replicate autonomously. 2 μ sequences are especially suitable for high-frequency transformation plasmids and give rise to high copy numbers.

In addition, the hybrid vectors according to the invention may include a DNA sequence as part of a gene present in the host yeast chromosome (e.g. the PHO5 gene or its promoter linked to the TPA coding region). By virtue of the homologous sequence, which should amount to a stretch of about 20 to 100 deoxynucleotides, the whole vector or linear fragments thereof can be stably incorporated into the host chromosome by recombination. Thus, during propagation the progeny cells will retain the introduced genetic material even without selective pressure. For example, the TPA gene flanked by the promoter region of a yeast PHO5 chromosomal gene at the 5'-end and (part of) the 3'-flanking region of said gene at the 3'-end, can be stably integrated at the locus of said chromosomal gene. In one preferred embodiment of the present invention, a linear DNA segment containing the PHO5 promoter, the TPA coding region and the PHO5 3'-flanking region is used to integrate the TPA gene into the respective yeast chromosome, viz. yeast chromosome II, at the PHO5 locus.

As to the selective gene marker for yeast, any marker gene can be used which facilitates the selection for transformants due to the phenotypic expression of the marker. Suitable markers for yeast are particularly those expressing antibiotic resistance or, in the case of auxotrophic yeast mutants, genes which complement host lesions. Corresponding genes confer, for example, resistance to the antibiotic cycloheximide or provide for prototrophy in an auxotrophic yeast mutant, for example the URA3, LEU2, HIS3 or TRP1 gene. It is also possible to employ as markers structural genes which are associated with an autonomously replicating segment providing that the host to be transformed is auxotrophic for the product expressed by the marker.

Advantageously, the additional DNA sequences which are present in the hybrid vectors according to the invention also include a replication origin and a selective genetic marker for a bacterial host, especially *Escherichia coli*. There are useful features which are associated with the presence of an *E. coli* replication origin and an *E. coli* marker in a yeast hybrid vector: Firstly, large amounts of hybrid vector DNA can be obtained by growth and amplification in *E. coli* and, secondly, the construction of hybrid vectors is conveniently done in *E. coli* making use of the whole repertoire of cloning technology based on *E. coli*. *E. coli* plasmids, such as pBR322 and the like, contain both *E. coli* replication origin and *E. coli* genetic markers conferring resistance to antibiotics, for example tetracycline and ampicillin, and are advantageously employed as part of the yeast hybrid vectors.

Preferably, the hybrid vectors according to the present invention comprise also the 3'-flanking sequence of a yeast gene which contains the proper signals for transcription termination and polyadenylation. Suitable 3'-flanking sequences are for example those of the yeast PHO5 gene.

The additional DNA sequence which contains, for example, replication origin and genetic markers for yeast and a bacterial host (see above) are hereinafter referred to as "vector DNA" which together with the yeast promoter and the TPA coding region is forming a hybrid vector according to the invention.

The hybrid vectors are prepared by methods known in the art, for example by introducing into a vector DNA the yeast PHO5 promoter and the TPA coding region which is controlled by said promoter.

Conveniently mapped linear or, preferably, circular vector DNA, for example bacterial plasmid DNA or the like (see above), having at least one restriction site, preferably two or more restriction sites, can be employed. Advantageously, the vector DNA already contains replication origins and gene markers for yeast and/or a bacterial host. The vector DNA is cleaved using an appropriate restriction endonuclease. The restricted DNA is ligated to the DNA fragment containing the yeast PHO5 promoter and to the DNA segment coding for TPA. Prior to or after linking of the promoter and the TPA coding region (or simultaneously as well), it is also possible to introduce replication origins and/or markers for yeast or a bacterial host. At all events, the restriction and annealing conditions are to be chosen in such a manner that there is no interference with the essential functions of the vector DNA and of the promoter. The hybrid vector may be built up sequentially or by ligating two DNA segments comprising all sequences of interest.

Various techniques may be used to join DNA segments *in vitro*. Blunt ends (fully base-paired DNA duplexes) produced by certain restriction endonucleases may be directly ligated with T4 DNA ligase. More usually, DNA segments are linked through their single-stranded cohesive ends and covalently closed by a DNA ligase, e.g. T4 DNA ligase. Such single-stranded "cohesive termini" may be formed by cleaving DNA with another class of endonucleases which produce staggered ends (the two strands of the DNA duplex are cleaved at different points at a distance of a few nucleotides). Single strands can also be formed by the addition of nucleotides to blunt ends or staggered ends using terminal transferase ("homopolymeric tailing") or by simply chewing back one strand of a blunt-ended DNA segment with a suitable exonuclease, such as λ-exonuclease. A further approach to the production of staggered ends consists in ligating to the blunt-ended DNA segment a chemically synthesized linker DNA which contains a recognition site for a staggered-end forming endonuclease and digesting the resulting DNA with the respective endonuclease.

In order to be efficiently expressed, the TPA gene must be properly located with respect to sequences containing transcriptional (yeast promoter) and translational functions (ribosome binding sites). Firstly, the ligation of the DNA segment comprising the PHO5 promoter with the TPA coding region has to be achieved in the proper orientation. If two orientations are possible the correct one is determined by conventional restriction analysis. Hybrid vectors containing an incorrectly oriented TPA gene insert are re-oriented by excising the gene insert with a suitable restriction endonuclease and re-ligating the gene with the hybrid vector fragment. In any case improper orientation is avoided by ligating two DNA segments each with different restriction sites at their ends. Furthermore, the construction of the hybrid vector should be done in such a way that it allows correct transcription initiation and termination. As to the latter point, the transcript should preferably end in a DNA sequence derived from yeast chromosomal DNA or yeast 2 μ plasmid. Advantageously, the transcipt ends in a DNA sequence containing transcription termination signals of a yeast gene, e.g. of PHO5 or TRP1. Secondly, a proper reading frame must be established. As the nucleotide sequences of the promoter region and the TPA coding region are known no problems arise in establishing the correct reading frame.

Intermediate products, such as vectors still lacking one or more essential functions, as well as the final hybrid vectors according to the invention are optionally transformed into a bacterial host, especially *E. coli*, for the above reasons (e.g. production of large amounts of intermediate products and hybrid plasmids, respectively). Bacterial vectors, such as the *E. coli* plasmid pBR322 and those fragments thereof which contain a bacterial replication origin and gene marker(s) are the most preferred vectors for that reason. When using such a bacterial vector, the final steps for the preparation of the yeast hybrid vectors preferably also include the introduction of a genetic marker and a replication origin for yeast.

2. Transformation of yeast with hybrid vectors containing a TPA coding sequence

Another aspect of the present invention involves a process for the production of transformed yeast cells capable of producing a tissue plasminogen activator, which process comprises transforming yeast cells with any of the hybrid vectors described in paragraph 1, or integrating the gene for tissue plasminogen activator into a yeast chromosome.

Useful yeasts include species of the genera *Saccharomyces, Schizosaccharomyces, Torulopsis* and related genera [cf. (11)], especially strains of *Saccharomyces cerevisiae.*

The transformation of yeast with the hybrid vectors may be accomplished by procedures known in the art, e.g. according to the method described by Hinnen et al (12). This method can be divided into three steps:

(1) Removal of the yeast cell wall.

(2) Treatment of the "naked" yeast cells (spheroplasts) with the transforming DNA in the presence of PEG (polyethyleneglycol) and $Ca^{2+}$ ions.

(3) Regeneration of the cell wall and selection of the transformed cell in a solid layer of agar.

Preferred methods:

ad (1): The yeast cell wall is removed enzymatically using various preparations of glucosidases, such as snail gut juices (e.g. Glusulase® or Helicase®) or enzyme mixtures obtained from microorganisms (e.g. Zymolyase®) in osmotically stabilized solutions (e.g. 1 M sorbitol).

ad (2): The yeast spheroplasts aggregate in the presence of PEG and local fusions of the cytoplasmic membranes are induced. The generation of "fusion-like" conditions is crucial and many transformed yeast cells become diploid or even triploid during the process of transformation. Procedures which allow selection of fused spheroplasts can be used to enrich for transformants, i.e. transformed cells can easily be screened for among preselected fusion products.

ad (3): Since yeast cells without cell wall do not divide, the cell wall has to be generated. This regeneration is conveniently done by embedding the spheroplasts into agar. For example, molten agar (about 50°C) is mixed with the spheroplasts. Upon cooling the solution to yeast growth temperatures (about 30°C), a solid layer is obtained. This agar layer is to prevent rapid diffusion and loss of essential macromolecules from the spheroplasts and thereby facilitates regeneration of the cell wall. However, cell wall regeneration may also be obtained (although at lower efficiency) by plating the spheroplasts onto the surface of preformed agar layers.

Preferably, the regeneration agar is prepared in a way to allow regeneration and selection of transformed cell at the same time. Since yeast genes coding for enzymes of amino acid biosynthetic pathways are generally used as selective markers (cf. chapter 1), the regeneration is preferably performed in yeast minimal medium agar. If very high efficiencies of regeneration are required following two step procedure is advantageous: (1) regeneration of the cell wall in a rich complex medium, and (2) selection of the transformed cells by replica plating the cell layer onto selective agar plates.

If the hybrid vector does not contain any marker gene the transformed cells can also be identified by means of alternative methods. Such methods include, for example, *in situ* hybridization with a labeled DNA fragment homologous to sequences of the hybrid vector [e.g. according to Hinnen et al. (12)], *in situ* immunoassays provided that an antibody for the product of the introduced gene is available, or other screening methods which measure gene products encoded by the transforming plasmid(s).

Alternatively, the yeast can be co-transformed with a hybrid vector according to the invention and a second vector containing a genetic marker for yeast. If the two different vectors have DNA sequences in common (these can be bacterial sequences present on the vectors), recombination takes place leading to a fused selectable hybrid molecule.

The yeast can also be cotransformed with a linear DNA segment containing the TPA gene flanked by the PHO5 promoter and PHO5 terminator sequences, and a vector containing a selective marker for yeast. Cotransformation allows enrichment for those yeast cells which have taken up DNA that cannot be directly selected for. Since competent cells take up any type of DNA a high percentage of cells transformed with a selective vector will also harbor any additional DNA (such as the linear segment containing the TPA gene with other flanking sequences (50). By virtue of sufficient long homologous sequences (e.g. about 20 to 100 deoxynucleotides in length) the TPA gene will be stably integrated into the host chromosome at the location of the PHO5 gene, viz. in yeast chromosome II.

A linear DNA segment comprising the yeast PHO5 promoter operably linked to a DNA sequence consisting of the PHO5 signal sequence linked in frame to the gene for human tissue plasminogen activator, and a DNA sequence containing transcription termination signals of the yeast PHO5 gene is also a subject of the present invention.

The obtained yeast strains containing the hybrid plasmids according to the invention can be improved in TPA production by mutation and selection using methods known in the art. The mutation can be effected, for example, by U.V. irradiation or suitable chemical reagents.

The invention also relates to a transformed yeast strain selected from the group consisting of a yeast strain transformed with a hybrid vector comprising the yeast PHO5 promoter operably linked to a DNA sequence consisting of a signal sequence linked in frame to the human tissue plasminogen activator coding region, and a DNA sequence containing transcription termination signals of a yeast gene, and a yeast strain with the gene for human tissue plasminogen activator stably integrated in the yeast chromosome and being under the control of the chromosomal yeast PHO5 promoter, and to mutants thereof.

3. Cultivation of transformed yeast cells and isolation of the expressed TPA

The primary product of TPA gene expression in yeast according to the present invention is the one-chain precursor protein of TPA ("pro-TPA"). If, however, proteases are present either in the yeast host cell, in the periplasmic space or in the culture fluid, the primarily expressed pro-TPA may at least partially be converted into two-chain TPA. The actually isolated product may therefore consist of TPA, pro-TPA or mixtures thereof. Since the TPA coding region is preceded by a signal sequence in the hybrid vectors according to the present invention, secretion is associated with, at least partial, glycosylation. The obtained product may therefore also include glycosylated and unglycosylated proteins.

The invention concerns also a method for producing TPA, pro-TPA or mixtures thereof wherein the TPA and pro-TPA is present in glycosylated or unglycosylated form or in a mixture of these forms, comprising the steps of culturing under appropriate nutrient conditions a yeast strain transformed with a hybrid vector comprising the yeast PHO5 promoter operably linked to a DNA sequence consisting of a signal sequence linked in frame to the human tissue plasminogen activator coding region, and a DNA sequence containing transcription termination signals of a yeast gene, or a yeast strain with the gene for human tissue plasminogen activator stably integrated in the yeast chromosome and being under the control of the chromosomal yeast PHO5 promoter, or a mutant of such a yeast strain, and isolating and purifying said proteins, and, if desired, separating a mixture of TPA and pro-TPA obtained into the individual components, and, for the production of TPA which is free of pro-TPA, converting the produced pro-TPA into TPA, and, if desired, separating glycosylated from unglycosylated products obtained in a mixture and, for the production of unglycosylated protein which is free of glycosylated protein, removing glycosyl residues in the obtained proteins.

a. Cultivation of transformed yeast cells

The transformed yeast strains according to the present invention are cultured in a liquid medium containing assimilable sources of carbon, nitrogen and inorganic salts.

Various carbon sources are usable. Examples of preferred carbon sources are assimilable carbohydrates, such as glucose, maltose, mannitol or lactose, or an acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate, which can be used either alone or in suitable mixtures. Inorganic salts which may be used include, for example sulphates, chlorides, phosphates and carbonates of sodium, potassium, magnesium and calcium. Additionally, the nutrient medium may also contain growth promoting substances. Substances which promote growth include, for example, trace elements, such as iron, zinc, manganese and the like, or individual amino acids.

To a varying extent, yeast cells transformed with autonomously replicating plasmids, for example, plasmids containing yeast 2 μ plasmid DNA, tend to lose the introduced hybrid plasmid [cf. (13)]. For this reason, such yeast cells have to be grown under selective conditions, i.e. conditions which require the expression of a plasmid-encoded gene for growth. Most selective markers currently in use are genes coding for enzymes of amino acid or purine biosynthesis. This makes it necessary to use synthetic minimal media deficient in the corresponding amino acid or purine base. However, some genes conferring resistance to an appropriate biocide may be used as well [e.g. genes conferring resistance to cycloheximide, to the amino-glycoside G 418 (14), to a heavy metal, or the like]. Yeast cells transformed with vectors containing antibiotic resistance genes are grown in complex media containing the corresponding antibiotic whereby faster growth rates and higher cells densities are reached.

Yeast cells transformed with DNA integrating into the chromosomes do not require selective growth conditions. These transformed cells are sufficiently stable to allow growth without selective pressure. For the above reason, these cells are advantageously grown in complex media.

Since the TPA gene is under the control of the regulated PHO5 promoter the composition of the growth medium has to be adapted in order to obtain maximum levels of mRNA transcripts, i.e. the growth medium must contain low concentration of inorganic phosphate for derepression of this promoter.

The cultivation is carried out by employing conventional techniques. The culturing conditions, such as temperature, pH of the medium and fermentation time are selected in such a way that maximal levels of TPA are produced. A chosen yeast strain is preferably grown under aerobic conditions in submerged culture with shaking or stirring at a temperature of about 25° to 35°C, preferably at about 30°C, at a pH value of from 4 to 8, for example at approximately pH 7, and for about 4 to 20 hours, preferably until maximum yields of proteins are reached.

b. Isolation and purification of the expressed TPA

After the transformed yeast cells have been grown to a satisfactory cell density, the first step for the recovery of the expressed protein consists in liberating the protein from the cell interior. In most procedures the cell wall is first removed by enzymatic digestion with glucosidases (cf. section 2). Subsequently, the resulting spheroplasts are treated with detergents, such as Triton. Alternatively, mechanical forces, such as shearing forces (for example X-press, French-press) or shaking with glass beads, are suitable for breaking cells. The resulting protein mixture is enriched for TPA by conventional means, such as removal of most of the non-proteinaceous material by treatment with polyethyleneimine, precipitation of the proteins by saturating the solution with ammonium sulphate or trichloroacetic acid, gel electrophoresis, dialysis, chromatography, for example, ion exchange chromatography size-exclusion chromatography, HPLC or reverse phase HPLC, molecular sizing on a suitable Sephadex® column, or the like. The final purification of the pre-purified product is achieved, for example, by means of affinity chromatography, for example antibody affinity chromatography, especially monoclonal antibody affinity chromatography using monoclonal anti-TPA antibodies fixed on an insoluble matrix by methods known in the art, and the like.

Further preferred methods for isolating TPA from culture fluids consist in selectively adsorbing TPA on a support of specific affinity of soluble fibrin fragments which are covalently fixed on an insoluble matrix, e.g. dextran [e.g. (15)] or, in particular, on a DE-3 Sepharose® column DE-3 is a trypsin inhibitor which is contained in the seeds of the legume Erythrima latissima (16). It has now been found that DE-3 is also able to inhibit TPA activity. Thus, the purified DE-3 inhibitor may be coupled to an insoluble matrix, e.g. BrCN activated Sepharose®, using standard procedures. TPA will be adsorbed to the inhibitor matrix and can be eluted by a buffer which contains a chaotropic agent.

In a preferred embodiment of the present invention, the culture medium, optionally after having passed through one or more of the above-mentioned purification steps, is passed at room temperature through a column consisting of BrCN activated Sepharose® to which the DE-3 inhibitor has been coupled. After washing the column, the adsorbed tissue plasminogen activator is eluted by treating the column with a buffer solution, for example phosphate buffered saline, having a pH of approximately 5.5—6.0 and containing a chaotropic agent, such as KSCN, from about 1.4 to about 2.0 molar, preferably 1.6 molar. Alternatively, benzamidine or arginine may be chosen as releasing agent. Advantageously, a detergent, especially a nonionic detergent, such as Triton X-100® or Tween 80®, is added to all buffer solutions used in the purification steps, in order to prevent the adsorption of tissue plasminogen activator to the vessel surfaces and to improve stability. The detergent may be added to a final concentration of 0.01—0.1%.

In the case where the tissue plasminogen activator is secreted by the yeast cell into the periplasmic space, a simplified protocol can be used. The protein is recovered without cell lysis by enzymatic removal of the cell wall or by treatment with chemical agents, e.g. thiol reagents or EDTA, which give rise to cell wall damages permitting the produced TPA to be released. In the case where TPA is secreted into the culture broth, it can be recovered directly therefrom.

Cultivation of yeast cells harboring a chromosomally integrated TPA gene and isolation and purification of the expressed TPA is effected in the same way as described above.

For the preparation of TPA which is substantially free of any pro-TPA, the pro-TPA is enzymatically converted into TPA, for example by the action of plasmin or an enzyme having an equivalent effect on pro-TPA.

For the preparation of pro-TPA which is substantially free of TPA, a protease inhibitor, such as aprotinin (Trasylol®) or basic pancreatic trypsin inhibitor, is advantageously included during the purification procedure in order to inhibit traces of proteases which may be present and which may cause (partial) conversion of pro-TPA into TPA. The final purification is then achieved by chromatography on a column containing a selective affinity reagent, such as DE-3, in the presence of an inhibitor which selectively binds only TPA and not pro-TPA, such as, for example, diisopropylfluorophosphate (DFP) or nitrophenyl guanidinobenzoate. These reagents prevent TPA from adsorbing to the affinity column. Consequently, the TPA will pass through the DE-3 column whereas pro-TPA will adsorb to the column and can be eluted as described above.

A mixture of glycosylated and unglycosylated proteins may be separated, for example, by chromatography on a concanavalin-A Sepharose® column. Unglycosylated products will pass through the column whereas glycosylated products will selectively adsorb and can be eluted by conventional means, e.g. α-methylmannoside in combination with a chaotropic agent, such as KSCN.

It is also possible to remove glycosyl residues enzymatically, e.g. by the action of endoglycosidase H. This method permits the production of unglycosylated products in substantially pure form.

The TPAs and pro-TPAs obtainable according to the present invention, exhibit valuable pharmacological properties. Thus, these proteins can be used in analogy to known plasminogen activators in humans for the prevention or treatment of thrombosis or other conditions where it is desired to produce local fibrinolytic or proteolytic activity via the mechanism of plasminogen activation, such as arteriosclerosis, myocardial and cerebral infarction, venous thrombosis, thromboembolism, post-surgical thrombosis, thrombophlebitis and diabetic vasculopathies.

The invention concerns especially the hybrid vectors, the transformed yeast strains and the processes for their preparation as well as the method for producing a TPA, a pro-TPA or mixtures thereof as described in the Examples.

Brief description of the drawings

In the following experimental part various embodiments of the present invention are described with reference to the accompanying drawings in which:

Figure 1 is a partial restriction endonuclease map of the plasmids pJDB207/PHO5, PHO3 and pBR322/PHO5Bam-Sal used as sources of the PHO5 gene or for DNA sequencing, respectively.

Figure 2 shows the localization of the PHO5 and the PHO3 acid phosphatase genes within a 5.1 kb BamHI fragment isolated from a yeast gene library.

Figures 3a and 3b provide the DNA sequences of the promoter region of PHO5 and PHO3, respectively.

Figure 4 is a schematic diagram showing the construction of the hybrid plasmid p30.

Figure 5 is a schematic outline of the construction of plasmid p31 containing a PHO5 termination fragment.

Figure 6 shows the nucleotide sequence of the Sau3A-PstI PHO5 transcription termination fragment.

Figure 7 displays a schematic outline of the process for deleting the PHO5 signal sequence in expression plasmid p31 and specifically shows the construction of plasmid p31/R.

Figure 8 schematically shows the collection of clones obtained in the process outlined in Figure 7.

Figures 9 and 10 display the nucleotide sequences of the BamHI-EcoRI restriction fragments containing the PHO5/R and PHO5/Y promoter regions.

Figure 11 provides the DNA and the corresponding amino acid sequences of the TPA cDNA clone isolated from HeLa cells.

Figure 12 is a schematic outline of the construction of the cloning vehicle M13mp9/PHO5-TPA.

Figure 13 depicts the construction of the plasmid pJDB207/PHO5-TPA (1A), including the PHO5 signal sequence.

Figure 14 schematically illustrates the construction of the plasmid pJDB207/PHO5-TPAΔ containing a shortened PHO5 transcription termination fragment.

Figure 15 shows the sequence of the shortened PHO5 transcription termination fragment.

Figure 16 shows the construction of the hybrid plasmid p31RL/TPA (12-2), a construction without signal sequence. The coding sequence of mature TPA is ligated to the PHO5 promoter.

Figure 17 is a schematic diagram showing the construction of the plasmid pJDB207R/PHO5-TPA (12-2).

Figure 18 depicts the construction of the plasmid p31/PHO5-TPA18.

Figure 19 shows the junction between the PHO5 signal sequence and the coding region of mature TPA in the plasmid p31/PHO5-TPA18.

Figure 20 is a schematic outline of the isolation of DNA fragments containing parts of the prepro-TPA coding region.

Figure 21 displays the construction of the plasmid P31R/SS-TPAΔ2.

Figure 22 shows the construction of the intermediate plasmid pBR322/PHO5Bam-Rsa 637.

Figure 23 shows the construction of plasmids with the PHO5 signal sequence extended into the PHO5 coding region.

Figure 24 is a schematic outline showing the linkage of the PHO5 promoter and part of the PHO5 coding sequence to the coding sequence of mature TPA via linker A, B or C.

Figure 25 is a schematic outline of the chromosomal replacement of the PHO5 gene by the TPA gene.

The following Examples serve to illustrate the present invention but should not be construed as a limitation thereof.

Experimental part

The following abbreviations are used in the Examples:

| | |
|---|---|
| BSA: | bovine serum albumin |
| DTT: | 1,4-dithiothreitol (1,4-dimercapto-2,3-butanediol) |
| EDTA: | ethylenediaminetetracetic acid |
| SDS: | sodium dodecyl sulphate |
| TNE: | solution containing 100 mM NaCl, 10 mM Tris · HCl (pH 7.5), and 1 mM EDTA. |
| Tris · HCl | tris-(hydroxymethyl)-aminomethane, pH adjusted with HCl |
| TE: | solution containing 10 mM Tris · HCl (pH 7.5) and 1 mM EDTA |
| MOPS: | 3-morpholine-propane-1-sulfonic acid |

Example 1

Construction of a yeast gene library

Thirty μg of total high molecular weight yeast DNA (17) from wild type *Saccharomyces cerevisiae* strain S288C is incubated for 30 min at 37°C with 2 units of EcoRI methylase (New England Biolabs) in 250 μl of EcoRI methylation buffer as recommended by the supplier. DNA is precipitated by ethanol, resuspended in 500 μl of 25 mM Tris · HCl pH 8.5, 2 mM $MgCl_2$ (EcoRI* buffer) (18) and digested with EcoRI (Boehringer) until the size distribution of the DNA fragments has a maximum in the 30—50 kb range (a XhoI digest of λDNA provides appropriate 33 kb and 17 kb markers). The yeast DNA digested under EcoRI* conditions is size-fractionated on a sucrose gradient (5—20% sucrose in 10 mM Tris · HCl pH 7.5, 1 mM EDTA) for 6 hrs. at 38,000 rpm in a SW 40 rotor. Thirty fractions of 0.4 ml each are collected from the top of the gradient. Fraction 16 contains DNA fragments of 30—40 kb in size. The DNA of this fraction (3 μg) is precipitated with ethanol and ligated for 16 hours at 15°C in a total volume of 15 μl to 1 μg of cosmid vector pYcl (19), linearized by EcoRI. Ligation is carried out with 300 U T4 DNA ligase (New England Biolabs) using the buffer system described by the supplier. The DNA is packaged *in vitro* into bacteriophage λ (20) and the assembled phages are used to transduce *E. coli* strain HB101 ($r_K^-$, $m_K^-$, $leu^-$, $pro^-$, $recA^-$). The efficiency of transduction is about 5000 ampicillin-resistant colonies per μg of pYcl vector. 3000 $amp^R$ colonies are picked and grown individually in the wells of microtiter dishes in LB medium [10 g Bacto-Tyrptone (Difco), 5 g Bacto Yeast Extract (Difco), 10 g NaCl] containing 100 μg/ml ampicillin.

Example 2

Isolation of the regulated acid phosphatase gene PHO5

Replicas of the gene library are grown on LB agar plates (LB medium plus 15 g/l agar) containing 100 μg/ml ampicillin. The cell material from 500 colonies is washed off the plates and pooled. DNA is isolated from individual pools using the following protocol:

The cells are harvested by centrifugation (Sorval, GSA rotor, 10 min at 6000 rpm, 4°C), resuspended in 100 ml TE (10 mM Tris · HCl, 1 mM EDTA, pH 8.0) and centrifuged again under the above conditions. The cell pellet is resuspended in 3 ml Tsuc [50 mM Tris · HCl, pH 7.5, 25% (w/v) sucrose] and transferred to SS-34 polypropylene Sorval tubes. All subsequent steps are carried out on ice: 0.3 ml of lysozyme solution (10 mg/ml, purchased from Worthington, 11,000 U/mg) is added, after 5 min 1.2 ml EDTA (500 mM, pH 8.0), and after another 5 min 4.8 ml detergent [0.1% Triton X-100 (Merck), 50 mM EDTA, 50 mM Tris · HCl, pH 8.0] are added. After 5 min the lysat is centrifuged in a precooled SS-34 rotor for 40 min at 4°C. The supernatant is carefully removed and solid CsCl is added (8.3 g CsCl to 8.7 ml of supernatant). After the addition of ethidium bromide (Sigma) (final concentration 1 mg/ml supernatant) the solution is transferred to 13.5 ml Quick Seal polyallomer tubes (Beckman) and centrifuged in a Beckman Ti50 rotor for 40 hrs at 40,000 rpm. Two fluorescent bands can be visualized with long wave UV (366 nm). The lower band contains supercoiled plasmid DNA which is collected by puncturing the tube from the side with a 2 ml syringe (18G needle). The ethidium bromide is removed by extracting 5 times with equal volumes of isopropanol (saturated with CsCl) and the product is transferred to 30 ml Corex tubes. 2.5 volumes of TE is added and the DNA is precipitated with ethanol. The solution is then kept for 12—15 hrs at −20°C. The precipitated DNA is collected by centrifugation in a Sorvall HB-4 rotor for 30 min at 12,000 rpm at 0°C and redissolved in 200 μl of TE. 50—100 μg of hybrid plasmid DNA are recovered from a 100 ml culture.

Plasmid DNA from these pools is used to transform *S. cerevisiae* strain AH216 (a, his3, leu2, pho3, pho5) according to the procedure described by Hinnen et al. (12). Yeast transformants are replicated on low $P_i$-minimal medium [as "Difco yeast minimal medium without aminoacids" supplemented with 20 g/l glucose, but prepared from the components according to the recipe of Difco (Difco Manual, Difco Laboratories, Detroit, USA) except that 0.03 g/l $KH_2PO_4$ plus 1 g/l KCl is used instead of 1 g/l $KH_2PO_4$] and stained for acid phosphatase activity by overlayering them with staining agar [1% Difco agar in 100 mM acetate buffer pH 4.0, 2 mg/ml Fast Blue B Salt (Serva) and 0.2 mg/ml α-naphthyl phosphate (Serva)]. Colonies with a functional PHO5 gene stain red upon derepression of the gene on low $P_i^-$ medium. By repeated subpooling (21) of the gene library 3 independent clones exhibiting repressible acid phosphatase activity are obtained.

One of these clones (pG7) is further analysed. The hybrid plasmid has a size of 42 kb. EcoRI and BamHI fragments of pG7 are subcloned in pBR322/HIS3 (13) and pJDB207 (22) respectively. Restriction digests are as recommended by the supplier (New England Biolabs) and ligations are performed in 20 μl with 150 U T4 DNA ligase (New England Biolabs) and 20 μg/ml of the individual digested plasmids (conditions as suggested by New England Biolabs). A 5.1 kb BamHI fragment which is part of a 8 kb EcoRI fragment is subcloned in yeast vector pJDB207 and, upon transformation of yeast strain AH216, this hybrid plasmid (pJDB207/PHO5, PHO3, see Figure 1) elicits high phosphatase activity under derepressed (low $P_i^-$) conditions (PHO5 gene) and low levels of activity in normal yeast minimal medium (expression of the PHO3 gene).

Example 3
Localisation of the PHO5 and PHO3 genes and DNA sequences analysis
a. The PHO5 gene

For the localisation of PHO3 and PHO5 within the BamHI fragment advantage is taken of the pattern of Sau3A restriction sites and a unique PstI site. Digestion of the BamHI fragment with restriction endonuclease Sau3A (New England Biolabs) generates 6 fragments (A—F, Figure 2). Subcloning of a partial Sau3A digest into the BamHI site of self-replicating yeast vector pJDB207 leads to plasmids with different combinations of Sau3A fragments. These plasmids are then used to transform the pho3, pho5 mutant yeast *S. cerevisiae* AH216. Transformants are checked for acid phosphatase activity after growth on either low $P_i^-$ or normal minimal medium plates. Clones containing at least Sau3A fragments A and B (Figure 2, No. 1—4) express acid phosphatase at the same level (qualitative estimates after overlayering with acid phosphatase staining agar, as described in Example 2) as the entire 5.1 kb BamHI fragment. Expression is regulated normally by the concentration of inorganic phosphate in the medium. Clones with Sau3A-fragment A only (Figure 2, No. 5, 6) express low levels of acid phosphatase, which is not influenced by the inorganic phosphate concentration in the medium. This indicates that information carried by the Sau3A fragment A is sufficient for constitutive acid phosphatase (PHO3) expression. Sau3A fragment B (Figure 2, No. 7) alone does not lead to any expression of acid phosphatase under either repressed or derepressed conditions. However, a subclone with the complete sequence between the BamHI and PstI sites (Figure 2, No. 10) shows regulated, but not constutitive synthesis of acid phosphatase. This subclone must therefore contain the yeast PHO5 gene (13).

The exact localisation of the PHO5 gene is determined by DNA sequencing using the method of Maxam and Gilbert (10). A 623 bp BamHI-SalI restriction fragment is cloned into plasmid pBR322 (See Figure 1), replacing the BamHI-SalI fragment which extends from position 375 to 650 (pBR322 nomenclature), using digestion and ligation conditions as described above (all enzymes are from New England Biolabs). DNA fragments of the BamHI-SalI DNA insert are asymmetrically labelled at their 5′ ends at the following sites: BamHI (−541), Sau3A (−200) and SalI (+82), (for numbering see Figure 3a). The nucleotide sequence of the 623 bp BamHI-SalI DNA insert is depicted in Figure 3a. It reveals that the insert contains the PHO5 promoter region and part of the PHO5 phosphatase protein coding region.

b. The PHO3 gene

The exact localization of the PHO3 gene is determined by DNA sequence analysis according to the manual "M13 cloning and DNA sequencing system" published by New England Biolabs. A 415 bp (5′)PstI-RsaI(3′) fragment is subcloned in vectors M13mp8 and M13mp9 (23), using unique PstI and SmaI restriction sites. The nucleotide sequence of the 416 bp PstI-RsaI DNA insert is shown in Figure 3b. It reveals that the insert contains the PHO3 promoter region and part of the PHO3 acid phosphatase protein coding sequence.

Example 4
Construction of plasmid p30 (see Figure 4)
a) Elimination of the BalI restriction site in plasmid pBR322

3 μg of pBR322 are digested to completion with restriction endonucleases BalI (BRL) and PvuII (biolabs) according to the recommendations of the suppliers. The BalI/PvuII double digest of pBR322 results in two restriction fragments of 3738 bp and 622 bp in size. The two fragments are separated on a 1% low melting agarose gel (Sigma) in TBE (90 mM Tris · HCl pH 8.3, 2.5 mM EDTA, 90 mM boric acid) buffer. The DNA bands are stained with ethidiumbromide and visualized under long wave UV light at 366 nm. The piece of agarose containing the 3738 bp fragment is cut out from the gel, liquified at 65°C, adjusted to 500 mM NaCl' and incuabted at 65°C for 20 min. One volume of phenol (equilibrated with 10 mM Tris · HCl pH 7.5, 1 mM EDTA, 500 mM NaCl) is added. The aqueous phase is reextracted twice with phenol and once with chloroform. The DNA is precipitated with 2.5 volumes of cold absolute ethanol and collected by centrifugation. The DNA pellet is washed with cold 80% ethanol and then dried in vacuum. The DNA is resuspended in TE at a concentration of 0.15 mg/ml.

The isolated 3738 bp DNA fragment has two blunt ends resulting from the BalI and PvuII double digests. The DNA is circularized by blunt end ligation. 0.6 µg of DNA are incubated over night at room temperature in 30 µl of 60 mM Tris · HCl pH 7.5, 10 mM $MgCl_2$, 10 mM DTT, 4 mM ATP, and 900 U of T4 DNA ligase (Biolabs). 5 µl aliquots of the ligation mixture are added to 50 µl of calcium treated, transformation competent *E. coli* HB101 cells, prepared by the method of Mandel et al. (24). The mixture is kept on ice for 5 min, then incubated for 2 min at 37°C and left 10 min at room temperature before plating on LB agar plates containing 100 µg/ml of ampicillin. Six $amp^R$ colonies are picked and grown individually in 100 ml of LB (as above but without agar) medium containing 100 µg/ml ampicillin. Plasmid DNA is prepared from the cells using the procedure described in Example 2. Restriction digests with HaeIII (purchased from Biolabs, digestion conditions as suggested by supplier). PvuII and BalI of the plasmids are analyzed on a 1.5% agarose gel in TBE buffer. The restriction pattern and the predicted size of the newly formed junction fragment indicates that the plasmids are identical and contain all of the pBR322 sequences except for the BalI—PvuII fragment. These plasmids lack the BalI restriction site and are referred to as pBR322ΔBalI.

b) Cloning of a yeast 5.1 kb BamHI restriction fragment containing PHO5 and PHO3 into pBR322ΔBalI

pJDB207/PHO5, PHO3 (see Figure 1) contains a yeast 5.1 BamHI insert with the genes for regulated and constitutive yeast acid phosphatase (PHO5 and PHO3). pJDB207/PHO5, PHO3 as well as plasmid pBR322ΔBalI are digested with restriction endonuclease BamHI. After complete digestion the enzyme is inactivated for 2 min at 65°. Both DNAs are precipitated by ethanol and resuspended in 10 mM Tris · HCl pH 8.0 at a concentration of 0.2 mg/ml each. 0.5 µg each of the two BamHI-digested DNAs are combined and ligated in 20 µl of ligation buffer (as suggested by New England Biolabs), containing 300 U of T4 DNA ligase, for 20 hrs. at 15°C. 5 µl aliquots of the ligation mixture are added to 50 µl of calcium-treated *E. coli* cells and transformation is carried out as described in Example 4a. The transformed *E. coli* cells are tested for their resistance towards ampicillin and tetracyclin. Eight $amp^R$, $tet^S$ colonies are isolated and grown in 100 ml of LB medium containing 100 µg/ml of ampicillin. Plasmid DNA is isolated from the cells (see Example 2). Restriction digests with BamHI show that 4 plasmids contain a 5.1 kb insert besides the 3.7 kb vector fragment (pBR322ΔBalI). Restriction digests with SalI (New England Biolabs) determine the orientation of the inserted 5.1 kb fragment: two plasmids have the insert oriented as shown in Figure 4. One of them is referred to as p30. The direction of transcription of the PHO5, PHO3 genes in the 5.1 kb insert is anticlockwise as indicated in Figure 4.

Example 5

Construction of an expression plasmid containing the PHO5 promoter and PHO5 transcription termination signals (see Figure 5).

a) Elimination of the EcoRI restriction site in plasmid p30

Five µg of p30 DNA (cf. Example 4) are digested to completion with restriction endonuclease EcoRI (Boehringer). In order to fill in the resulting sticky ends, 1 µg of EcoRI digested p30 in 50 µl of 50 mM NaCl, 10 mM Tris · HCl pH 7.5, 10 mM $MgCl_2$, 1 mM DTT, 0.25 mM dATP and 0.25 mM dTTP is incubated for 30 min 37°C with 1 unit of DNA polymerase (Klenow large fragment, BRL). The DNA recovered from ethanol precipitation is ligated as usual and used for transformation of competent *E. coli* HB101 cells as described in Example 4. Clones that are resistant to EcoRI digest are referred to as p30 ($EcoRI^R$).

b) Isolation of a 0.37 kb Sau3A-PstI PHO5 transcription termination fragment:

The PHO5 transcript has been mapped by S1 nuclease mapping (25). The signals for transcription termination have been shown to be located in a 0.37 kb Sau3A-PstI fragment of the PHO5 gene. The nucleotide sequence of the Sau3A-PstI fragment is given in Figure 6.

Five µg of pJDB207/PHO5, PHO3 DNA (cf. Example 2) are digested to completion with restriction endonucleases Sau3A and PstI. The restriction fragments are separated on a vertical 1.5% low melting agarose gel in TBE buffer. The 0.37 kb Sau3A-PstI fragment is localized by ethidiumbromide staining and a gel block as small as possible is cut out containing this DNA fragment.

c) Cloning of the Sau3A-PstI PHO5 fragment in M13mp9:

M13mp9 phage DNA is a useful cloning vector with a cluster of unique restriction sites (23). Five µg of M13mp9 DNA are digested to completion with restriction endonucleases BamHI and PstI. The larger 7.1 kb DNA fragment is separated from a very small fragment (8 bp) on a 0.8% low melting agarose gel. The gel block containing the large DNA fragment is cut out of the gel. Gel blocks with the 0.37 kb Sau3A-PstI fragment of pJDB207/PHO5, PHO3 (cf. Example 5b) and the 7.2 kb BamHI-PstI fragment of M13mp9 are liquefied at 65°C, mixed in about equimolar amounts and diluted with $H_2O$ to lower the agarose concentration to 0.3%. Ligation is carried out in a 200 µl solution containing 60 mM Tris · HCl pH 7.5, 10 mM

$MgCl_2$ 10 mM DTT, 1 mM ATP and 600 units of T4 DNA ligase (Biolabs). Transduction of competent cells of the strain *E. coli* JM101 (Ca++) is done according to the manual "M13 cloning and DNA sequencing system" published by New England Biolabs. Phages from a number of white plaques are grown and analyzed for the size of their DNA insert by cleavage with restriction endonucleases EcoRI and PstI.

A M13mp9 derived clone containing the Sau3A-PstI PHO5 transcription termination fragment is isolated and referred to as M13mp9/PHO5 (Sau3A-PstI).

d) Cloning of the PHO5 transcription termination fragment in P30(EcoRI$^R$)

The original PHO5 transcription termination fragment cloned in phage M13mp9 (M13mp9/PHO5 (Sau3A-PstI)) is recloned as a HaeIII-HindIII fragment in plasmid p30(EcoRI$^R$) cleaved with BalI and HindIII: M13mp9/PHO5 (Sau3A-PstI) DNA is cleaved to completion with restriction endonucleases HaeIII and HindIII. The resulting two DNA fragments are separated on a 1.5% vertical low melting agarose gel in TBE buffer. The 0.39 kb fragment is isolated in a gel block cut out of the gel. P30 (EcoRI$^R$) DNA is digested with BalI and HindIII. The large 3.98 kb fragment is separated on a 0.8% low melting agarose gel in TBE buffer and isolated by cutting a gel block containing the DNA fragment.

Gel blocks with the 0.39 kb HaeIII-HindIII PHO5 transcription termination fragment and the 3.98 kb BalI-HindIII fragment of p30(EcoRI$^R$) are melted at 65°C and mixed in about equimolar amounts. Ligation and transformation of competent *E. coli* HB101 cells are as described in Example 4. DNA of transformed cells is analyzed and referred to as p31 (see Figure 5).

Expression plasmid p31 contains the PHO5 promoter region with part of the signal sequence of PHO5 and adjacent to it a DNA fragment with the PHO5 transcription termination signals. Foreign coding sequences to be expressed in this vector may conveniently be inserted between promoter and transcription termination sequences.

Example 6

Deletion of the PHO5 signal sequence in the expression plasmid p31 (see Figure 7)

Expression plasmid p31 contains the PHO5 promoter sequence including the mRNA start sites, the translation start codon ATG of acid phosphatase and addition 40 nucleotides coding for part of the acid phosphatase signal sequence. In this construction, the nucleotides for the signal sequence and the ATG are eliminated by Bal31 digestion. EcoRI linkers are introduced to allow joining of the PHO5 promoter to mature TPA coding sequence.

a) Bal31 digestion of BalI cleaved plasmid p30

20 µg of p30 DNA (see Example 4b) are digested with restriction endonuclease BalI, resulting in 2 fragments of 3.7 and 5.1 kb. After extraction with phenol/chloroform, the DNA is precipitated with ethanol. The DNA is resuspended in 10 mM Tris pH 8.0 at a concentration of 0.5 µg/ml. 9 µg of BalI cleaved p30 DNA are digested with 2 U of exonuclease Bal31 (BRL) in 100 µl of 20 mM Tris pH 8.0, 199 mM NaCl, 12 mM $MgCl_2$, 12 mM $CaCl_2$ and 1 mM EDTA. Aliquots of 2 µg DNA each are withdrawn after 15 sec., 30 sec., 45 sec., and 60 sec. of incubation at 30°C and are immediately mixed with 50 µl phenol and 60 µl TNE. After extraction with phenol/chloroform and ethanol precipitation, the DNA is resuspended in 10 mM Tris pH 8.0 at a concentration of 100 µg/ml. To analyse the extent of exonucleolytic cleavage by Bal31 0.5 µg of DNA from each time point are digested with endonuclease BamHI and analysed on a 1.5% agarose gel in Tris-borate buffer pH 8.3. On the average 70 bp are removed from each end of the fragment after 45 sec. of Bal31 digestion. For further experiments DNA from the 45 second time point is used.

b) Addition of EcoRI linkers to the Bal31 treated DNA

Two $A_{260}$ units of EcoRI linkers (5'-GGAATTCC-3', BRL) are resuspended in 250 µl of 10 mM Tris pH 8, 1 mM EDTA. Two µg of EcoRI linkers are kinased in 75 µl of 60 mM Tris pH 7.5, 10 mM $MgCl_2$, 15 mM DDT, 10 µM ATP and 33 U of T4 polynucleotide kinase (Boehringer). After 1 h at 37°C the mixture is allowed to cool to room temperature and is then stored at −20°C.

The annealed, double stranded EcoRI linkers are ligated with their blunt ends to the Bal31 treated DNA fragments. Half a microgram of Bal31 treated DNA (see Example 6a) is incubated for 16 hours at room temperature with a 50 fold excess of kinased EcoRI linkers in 20 µl of 60 mM Tris pH 7.5, 10 mM $MgCl_2$, 10 mM DTT, 4 mM ATP and 600 U of T4 DNA ligase (Biolabs). After inactivation of the T4 DNA ligase (10 min at 65°C) the excess of EcoRI linkers is cleaved by 50 U of EcoRI (Boehringer) in a volume of 50 µl. The DNA is extracted with phenol/chloroform, precipitated by ethanol and resuspended in 10 mM Tris, 1 mM EDTA.

Restriction endonuclease EcoRI not only cleaves the terminally added EcoRI linkers of both BalI fragments (3.7 kb and 5.1 kb) but also at an internal EcoRI site in the 5.1 kb fragment giving rise to a 3.9 kb and a 1.2 kb fragment. The 3.7 kb and 3.9 kb fragments are separated from the 1.2 kb fragment on a 0.8% low melting agarose gel (Sigma) in 90 mM Tris-HCl pH 8.3, 90 mM boric acid and 2.5 mM EDTA. The DNA bands are stained with ethidium bromide and visualized under long wave UV light at 366 nm. The two large DNA fragments of 3.7 kb and 3.9 kb are not separated. They are cut out of the gel in a single gel block and are extracted as described in Example 4a.

The linear fragments terminating in EcoRI sticky ends are circularized by ligation. About 0.25 µg of fragments are ligated in 100 µl of 60 mM Tris pH 7.5, 10 mM $MgCl_2$, 10 mM DDT, 1 mM ATP and 600 U T4 DNA ligase for 4 hours at 15°C.

Ten µl aliquots of the ligation mixture are added to 100 µl of calcium treated, transformation competent *E. coli* HB101 cells (see Example 4a). 35 transformed, $amp^R$ colonies are grown individually in LB medium containing 100 µg/ml of ampicillin. Plasmid DNA is prepared according to the method of Holmes *et al.* (26) and is analysed by EcoRI/BamHI double digestion.

c) Nucleotide sequence analysis to determine the position of the EcoRI linker addition

Most of the 35 clones will differ from each other in the position of EcoRI linker addition in the PHO5 promoter region depending on the degree of Bal31 digestion of the individual DNA molecules. For nucleotide sequence analysis plasmid DNA is digested with EcoRI.

After extraction with phenol/chloroform the restricted DNA is precipitated with ethanol. The DNA is dephosphorylated and 5'-terminally labeled. The labeled DNA fragments are cleaved with a second restriction endonuclease, BamHI. The products are separated on a 8.0% low melting agarose gel. The 0.5—0.6 kb 5'-labeled EcoRI-BamHI fragment is isolated from low melting agarose as described in the Example 4a. For the determination of the nucleotide sequence adjacent to the EcoRI linker the different DNA fragments are chemically degraded and the products are separated by polyacrylamide gel electrophoresis as described by Maxam and Gilbert (10).

The different clones and the position of the corresponding last nucleotide of the PHO5 sequence (then followed by an EcoRI linker) is listed in Table 1 (see also Figure 8).

TABLE 1

| Clone | Position of last nucleotide of the PHO5 sequence |
|---|---|
| pE | +25 |
| pG | +16 |
| pe | +15 |
| pd | +12 |
| pY | −4 |
| pR | −10 |
| pP | −16 |
| pV | −18 |
| pL | −21 |
| pN | −22 |
| pC | −24 |
| pH | −27 |
| pS | −28 |
| pk | −29 |
| pl | −38 |
| pM | −50 |
| pO | −53 |
| pF | −59 |
| pm | −67 |
| pK | −73 |
| pi | −81 |
| ph | −137 |

d) Isolation of a 0.53 kb BamHI-EcoRI fragment containing the PHO5R promoter:

Plasmid pR contains the PHO5R promoter on a 534 bp BamHI-EcoRI fragment. According to the numbering in Figure 3a, the fragment covers PHO5 promoter sequences from nucleotide-541 (BamHI site) to nucleotide-10. An EcoRI linker, ligated to nucleotide-10 (see Example 6b) contributes two G-residues upon EcoRI cleavage.

Plasmid pR is digested with restriction endonucleases BamHI and EcoRI. The 0.53 kb BamHI-EcoRI fragment is separated on a 0.8% low melting agarose gel and isolated as described in Example 4a. The nucleotide sequence is given in Figure 9.

In an analogous manner plasmid pY is digested and a 0.53 kb BamHI-EcoRI fragment is isolated containing the PHO5Y promoter. The nucleotide sequence is given in Figure 10.

e) Replacement of the SalI-EcoRI fragment in plasmid p31 by a SalI-EcoRI fragment of the new constructions

Five µg of plasmid p31 (cf. Example 5d) are digested with restriction endonuclease SalI. The restricted DNA is precipitated with ethanol and resuspended in 50 µl of 100 mM Tris pH 7.5, 50 mM NaCl, 5 mM

$MgCl_2$. The DNA is digested with EcoRI to completion. The restriction fragments are separated on a 0.8% low melting agarose gel in Tris-borate-EDTA buffer pH 8.3. A 3.5 kb DNA fragment is isolated in a small gel block containing the DNA band.

Five μg each of clones pR and pY (cf. Table 1 and Figure 8) are digested with SalI and EcoRI in the same way as described above. The 0.8 kb DNA fragments are isolated in small blocks of low melting agarose gel.

0.67 μg of the 3.5 kb SalI-EcoRI fragment of vector p31 is ligated to 0.34 μg of the 0.8 kb SalI-EcoRI fragment of plasmid pR or pY, respectively. Appropriate gel blocks, containing the DNA fragments are mixed and melted at 65°C. The liquified gel is diluted three times. Ligation is performed in a total volume of 240 μl of 60 mM Tris pH 7.5, 10 mM $MgCl_2$, 10 mM DTT, 1 mM ATP with 750 U of T4 DNA ligase (Biolabs) overnight at 15°C. A 2 μl aliquot of each of the ligations is added to 100 μl of calcium treated, transformation competent *E. coli* HB101 cells (see Example 4a).

8 transformed, $amp^R$ colonies each are grown individually in LB medium containing 100 μg/ml ampicillin. Plasmid DNA is analysed by restriction analysis. The clones of each group are identical. One clone each is further used and referred to as p31R or p31Y, respectively (Figure 7).

Example 7
Preparation of the TPA gene
a. Preparation of mRNA

Falcon cell culture flasks (175 $cm^2$) are seeded with a primary inoculum of $10\times10^6$ HeLa cells and maintained in 25 ml of Dulbecco's modified Eagle's medium (DME) supplemented with 5% fetal bovine serum. The medium is changed every three days until confluency is attained.

Confluent monolayer cultures are washed twice with PBS (phosphate-buffered saline: 80 g NaCl, 2 g KCl, 14.4 g $Na_2HPO_4$ and 2 g $KH_2PO_4$ per 1 solution) and subsequently maintained for 16 hours in DME supplemented with 100 ng/ml TPA. Total RNA is extracted as described (27), applying lysis buffer directly to the cell monolayers. Poly(A)-rich sequences are isolated from total RNA according to the method of Nagamine et al. (28).

Sucrose gradient centrifugation is performed in a Beckman ultracentrifuge equipped with a SW-4 rotor. 100 μg of poly(A) RNA in 200 μl $H_2O$ are centrifuged through a 15—30% sucrose gradient in 0.02 M Tris · HCl, pH 7.4, 0.1% SDS, 0.01 M EDTA, 0.04 M NaCl at 30,000 rpm for 12 hours at 18°C. The gradient is fractioned from the bottom into 36 fractions and RNA is precipitated by the addition of NaCl to 0.2 M and 2.5 volumes EtOH. After an overnight incubation at −20°C, RNA is recovered by centrifugation and dissolved in 20 μl of $H_2O$.

Stage six oocytes are selected by visual inspection, maintained in modified Barth's medium and injected with mRNA from the sucrose gradient fractions essentially as described (29). Plasminogen activator secretion by injected oocytes is demonstrated by radial caseinolysis as described by Nagamine et al. (28). Maximal TPA secretion is seen in oocytes injected with the 21—23S fractions.

b. cDNA synthesis and molecular cloning

HeLa poly(A) RNA enriched for TPA mRNA is copied into cDNA. Eight μg of 50 mM Tris · Hcl, pH 8.3, 75 mM KCl, 8 mM $MgCl_2$, 1% v/v ethanol, 0.2 mM EDTA, 25 μg/ml oligo $dT_{12-18}$; 0.5 mM each of dNTP and 12 μCi of [$\alpha^{32}$P]dCTP (New England Nuclear). The solution is cooled to 0°C and 3 mM sodium pyrophosphate, 1 mM DTT and 144 units reverse transcriptase (Life Sciences Ltd) are added. After 60 min at 39°C, 32 additional units of reverse transcriptase are added. After 100 min incubation the reaction is stopped by the addition of SDS to 0.2%, EDTA to 15 mM. Incorporation of [$\alpha^{32}$P]dCTP into cDNA is assessed by trichloroacetic acid precipitation of aliquots of the reaction mixture. 1.15 μg cDNA is synthesized from 8 μg RNA (14.4% copy). Following two extractions with chloroform-isoamylalcohol (24:1 v/v), the aqueous phase is desalted on a 4 ml column (5×0.5 cm) of Sephadex G50 fine by centrifugation in 20 mM Tris · HCl, pH 9, 100 mM NaCl, 1 mM EDTA. Void volume fractions are pooled and NaOH is added to 0.3 N. After 12 hours at 20°C, HCl is added to 0.3 N and nucleic acids are precipitated by the addition of 3 volumes ethanol. Second strand synthesis is performed in a 200 μl solution containing 200 mM N-(2-hydroxyethyl)-piperazine-N'-ethane-2-sulfonic acid (Hepes), pH 6.9, 30 mM KCl, 10 mM DTT, 10 mM $MgCl_2$, 0.5 mM each of dATP, dCTP, dTTP and 25 units Klenow fragment per μg cDNA. After 2 hour incubation at 25°C the reaction is stopped by the addition of SDS to 0.1% and the solution desalted on a 4 ml column of Sephadex G50 fine by centrifugation in 30 mM sodium acetate, pH 4.5, 0.2 m NaCl, 3 mM $ZnCl_2$, 0.1% SDS. 2.5 units/ml nuclease S1 are added to the void volume fractions and the solution incubated at 37°C for 30 min. The solution is extracted with phenol-chloroformisoamylalcohol (24:24:1, v/v) and nucleic acid in the aqueous phase precipitated with 0.3 M NaCl and 3 vol. ethanol. 76% of the [$\alpha^{32}$P]dCTP incorporated into the first strand remain precipitable by trichloroacetic acid following S1 digestion, and analysis on an alkaline agarose gel (30) shows double-stranded cDNA ranging in size from 500 to over 3000 bp. The ds cDNA is size-fractionated on a 5—20% sucrose gradient in 10 mM Tris · HCl, pH 7.4, 100 mM NaCl, 1 mM EDTA, 0.1% SDS. After 10 hours centrifugation at 12°C in a SW41 rotor at 39K, those fractions containing the largest size cDNA (43% of total samples) are pooled and precipitated by the addition of NaCl to 0.3 M, 5 μg/ml BSA and 3 volumes ethanol. 25 ng of size-fractionated cDNA are tailed with dC as described (31) in a 85 μl reaction containing 450 units/ml terminal transferase, 0.1 M potassium cacodylate, pH 7.5, 2 mM $CoCl_2$, 1 mM EDTA, 0.1 mM DTT and 5 μM[$\alpha^{32}$P]dCTP (20 μCi). After 3 min at 30°C the reaction is stopped by the addition of EDTA to 10 mM, SDS to 0.2% w/v and tRNA to 100 μg/ml. The solution is extracted twice with chloroform-isoamylalcohol and desalted on a 5 ml column of Sephadex G50 fine in 20 mM Tris · HCl, pH 9, 100 mM NaCl, 1 mM EDTA. Void volume fractions were pooled and nucleic acid precipitated by the addition of NaCl to 0.3 M, 25 μg/ml tRNA and 3 volumes EtOH. 20 ng tailed, ds cDNA is annealed to 60 ng pBR322 tailed with dG at the PstI site (32) in 20 μl 0.4 M NaCl, 10 mM Tris · HCl, pH 7.5, 1 mM EDTA for 10 min at 65°C, 2 hours at 45°C, 2 hours at 22°C and then slowly cooled to 4°C overnight. The annealed cDNA is mixed at 0°C with 100 μl suspension of competent *E. coli* HB101/LM1035 (33).

Following 15 min incubation at 0°C and 5 min at 37°C, 1.9 ml LB is added to the bacterial suspension and incubation continued at 37°C for 2 hours. Cells are plated onto LB agar plates containing 10 μg/ml tetracycline. 100 transformants/ng vector are obtained with a background frequency of re-annealed vector of 13%. 5400 transformants are toothpicked from isolated colonies into 96 well microtiter plates containing 200 μl LB and 10 μg/ml tetracycline, grown overnight at 37°C, then stored frozen at −80°C after the addition of glycerol to 5% v/v.

c. Screening for TPA DNA sequences

Two synthetic oligonucleotides having the sequences

d5'-GGCAAAGATGGCAGCCTGCAAG-3' and d5'-GCTGTACTGTCTCAGGCCGCAG-3'

of human tissue plasminogen activator cDNA (34) are synthesized by the modified tri-ester method (35), purified by reverse-phase HPLC, and preparative polyacrylamide electrophoresis. The oligonucleotides are labelled with [$\gamma^{32}$P]ATP (New England Nuclear) on the 5' terminus using T4 kinase to a specific activity of $1\times10^6$ cpm (Cherenkov)/pmole (10).

Microtiter plates are thawed and transformant cultures are transferred in a 6 by 8 grid array to 82 mm Millipore HATF nitrocellulose filters, grown for 18 hours on LB agar plates containing 10 μg/ml tetracycline, and plasmids are amplified for 12 hours on LB agar plates containing 10 μg/ml tetracycline, 10 μg/ml chloroamphenicol. DNA is fixed to the filters by blotting each filter on Whatman 3M paper saturated with 10% SDS for 3 min; 0.5 N NaOH, 1.5 M NaCl for 5 min; 0.5 M Tris · HCl, pH 8, 1.5 M NaCl for 5 min; and 2×SSPE (SSPE: 0.15 M NaCl, 10 mM $NaH_2PO_4$, pH 7.4, 1 mm EDTA) for 5 min (42).

Filters are air dried, then baked under vacuum at 80°C for 2 hours. Baked filters are washed 2 hours at 37°C in 50 mM Tris · HCl, pH 7.5, 10 mM EDTA, 1 M NaCl, 1% SDS to remove adherent bacterial debris. Hybridization and washing is done as described by Wallace et al. (1981), using the empirical relationships between nucleotide length, G+C content and Tm that have been determined by Suggs et al. (45) and Smith (46). Filters are prehybridized (2 hours) and hybridized (12 hours) at 60°C in 1.6 ml filter of 900 mM NaCl, 60 mM $NaH_2PO_4$, pH 7.4, 7.2 mM EDTA (6×SSPE), 0.1%, w/v each BSA, polyvinylpyrrolidone, Ficoll 400 (5×Denhardt's solution), 200 μg/ml tRNA and 0.1% w/v SDS. A mixture of both $^{32}$P-labelled oligonucleotides at 0.3 pmol/ml are added to the pre-hybridization solution. Following hybridization, filters are washed in 6×SSC (SSC: 0.15 mM NaCl, 15 mM trisodium citrate) for 3×10 min at 4°C and 3×10 min at 60°C. Dried filters are exposed to Kodak XB5 X-ray film with a Dupont intensifying screen for 36 hours at −70°C. The microtiter wells that produce positive hybridization signals are identified and single colonies from these cultures are rescreened as above, hybridizing duplicate filters to each probe separately, and using 63°C for hybridization and washing the filters. Colonies showing positive signals with both sets of probes are picked from the master filter and grown overnight at 37°C in LB containing 10 μg/ml tetracycline. One of these colonies is referred to as pW349F. Plasmid preparations are obtained using the modified alkaline lysis method (36) followed by CsCl equilibrium centrifugation.

DNA of plasmid pW349F is analysed by digestion with restriction endonucleases PstI and BglII. The restriction fragments are of the expected sizes. It is therefore concluded that plasmid pW349F (7.1 kb) contains the complete structural gene for human tissue plasminogen activator.

d. DNA sequence of the TPA cDNA clone (cf. Figure 11)

Using a combination of the methods of Maxam and Gilbert (10) and Sanger (53) the TPA cDNA clone is sequenced. The results are given in Figure 11. Comparing the sequence data with those published by Pennica et al. (7), only one change can be observed which is located at nucleotide position 113 and gives rise to a change in the silent position of an amino acid of the pre-sequence of TPA (CTG→CTA).

Example 8

Construction of plasmids pJDB207/PHO5-TPA(1A) and pJDB207/PHO5-TPAΔ (Figures 12—15)

The PHO5 promoter and the PHO5 signal sequence are joined in frame to the coding sequence for mature TPA. A PHO5 transcription termination signal is also included in the construction.

a) Construction of M13mp9/PHO5Bam-Sal (see Figure 12)

A 623 bp BamHI-SalI fragment containing the PHO5 promoter (Example 3a, Figure 3a) is obtained by cutting 2 μg of pBR322/PHO5Bam-Sal (see Figure 1) with the restriction endonucleases BamHI and SalI (both from Biolabs) under the conditions indicated by the supplier. 2 μg of replicated form (RF) of the single stranded phage vector M13mp9 (23) is digested with the same enzymes. Both DNA preparations are loaded on a 0.6% soft agarose gel as described in Example 5. A 623 bp fragment derived from plasmid pBR322/PHO5Bam-Sal and a 7.2 kb band derived from M13mp9 are extracted as described in Example 5c. 150 ng of the 623 bp fragment and 150 ng of the 7.2 kb M13mp9 DNA fragment are ligated with 200 units of $T_4$ DNA ligase (Biolabs) for 4 hours at 15°C in 60 mM Tris · HCl, pH 7.5, 10 mM $MgCl_2$, 10 mM DTT and 1 mM ATP.

*E. coli* strain JM 101 is transformed as described by Messing (23) and 12 white plaques are picked and analysed by restriction analysis of the RF plasmid (38). All of the analysed clones contain the 623 bp fragment inserted into the Bam-Sal site of M13mp9. A single isolate is selected and called M13mp9/PHO5 Bam-Sal (see Figure 12).

b) Joining of the TPA protein coding sequence to the PHO5 signal sequence (see Figure 12)

2 µg of plasmid pW349F (cf. Example 7) is digested with BglII endonuclease (New England Biolabs) under conditions given by the supplier. The DNA is ethanol precipitated and resuspended in 6 mM Tris · HCl, pH 7.5, 50 mM NaCl, 6 mM $MgCl_2$. The 3′ recessed termini of the DNA are filled using the Klenow fragment of *E. coli* DNA polymerase. The reaction takes place in a total volume of 50 µl with 2 units of enzyme in the presence of 80 µM of dATP, dGTP, dCTP and dTTP for 30 min at 37°C. The incubation is stopped by phenol extraction and the DNA is precipitated with ethanol.

2 µg of RF plasmid M13mp9/PHO5Bam-Sal is digested with restriction endonuclease KpnI (Biolabs) as indicated by the supplier. The DNA is precipitated with ethanol and resuspended in 10 µl of 200 mM NaCl, 1 mM $ZnSO_4$ and 60 mM Na-acetate pH 4.6. One unit of $S_1$ exonuclease is added and the mixture is incubated for 60 min at 37°C. The reaction is stopped by phenol extraction, the DNA is ethanol precipitated and resuspended in 50 µl of 50 mM Tris · HCl, pH 7.5, 1 mM $MgCl_2$. 10 units of calf intestine alkaline phosphatase (Boehringer) are added and the mixture is incubated for 60 min at 37°C followed by 60 min at 65°C. The DNA is purified by DE52 (Whatman) ion exchange chromatography (see Example 9Aa) and then precipitated with ethanol.

1.5 µg of BglII digested plasmid pW349F treated with Klenow DNA polymerase (see above) is ligated to 0.5 µg of KpnI cut, $S_1$ digested and phosphatase treated M13mp9/PHO5Bam-Sal in a volume of 10 µl using 900 units $T_4$ DNA ligase in a buffer as described above, except that 4 mM ATP are used and the incubation takes place for 18 hours at room temperature. *E. coli* JM 101 cells are transformed, 6 white plaques are selected and the single stranded phage template is prepared as described (38). DNA sequencing of the junction between the 2.0 kb BglII fragment and the M13mp9 vector is performed using the dideoxy chain termination method (39). The following oligodeoxynucleotide primer is used:

5′ AGTATGGCTTCATCTCTC 3′

The oligodeoxynucleotide is synthesized by the phosphotriester method (40, 41). It hybridizes within the PHO5 promoter and allows elongation by the Klenow fragment of *E. coli* DNA polymerase across the desired DNA junction point. One correct isolate is obtained which has the following DNA sequence arrangement (starting from the ATG of the PHO5 protein coding sequence):

ATG TTT AAA TCT GTT GTT TAT TCA ATT TTA GCC GCT TCT TTG GCC AAT GCA GGA TCT TAC CAA GTG

______ PHO5 protein coding sequence
\- - - - - - TPA protein coding sequence

This construction is called M13mp9/PHO5-TPA (see Figure 12).

c) Construction of a shortened PHO5 transcription termination fragment (see Figure 13)

10 µg of p31R plasmid DNA (see Figure 7) are digested with restriction enzyme SmaI, the DNA is extracted with phenol and ethanol precipitated. The DNA is resuspended in 10 mM Tris · HCl pH 8.0 at a concentration of 0.5 mg/ml. 10 µg of the SmaI cleaved DNA are digested with 2 U of endonuclease Bal31 (BRL) in 100 µl of 20 mM Tris pH 8.0, 100 mM NaCl, 12 mM $MgCl_2$, 12 mM $CaCl_2$ and 1 mM EDTA.

Aliquots of 3 µg of DNA each are withdrawn after 90, 120 and 150 seconds of incubation at 30°C and are immediately mixed with 50 µl of phenol and 60 µl TNE. After extraction with phenol/chloroform and ethanol precipitation, the DNA is resuspended in 10 mM Tris · HCl pH 8.0 at a concentration of 100 µg/ml. To analyze the exonucleotytic digestion of Bal31, aliquots of 0.7 µg of DNA from each time point are digested with HindIII and analyzed by agarose gel electrophoresis. For further analysis the DNA from the 90 second time point is used. 2.2 µg of the plasmid DNA are incubated for 1 hour at 37°C with 2.8 U of Klenow DNA polymerase (large fragment of polymerase I, BRL) in 35 µl of 60 mM Tris · HCl pH 7.5, 10 mM $MgCl_2$ and 0.1 mM dNTPs.

Three µg of XhoI linker (5′-CCTCGAGG-3′, Collaborative Research) are kinased in 50 µl of 6 mM Tris · HCl pH 7.5, 10 mM $MgCl_2$, 4 mM DTT, 0.5 mM ATP and 35 U of T4 polynucleotide kinase (Boehringer) for 30 min at 37°C.

0.67 µg of kinased XhoI linkers and 0.4 µg of Bal31 treated blunt end DNA of plasmid p31R are ligated overnight at room temperature in 25 µl of 60 mM Tris pH 7.5, 10 mM $MgCl_2$ 5 mM DTT, 3.5 mM ATP and 450 U of T4 DNA ligase. The ligated DNA is separated from excess linkers by isopropanol precipitation in the presence of 10 mM EDTA, 0.3 M sodiumacetate pH 6.0 and 0.54 volumes of isopropanol. After 35 min of incubation at room temperature the DNA is sedimented by centrifugation. The pellet is dried at the air and resuspended in 17 µl of 6 mM Tris pH 7.9, 150 mM NaCl, 6 mM $MgCl_2$ and 6 mM mercaptoethanol. The XhoI linkers ligated to the DNA are cleaved with XhoI, the DNA is precipitated with isopropanol as described before and circularized by ligation. After 6 hours of ligation at 15°C in 50 µl of 60 mM Tris · HCl pH 7.5, 10 mM $MgCl_2$, 10 mM DTT, 1 mM ATP and 600 U of T4 DNA ligase, 10 µl of each ligation mixture are added to 100 µl of calcium-treated, transformation competent *E. coli* HB101 cells (see Example 4a).

24 $amp^R$ colonies are grown individually in LB medium containing 100 mg/l ampicillin. Plasmid DNA[p31R(XhoI)] is isolated and analysed by HaeIII digestion. Two plasmids are chosen and the nucleotide sequence adjacent to the XhoI site in the terminator fragment is determined by the technique of Maxam and Gilbert (10). The sequence is shown in Figure 15.

d) Transfer of the PHO5-TPA hybrid gene to the yeast vector pJDB207 (see Figure 13)

A RF plasmid preparation of M13mp9/PHO5-TPA is performed as described above. 2 µg of this DNA is digested with restriction endonuclease BamHI (Biolabs) and SalI (Biolabs) and the 2.6 kb fragment is isolated by electrophoresis on a low melting agarose gel (see Example 5c). Likewise, 100 ng of the 134 bp HindIII-XhoI fragment of plasmid p31R(XhoI) containing the PHO5 terminator are prepared. In addition, 1 µg of plasmid pJDB207 (22) is digested with BamHI and HindIII and the 6.5 kb fragment is isolated by electrophoresis on a low melting agarose gel.

0.5 µg of each of the three fragments are ligated in 20 µl of 60 mM Tris · HCl, pH 7.5, 10 mM $MgCl_2$, 10 mM DTT, 1 mM ATP with 200 units of $T_4$ DNA ligase (Biolabs) overnight at 15°C. Transformation of *E. coli* HB101 is performed as described in Example 4, selecting for ampicillin resistant colonies. Four colonies are picked, plasmid DNA is prepared (using the method described in Example 2) and the DNA is analyzed using the following restriction sites as landmarks: BamHI, HindIII, BstEII. All isolates are found to be correct. One of the plasmids is referred to as pJDB207/PHO5-TPA(1A).

e) Construction of pJDB207/PHO5-TPAΔ (see Figure 14)

10 µg of double stranded DNA of M13mp9/PHO5-TPA is digested with SalI. After phenol extraction and ethanol precipitation the DNA is resuspended in 10 mM Tris · HCl pH 8.0 at a concentration of 0.5 mg/ml. Bal31 digestions are performed essentially as described under Example 8c) and XhoI linkers are added. After transformation into *E. coli* strain JM101 RF DNA is isolated and the position of the XhoI linker is determined by the dideoxy sequencing method (39), using the oligodeoxynucleotide primer 5'-GAACGGTAGGTATTGATTG-3'.

The M13 derivatives containing the XhoI linkers at different positions are named

M13mp9/PHO5-TPA (XhoI-1)
M13mp9/PHO5-TPA (XhoI-2)
M13mp9/PHO5-TPA (XhoI-3)
M13mp9/PHO5-TPA (XhoI-4)

Position 1—4 of the XhoI linkers are shown below.

```
                                                                  2
CGACCG[TGA]CCAGGAACACCCGACTCCTCAAAAGCAAATGAGATCC↓CGCCTCTTCTTCTTCA

          1      3       4
GAAGACACTG↓CAAAGG↓CGCAGTG↓CTTCTCT
```

[TGA] translational stop codon for TPA
1, 2, 3, 4 positions of XhoI linker (5'-CCTCGAGG-3')

2 µg of RF DNA from each of the four above M13 derivatives [M13mp9/PHO5-TPA (XhoI-1 to 4)] are digested with BamHI and XhoI and the 2.5 kb fragments are isolated by low melting agarose gel electrophoresis (see Example 5c). Likewise, 2 µg of p31R(XhoI) are digested with XhoI and HindIII and the 134 bp fragment is isolated. 2 µg of yeast plasmid pJDB207 is digested with BamHI and HindIII and the 6.5 kb DNA fragment is isolated as above. The three fragments are ligated at 15°C for 15 hours and *E. coli* HB101 is transformed to ampicilline resistance. Plasmid DNA is isolated from sets of 12 colonies and the constructions are verified by restriction analysis with HindIII, XhoI and BamHI restriction endonucleases. Isolates of the four cloning experiments are picked and referred to as

pJDB207/PHO5-TPAΔ1,
pJDB207/PHO5-TPAΔ2,
pJDB207/PHO5-TPAΔ3,
pJDB207/PHO5-TPAΔ4.

Example 9

Construction of plasmid p31RL/TPA(12-2) (Figure 16)

The junction of the PHO5 promoter and the coding sequence of mature TPA is done by a synthetic oligodeoxynucleotide. A plasmid containing this oligodeoxynucleotide linker at the 3′ end of the PHO5 promoter is used as an acceptor DNA to clone the coding sequence for mature TPA.

A. Preparation of acceptor plasmid p31RL (Figure 16):

a) Digestion of plasmid p31R with EcoRI and alkaline phosphatase:

3 μg of plasmid p31R DNA (see Example 6e) are digested with 6 units of restriction endonuclease EcoRI for 60 min at 37°C under conditions recommended by the supplier (Boehringer). After complete digestion, the sample is heated for 10 min. at 65°C to inactivate the enzyme. 0.05 volumes of 1 M Tris · HCl pH 8.0 and 2 U of calf intestine alkaline phosphatase (Boehringer) are added. The mixture is incubated for 60 min. at 37°C, and then for 1 hour at 65°C to heat-inactivate the phosphatase. The DNA is purified by DE52 (Whatman) ion exchange chromatography. The DNA is absorbed to DE52 (100 μl bed volume packed in a siliconized Pasteur pipet) in low salt buffer (150 mM NaCl, 10 mM Tris · HCl pH 8.0, 1 mM EDTA) and is eluted with a high salt buffer solution (1.5 M NaCl, 10 mM Tris · HCl, 1 mM EDTA). The eluted DNA is precipitated with ethanol and resuspended in 10 mM Tris · HCl pH 8.0 at a concentration of 0.15 mg/ml.

b) Ligation of a chemically synthesized DNA linker to p31R digested with EcoRI and alkaline phosphatase:

An oligodeoxynucleotide of the formula

5′-GAATTCCATGGTACCATGGAATTC-3′

is synthesized by the phosphotriester method (Itakura et al. (40), de Rooij et al. (41)). The sequence of the oligodeoxynucleotide is self-complementary. Annealing leads to a double-stranded DNA linker of 24 base pairs. 5 μg of the synthetic oligodeoxynucleotide are phosphorylated at its 5′ ends with 7.5 μCi of [γ-$^{32}$P]-ATP (5000 Ci · mmol$^{-1}$, Amersham) in 50 μl of 60 mM Tris · HCl pH 7.5, 10 mM $MgCl_2$, 4 mM DTT, 0.5 mM ATP and 35 units of $T_4$ polynucleotide kinase (Boehringer) for 30 min. at 37°C. The mixture is further incubated for 5 min. at 75°C, is then allowed to cool to room temperature and is stored at −20°C.

2.5 μg of the radioactively phosphorylated, annealed linker DNA are self-ligated in 50 μl of 60 mM Tris · HCl pH 7.5, 3.5 mM ATP, 5 mM DTT and 2000 units of $T_4$ DNA ligase (Biolabs) at 6°C overnight. The $T_4$ DNA ligase is inactivated at 65°C for 10 min. The resulting multimers of the linker DNA are digested with restriction endonuclease EcoRI in 60 μl of 100 mM Tris · HCl pH 7.5, 10 mM $MgCl_2$, 50 mM NaCl and 55 units of EcoRI (Boehringer) for 60 min at 37°C. The reaction is stopped by freezing the sample in liquid nitrogen.

0.5 μg of phosphorylated, EcoRI-cleaved linker DNA are ligated to p31R digested with EcoRI and alkaline phosphatase in 10 μl of 60 mM Tris/HCl pH 7.5, 10 mM $MgCl_2$, 1 mM ATP, 5 mM DTT and 300 units of $T_4$ DNA ligase (Biolabs) at 15°C for 3 hours. Aliquots of 4 μl of the ligation mixture are added to 100 μl of calcium-treated, transformation competent *E. coli* HB101 cells (see Example 4a). Four transformed, amp$^R$ colonies are grown individually in LB medium containing 100 μg/ml of ampicillin. Plasmid DNA is prepared according to the method of Holmes et al. (26) and is analysed by KpnI and SmaI/BamHI digests.

One clone, which is cleavable by restriction endonuclease KpnI showing the expected restriction fragments after SmaI/BamHI double digest is chosen for further constructions and is referred to as p31RL.

Plasmid p31R can be replaced by plasmid p31Y or any of the following plasmids mentioned in Example 6c: p31P, p31V, p31L, p31N, p31C, p31H, p31S, p31k, p31I. EcoRI-cleaved, dephosphorylated p31Y DNA is ligated to phosphorylated EcoRI-cleaved linker DNA. Transformation and analysis of amp$^R$ colonies is performed as described above. One clone with the expected restriction fragments is chosen and referred to as p31YL.

B. Insertion of TPA DNA into plasmid p31RL (Figure 16):

a) Digestion of plasmid p31RL with NcoI:

5 μg of p31RL DNA are digested with restriction endonuclease NcoI under conditions recommended by the supplier (Biolabs). After complete digestion the solution is deproteinized by phenol/chloroform extraction and the DNA is concentrated by ethanol precipitation. The DNA is redissolved in 10 mM Tris · HCl pH 8 at a concentration of 0.25 mg/ml.

b) Reacion of Klenow DNA polymerase with NcoI cleaved p31RL DNA:

5 μg of p31RL DNA cleaved with NcoI is incubated in 100 μl of 60 mM Tris · HCl pH 7.5, 10 mM $MgCl_2$, 0.4 μM dATP, 30 μCi[α-$^{32}$P]-dATP, 0.1 mM dTTP, 0.1 mM dCTP, 0.1 mM dGTP and 8 units of Klenow DNA polymerase (large fragment; BRL). After 15 min. at room temperature the concentration of unlabeled dATP is raised to 0.1 mM. The incubation is continued for additional 45 min at room temperature. The reaction is stopped by phenol/chloroform extraction. The DNA is concentrated by ethanol precipitation and redissolved in 10 mM Tris · HCl pH 8 at a concentration of 0.25 mg/ml.

As an alternative to reactions a) and b) p31RL DNA can also be treated with restriction endonuclease KpnI and $T_4$ DNA polymerase as described reaction c) and d):

c) Digestion of plasmid p31RL with KpnI:

10 μg of p31RL DNA are digested to completion as recommended by the supplier (Biolabs). The mixture is extracted with phenol/chloroform. The DNA is precipitated with ethanol and redissolved in 10 mM Tris · HCl pH 8, 0.1 mM EDTA at a concentration of 0.4 mg/ml.

d) Reaction of $T_4$ DNA polymerase with KpnI cleaved p31RL DNA:

9 μg of p31RL DNA cleaved with KpnI is incubated at 37°C for 2.5 min with 10 units of $T_4$ DNA polymerase (42) in 100 μl of 33 mM Tris-acetate pH 7.9, 66 mM potassium acetate, 10 mM magnesium acetate and 0.5 mM DTT (trimming step). Resynthesis of the trimmed DNA strand is performed in an increased volume of 200 μl in the presence of 33 mM Tris-acetate pH 7.9, 66 mM potassium acetate, 10 mM magnesium acetate, 0.5 mM DTT, 0.4 μM dATP, 30 μCi[α-$^{32}$P]-dATP, 0.1 mM dTTP, 0.1 mM dCTP and 0.1 mM dGTP. After 18 min at 37°C the concentration of unlabeled dATP is raised to 0.1 mM and incubation is continued for additional 35 min at 37°C. The reaction is stopped by phenol/chloroform extraction. The DNA is precipitated with ethanol and redissolved in 10 mM Tris · HCl pH 8 at a concentration of 0.4 mg/ml.

e) Digestion with SmaI of the linear, DNA polymerase-treated p31RL plasmid DNA:

4.5 μg of p31RL DNA treated with NcoI and Klenow DNA polymerase (Example 9Bb) or 4 μg of p31RL DNA incubated with KpnI and $T_4$ DNA polymerase (Example 9Bd) are digested with restriction endonuclease SmaI to completion. The reactions are stopped by phenol/chloroform extraction. The DNA is precipitated with ethanol and resuspended in 100 μl of 50 mM Tris · HCl pH 8.0.

f) Dephosphorylation and isolation of the 4.3 kb large DNA fragments:

1.4 units of calf intestine alkaline phosphatase (Boehringer) are added to each DNA sample. They are incubated for 1 hour at 37°C and subsequently for 1 hour at 65°C to inactivate the enzyme. The salt concentration of the mixtures is adjusted to 150 mM NaCl and the DNA is purified by DE52 ion exchange chromatography as described in Example 9Aa. The DNA is precipitated with ethanol and redissolved in 45 μl of 10 mM Tris · HCl pH 8.

The large 4.3 kb DNA fragments are isolated on a preparative 0.8% agarose gel in Tris-borate-EDTA pH 8.3 by cutting out small gel blocks which contain the DNA. DNA from each gel block is electroeluted separately in a dialysis bag filled with 1.5 ml of 5-fold diluted Tris-borate-EDTA buffer pH 8.3. The closed bags are submersed in Tris-borate-EDTA buffer pH 8.3. After 30 min. at 100 mA the polarity of the current is reversed for 45 sec to repell the DNA from the dialysis membrane. The buffer surrounding the gel block in the dialysis bag is recovered, adjusted to 150 mM NaCl and passed through a DE52 (Whatman) ion exchange column. The DNA is eluted with a high salt buffer (1.5 M NaCl, 10 mM Tris · HCl pH 8.0 and 1 mM EDTA), precipitated with ethanol and redissolved in 10 mM Tris pH 8.0 at a concentration of 0.2 mg/ml. The DNA fragments are referred to as p31RLΔ.

Reactions a) to f) can be performed in an analogous manner also with p31YL DNA instead of p31RL DNA to yield p31YLΔDNA fragments.

g) BalI digestion of plasmid pW349F:

Plasmid pW349F contains the structural gene for human tissue-specific plasminogen activator cloned in the PstI site of pBR322 (see Example 7c).

To prepare plasmid DNA a single colony of *E. coli* HB101 transformed with plasmid pW349F is used to inoculate 100 ml of LB medium containing 10 mg/l tetracyclin. The culture is shaken overnight at 37°C at 200 rpm. Plasmid preparation is performed as described in Example 2.

10 μg of pW349F plasmid DNA are digested with 10 units of restriction endonuclease BalI (BRL) in 200 μl of 6 mM Tris · HCl pH 7.5, 6 mM NaCl, 6 mM $MgCl_2$, 6 mM 2-mercaptoethanol and 0.1 mg/ml bovine serum albumin for 2 hours at 37°C. After complete digestion the solution is deproteinized by phenol/chloroform extraction. The DNA is precipitated with ethanol and resuspended in 10 mM Tris · HCl pH 8.0 at a concentration of 0.2 mg/ml.

Ligation of XhoI linkers to the blunt ends of the BalI cleaved DNA is an optional step to provide a convenient cloning site for further constructions.

h) Digestion of BalI cleaved pW349F DNA with BglII

4 μg of BalI cleaved pW349F DNA is digested with restriction endonuclease BglII as recommended by the supplier (Biolabs). After complete digestion proteins are extracted with phenol-chloroform. The DNA is precipitated with ethanol and is redissolved in $H_2O$ at a concentration of 0.2 mg/ml.

i) Limited reaction of Klenow DNA polymerase and nuclease $S_1$ at the BglII restriction site:

4 μg of pW349F DNA cleaved with BalI and BglII are incubated in 100 μl of 60 mM Tris · HCl pH 7.5, 10 mM $MgCl_2$, 0.1 mM dATP, 0.1 mM dGTP with 8 units of Klenow DNA polymerase for 30 min at room temperature. The reaction is stopped by the addition of EDTA to a final concentration of 10 mM. Protein is extracted by phenol/chloroform. The DNA is precipitated with ethanol and is resuspended in $H_2O$ at a concentration of 0.2 mg/ml.

For further cleavage with nuclease $S_1$ the DNA is incubated in 50 μl of 30 mM sodium acetate pH 4.6, 200 mM NaCl, 1 mM $ZnSO_4$ and 1.5 units of $S_1$ nuclease (Sigma) for 30 min at 37°C. After phenol/chloroform extraction the DNA is precipitated with ethanol and is redissolved in 45 μl of 10 mM Tris · HCl pH 8.0.

k) Isolation of a 1.86 kb BglII-BalI restriction fragment:

The 1.86 kb BglII-BalI DNA fragment, with the BglII restriction site converted to a blunt end by Klenow DNA polymerase and nuclease $S_1$ as described above, is isolated on a preparative 0.8% agarose gel in Tris-borate-EDTA pH 8.3. The DNA is recovered from gel blocks by electro-elution as described in Example 9Bf.

l) Cloning of the 1.86 kb DNA fragment into suitable acceptor DNA:

0.3 μg of the 1.86 kb blunt end DNA fragment (see Example 9Bk) is ligated to 0.6 μg of p31RLΔ (see Example 9Bf). The reaction is done in 10 μl of 60 mM Tris · HCl pH 7.5, 10 mM $MgCl_2$, 5 mM DTT, 3.5 mM ATP with 400 units of $T_4$ DNA ligase (Biolabs) for 20 hours at 15°C.

3 μl of 6 μl aliquots of the ligation mixtures are added to 100 μl each of calcium-treated, transformation competent *E. coli* HB101 cells (see Example 4a).

48 transformed, $amp^R$ colonies are each grown individually in LB medium containing 100 μg/ml of ampicillin. Plasmid DNA is prepared according to the method of Holmes *et al* (26) and analysed by EcoRI digestion. 12 clones have the 1.86 kb DNA fragment cloned in the right orientation. These clones are further analysed by sequencing the DNA at the junction between PHO5 promoter and TPA structural gene. A synthetic oligodeoxynucleotide is used that hybridizes close to the mRNA start site of the PHO5 promoter and allows sequencing of the downstream junction region. M13 reverse priming conditions (43) are used for denaturation of doublestranded plasmid DNA and hybridisation of the synthetic oligodeoxynucleotide. DNA sequencing is performed as described (39).

Two clones have the following correct junction sequence

→ PHO5 mRNA

GAATA|AGAACAACAACAAATAGAGCAAGCAAATTCGGAATTCC ATG TCT TAC CAA...
Met Ser Tyr Gln...

→ PHO5 mRNA start

———— PHO5 DNA
.............. linker DNA
- - - - - - - TPA DNA

These clones are identical and are referred to as p31RL/TPA(12-2).

Example 10

Subcloning of gene constructions in the high copy number yeast vector pJDB207 (cf. Figure 17)

The construction described in Example 9 contains the PHO5 promoter without its signal sequence, the TPA coding region and the PHO5 transcription termination signals in a tandem array, inserted in a pBR322 derived vector. For expression in yeast the whole insert is subcloned in yeast vector pJDB207 (22) allowing selection for leucine prototrophic colonies.

2 μg of p31RL/TPA(12-2) DNA are digested with restriction endonucleases SalI and HindIII. The restriction fragments are separated on a preparative 0.8% low melting agarose gel and the small 2.8 kb fragment is cut out of the gel.

5 μg of pJDB207 DNA are digested with restriction endonucleases SalI and HindIII. The large 6.2 kb fragment is isolated from a preparative 0.8% low melting agarose gel. Gel blocks containing the DNA fragments are liquified at 65°C and diluted with $H_2O$ to lower the agarose concentration to about 0.3%.

The 2.8 kb SalI-HindIII fragment of plasmid p31RL/TPA(12-2) is mixed with an equimolar amount of the 6.2 kb HindIII-SalI fragment of pJDB207. Ligations are carried out in 100 μl for 4 hours at 15°C. 10 μl of each ligation mixture are used to transform competent *E. coli* HB101 cells as described in Example 4a. Several $amp^R$ colonies from each experiment are grown individually in LB medium containing 100 μg/ml of ampicillin. The plasmid DNA is analysed for the size of the insert by cleavage with restriction endonucleases HindIII and SalI. A single clone with the correct insert is referred to as pJDB207R/PHO5-TPA(12-2).

Example 11

Transformation of *Saccharomyces cerevisiae* GRF18

The plasmids pJDB207/PHO5-TPA(1A), pJDB207/PHO5-TPAΔ1, pJDB207/PHO5-TPAΔ2, pJDB207/PHO5-TPAΔ3, pJDB207/PHO5-TPAΔ4 and pJDB207R/PHO5-TPA(12-2) are each introduced into *Saccharomyces cerevisiae* strain GRF18 (α, his3-11, his3-15, leu-2,3, leu2-112, $can^R$) using the transformation protocoll described by Hinnen et al. (12). One μg each of plasmid DNA is added to 100 μl of a spheroplast suspension and the mixture is treated with polyethylene glycole. The spheroplasts are mixed with 10 ml regeneration agar and plated onto yeast minimal medium plates without leucine. After incubation for 3 days at 30°C, about 200 transformed cells are obtained.

A single yeast colony from each of the yeast transformations is picked. The different colonies are referred to as

*Saccharomyces cerevisiae* GRF18/pJDB207/PHO5-TPA(1A),
*Saccharomyces cerevisiae* GRF18/pJDB207/PHO5-TPAΔ1,
*Saccharomyces cerevisiae* GRF18/pJDB207/PHO5-TPAΔ2,
*Saccharomyces cerevisiae* GRF18/pJDB207/PHO5-TPAΔ3,
*Saccharomyces cerevisiae* GRF18/pJDB207/PHO5-TPAΔ4 and
*Saccharomyces cerevisiae* GRF18/pJDB207R/PHO5-TPA (12-2).

Yeast cells are grown at 30°C in 10 ml of yeast minimal medium in a 100 ml Erlenmeyer flask with shaking for 24 hours until a density of about 2—3$\times10^7$ cells/ml is reached. The cells are washed once with 20 ml of low $P_i$ minimal medium. Three ml of the resuspended cells are used to inoculate 300 ml low-$P_i$ minimal medium and 300 ml normal minimal medium, respectively, in 1000 ml Erlenmeyer flasks. Incubation is at 30°C at 160 rpm. Induction of the PHO5 promoter is followed by measuring the appearance of acid phosphatase activity in whole cells as described by Toh-e et al. (44). The cells are grown to about 1—2$\times10^7$ cells/ml (26—30 hours of incubation).

Example 12

Preparation of yeast cell extracts and determination of the TPA activity

Cells from 35 ml of low $P_i$ culture medium (48) at a cell density of 1—2$\times10^7$/ml are collected by centrifugation in a Sorvall SS34 rotor for 10 min at 3000 rpm. The cells are washed in a buffer containing the salt components of the culture medium (i.e. without aminoacids, glucose, vitamins, trace elements). The cells are centrifuged at room temperature for 5 min at 3000 rpm. The sedimented cells are resuspended in a total volume of 4 ml of cold 66 mM sodium phosphate buffer pH 7.4 and 0.1% (v/v) Triton X-100. The cell suspension is transferred to a 30 ml Corex tube, 8 g of glass beads (0.4 mm in diameter) are added and the suspension is shaken on a Vortex Mixer (Scientific Instruments Inc., USA) at full speed for 4 min and then cooled in an ice bath. More than 90% of the cells are broken by this procedure. Cell debris and glass beads are sedimented by centrifugation for 10 min at 8000 rpm at 4°C in a Sorvall HB-4 rotor. The supernatant is transferred to Eppendorf tubes, frozen in liquid nitrogen and stored at −60°C.

TPA activity is determined according to the method of Ranby (49) with slight modifications. D-Val-Leu-Lys-pNA (Kabi S-2251) is used as substrate. The absorption at 405 nm is corrected for unspecific cleavage and related to an urokinase standard. The results are shown in Table 2.

TABLE 2
TPA activity in *S. cerevisiae* strain GRF18
transformed with different recombinant plasmids:

| Plasmid | TPA activity (units/1 yeast cell culture/$OD_{600}$) repressed (high $P_i$) | derepressed (low $P_i$) |
|---|---|---|
| pJDB207R/PHO5-TPA(12-2) | — | 60 |
| pJDB207/PHO5-TPA(1A) | 20 | 625 |
| pJDB207/PHO5-TPAΔ1 | — | 600 |
| pJDB207/PHO5-TPAΔ2 | — | 700 |
| pJDB207/PHO5-TPAΔ3 | — | 650 |
| pJDB207/PHO5-TPAΔ4 | — | 625 |

Example 13

Construction of a plasmid with a precise junction between the PHO5 signal sequence and the coding region for mature TPA (Figure 18)

This construction establishes a correct junction between the PHO5 signal sequence and the coding sequence of mature TPA using a synthetic oligodeoxynucleotide. For convenience the relevant region of plasmid pJDB207/PHO5-TPAΔ2 (see Example 8e) is subcloned in plasmid p31 (see Example 5).

a) Isolation of plasmid p31/PHO5-TPAΔ2,

2 μg each of plasmids pJDB207/PHO5-TPAΔ2 and p31 (see Examples 8e and 5) are digested with restriction endonucleases HindIII and SalI. After complete digestion the DNA fragments are separated on a 0.6% low melting agarose gel in Tris-borate-EDTA pH 8.3. The 2.6 kb HindIII-SalI fragment of pJDB207/PHO5-TPAΔ2 and the 3.1 kb HindIII-SalI fragment of p31 are cut out from the gel. The gel blocks are liquified at 65°C and diluted with $H_2O$ to lower the agarose concentration to 0.3%. Equimolar amounts of both fragment are mixed and ligated in 100 μl for 4 hours at 15°C. 10 μl of the ligation mixture are used to transform competent *E. coli* HB101 cells. Several amp$^R$ colonies are analyzed. A single clone with the correct insert is referred to as p31/PHO5-TPAΔ2.

The same construction are carried out with plasmids pJDB207/PHO5TPAΔ1, pJDB207/PHO5-TPAΔ3 and pJDB207/PHO5-TPAΔ4 (see Example 8e). The resulting plasmids are referred to as p31/PHO5-TPAΔ1, p31/PHO5-TPAΔ3 and p31/PHO5-TPAΔ4.

b) Construction of plasmid p31/PHO5-TPA18 (Figure 18)

30 μg of plasmid p31/PHO5-TPAΔ2 are digested with restriction endonuclease XhoI. The linearised DNA is extracted with phenol/chloroform, precipitated with ethanol and redissolved in 10 mM Tris · HCl pH 8 at a concentration of 0.5 mg/ml (p31/PHO5-TPAΔ2 · XhoI).

5 μg of the Xho-cleaved DNA is further digested with BalI. After complete digestion the DNA is extracted once with phenol/chloroform, precipitated with 2.5 volumes of ethanol and is resuspended in 10 mM Tris · HCl pH 8. The restriction fragments are separated on a 1.2% agarose gel in Tris-borate-EDTA buffer pH 8.3. The large 3.9 kb XhoI-BalI fragment is cut out from the gel in a small gel block. The DNA (fragment A, cf. Figure 18) is recovered from the gel block by electroelution and DE52 ion exchange chromatography as described in Example 9Bf. The DNA is concentrated by ethanol precipitation and redissolved in 10 mM Tris-HCl pH 8 at a concentration of 0.2 mg/ml.

20 μg of p31/PHO5-TPAΔ2 · XhoI (see above) are partially digested with restriction endonuclease PstI (0.75 units/μg DNA) in 200 μl of 10 mM Tris · HCl pH 7.5, 6 mM $MgCl_2$, 50 mM NaCl, 6 mM mercapto-ethanol and 0.01 mg/ml ethidium bromide for 45 min at 37°C. The reaction is stopped by extraction with phenol/chloroform. The DNA is precipitated with ethanol and redissolved in 10 mM Tris · HCl pH 8. The restriction fragments are separated on a 1.2% agarose gel in Tris-borate-EDTA pH 8.3. A 1.6 kb XhoI-PstI fragment (fragment B, cf. Figure 18) containing most of the coding region of mature TPA is recovered from a gel block by electroelution as described above. The DNA is ethanol precipitated and redissolved in 10 mM Tris · HCl pH 8 at a concentration of 40 μg/ml.

The correct junction between the PHO5 signal sequence and the coding region for mature TPA is provided by a synthetic linker of the formula

```
[C] 5'-CCAATGCATCTTACCAAGTGATCTGCA  -3'
[D] 3'-GGTTACGTAGAATGGTTCACTAG      -5'
```

Eight nucleotides at the 5′ end of the linker (5′-CCAATGCA...) represent part of the PHO5 signal sequence from the BalI site to the end and 19 nucleotides complement the sequence for mature TPA from the 5′ end of the coding region to the first PstI site (see Figure 19). Oligodeoxynucleotides C and D are synthesized by the phosphotriester method. They are individually phosphorylated at their 5′ ends, mixed in equal amounts and annealed as described in Example 9Ab.

60 ng (4 pmoles) of phosphorylated, annealed linker DNA are ligated to 0.4 μg (0.4 pmoles) of the 1.6 kb XhoI-PstI fragment (fragment B, see above) in 50 μl of 60 mM Tris · HCl pH 7.5, 10 mM $MgCl_2$, 1 mM ATP, 5 mM DTT and 600 units of T4 DNA ligase at 15°C for 16 hours. The ligase is inactivated for 10 min at 85°C. An excess of linkers is removed by precipitation of the DNA in the presence of 10 mM EDTA, 300 mM sodium acetate pH 6.0 and 0.54 volumes of isopropanol. Multiples of the linker DNA and the DNA fragment are resolved by digestion with BalI and XhoI. Due to the linker addition at the PstI site the 1.6 kb XhoI-PstI fragment is converted to a XhoI-BalI fragment.

0.4 μg of this 1.6 kb XhoI-BalI DNA fragment and 1 μg of the 3.9 kb XhoI-BalI fragment (fragment A, see above) are ligated in 10 μl of 60 mM Tris · HCl pH 7.5, 10 mM $MgCl_2$, 3.5 mM ATP, 5 mM DTT and 400 units of T4 DNA ligase at room temperature for 16 hours. Aliquots of 3 μl and 7 μl of the ligation mixture are added to 100 μl of calcium-treated, transformation competent *E. coli* HB 101 cells (see Example 4a). 10 transformed, amp$^R$ colonies are grown individually in LB medium containing 100 μg/ml ampicillin. Plasmid DNA is prepared according to Holmes et al. (26). The clones are analysed by sequencing across the linker region.

One clone which shows the correct in frame junction between the PHO5 signal sequence and the coding region for mature TPA (see Figure 19) is referred to as p31/PHO5-TPA18.

Plasmid p31/PHO5-TPAΔ2 can be replaced by p31/PHO5-TPAΔ1, p31/PHO5-TPAΔ3 or p31/PHO5-TPAΔ4. Constructions are performed as described above. In particular, one clone derived from plasmid p31/PHO5-TPAΔ3 is referred to as p31/PHO5-TPA42.

Example 14

Plasmid constructions with DNA coding for prepro-TPA (Figures 20, 21)

The PHO5 promoter is linked to DNA sequences coding for prepro-TPA. The nucleotide sequence for 35 aminoacids of the prepro-region is followed by the sequence coding for mature TPA.

a) Isolation of part of the TPA coding region including the TPA signal sequence (cf. Figure 20)

30 µg of plasmid pW349F are cleaved with restriction endonuclease BglI. After phenol/chloroform extraction and ethanol precipitation the DNA is further digested with EcoRI and BamHI. The restriction fragments are separated on a 1% agarose gel in Tris-borate-EDTA pH 8.3. The 0.9 kb BglI-EcoRI fragment is localised on the gel and cut out. The DNA is recovered by electro-elution as described in Example 9Bf.

3.5 µg of the 0.9 kb BglI-EcoRI fragment are digested with HgaI to completion resulting in 3 fragments. The mixture is extracted by phenol/chloroform and the DNA fragments are precipitated with ethanol. The sticky ends of the fragments created by the HgaI digestion are filled in a reaction with Klenow DNA polymerase (1 unit/µg DNA) containing 60 mM Tris · HCl pH 7.5, 10 mM $MgCl_2$ 0.1 mM TTP, 0.1 mM dCTP and 0.1 mM dGTP. After 60 min at 20°C the reaction is stopped by ethanol precipitation. The DNA fragments with blunt ends are mixed with a 40-fold molar excess of phosphorylated and annealed EcoRI linker (Biolabs). Ligation is carried out in 56 µl of 60 mM Tris · HCl pH 7.5, 10 mM $MgCl_2$, 5 mM DTT, 3.5 mM ATP and 2000 units of T4 DNA ligase for 16 hours at 15°C. Excess linker molecules are removed by precipitation of the DNA in the presence of 10 mM EDTA pH 7.5, 300 mM sodium acetate pH 6.0 and 0.54 volumes of isopropanol. The mixture of DNA fragments is digested with EcoRI to remove multiple linkers and with NarI. After complete digestion the restriction fragments are separated on a 1% agarose gel in Tris-borate-EDTA pH 8.3. The 446 bp EcoRI/[HgaI]-NarI fragment is cut out from the gel and the DNA is recovered by electroelution (see Example 9Bf).

b) Isolation of a DNA fragment containing the 3′ part of the TPA coding sequence and the yeast transcription termination signals (cf. Figure 20)

10 µg of plasmid p31/PHO5-TPAΔ2 are digested with HindIII. After phenol/chloroform extraction and ethanol precipitation the DNA is redissolved in $H_2O$ at a concentration of 0.1 mg/ml and digested with NarI. The restriction fragments are separated on a 1% agarose gel in Tris-borate-EDTA pH 8.3. The 1.4 kb NarI-HindIII fragment is isolated and recovered from the agarose gel block by electro-elution (see Example 9Bf).

c) Isolation of a DNA fragment containing vector sequences and the PHO5 promoter (cf. Figure 21)

5 µg of plasmid p31R are digested with restriction endonuclease HindIII and EcoRI. The restriction fragments are separated on a 1% agarose gel in Tris-borate-EDTA pH 8.3. The 3.9 kb HindIII-EcoRI DNA fragment is isolated and recovered from the agarose gel block by electro-elution.

d) Ligation of fragments (cf. Figure 21)

0.3 pmoles of the 3.9 kb HindIII-EcoRI fragment, 0.3 pmoles of the 1.4 kb NarI-HindIII fragment and 0.6 pmoles of the 446 bp EcoRI-NarI fragment are ligated in 12 µl of 60 mM Tris · HCl pH 7.5, 10 mM $MgCl_2$, 1 mM ATP, 5 mM DTT and 480 units of T4 DNA ligase at 15°C for 6.5 hours. 5 µl and 7 µl aliquots of the ligation mixture are added to 100 µl of calcium-treated, transformation competent *E. coli* HB 101 cells (see Example 4a).

23 transformed, $amp^R$ colonies are grown individually in LB medium containing 100 µg/ml ampicillin. Plasmid DNA of the different clones is analyzed by restriction with PstI. One of the clones with the expected pattern of restriction fragments is referred to as p31R/SS-TPAΔ2.

Plasmid p31/PHO5-TPAΔ2, can be replaced by p31/PHO5-TPAΔ3 or one of the other clones mentioned in Example 13a. Constructions are performed as described above. One clone derived from plasmid p31/PHO5-TPAΔ3 is referred to as p31R/SS-TPAΔ3.

Example 15

Plasmid constructions with the PHO5 signal sequence extended into the PHO5 coding region (cf. Figures 22—24).

Whenever foreign DNA is joined to the PHO5 signal sequence the resulting amino acid sequence in the vicinity of the signal peptide cleavage site is different from that of the genuine PHO5 protein. Changes in the amino acid sequence may influence the efficiency of processing of the protein by the signal peptidase. Therefore, the following DNA constructions use the PHO5 signal sequence with extensions into the coding region for mature acid phosphatase. Only at varying distances after the signal sequence the coding region for mature TPA is joined in frame via synthetic oligodeoxynucleotide linkers which contain processing sites for a yeast endopeptidase. Cleavage at these sites prevents the formation of fused proteins.

a) Subcloning of PHO5 sequences extending into the coding region for mature acid phosphatase (Figure 22)

DNA fragments are isolated which contain the PHO5 promoter, PHO5 signal sequence and varying length of coding sequence for mature acid phosphatase. RsaI restriction sites are located at positions 595, 637, 811 and 1312 from an upstream BamHI site (48). Since the PHO5 signal sequence extends to position 592, the different RsaI sites are located at position 3, 45, 219 or 720 bases, respectively, in the coding sequence for mature acid phosphatase, BamHI-RsaI fragments are subcloned via a synthetic oligodeoxynucleotide changing the RsaI site to a XbaI site.

Plasmid DNA of pJDB207/PHO5, PHO3 (cf. Example 2) is cut with BamHI and HpaI and the resulting 3.9 kb fragment containing the PHO5 and PHO3 genes is isolated by preparative gel electrophoresis. The 3.9 kb BamHI-HpaI fragment is sub-cloned into the vector pBR322, which has been cut with BamHI and PvuII. The resulting plasmid with the PHO5 and PHO3 genes as insert is called p29.

30 µg of plasmid p29 are digested with restriction endonucleases BamHI and EcoRI. After complete digestion the DNA is extracted with phenol/chloroform and precipitated with ethanol. The DNA is redissolved in 10 mM Tris · HCl pH 8.0 at a concentration of 0.5 mg/ml.

15 µg of BamHI, EcoRI cleaved p29 is partially digested with RsaI (0.9 unit/µg DNA) in 300 µl of 10 mM Tris · HCl pH 8.0, 6 mM $MgCl_2$, 50 mM NaCl, 6 mM mercapto-ethanol and 5 µg/ml of ethidium bromide for 45 min at 37°C. The digestion is stopped by phenol extraction. The DNA is precipitated with ethanol and redissolved in $H_2O$ at a concentration of 1 mg/ml.

An oligodeoxynucleotide of the formula

5′-CTAGATCTAG-3′

is phosphorylated at its 5′ end and is self-annealed as described in Example 9Ab. 4 µg (1400 pmoles) of phosphorylated and annealed linker DNA are ligated to 14 µg (140 pmoles) of restricted p29 DNA (see above) in 80 µl of 60 mM Tris · HCl pH 7.5, 10 mM $MgCl_2$, 3.5 mM ATP, 5 mM DTT and 3200 units of T4 DNA ligase at 20°C for 16 hours. T4 DNA ligase is inactivated for 10 min at 65°C. An excess of linkers is removed by precipitation in the presence of 10 mM EDTA, 300 mM sodium acetate pH 6.0 and 0.54 volumes of isopropanol. Digestion of the DNA with BamHI and XbaI (4 units/µg DNA) resolves multiple structures and leads to XbaI sticky ends. Due to the linker addition at the blunt end RsaI sites and subsequent XbaI cleavage of the linker all of the p29 BamHI-RsaI restriction fragments are converted to BamHI-XbaI fragments. They are separated on a 1% low melting agarose gel in Tris-borate-EDTA pH 8.3. Gel blocks containing the BamHI-XbaI fragments of 595 bp, 637 bp, 811 bp or 1312 bp in size are cut out from the gel.

Plasmid pBR322/PHO5 Eco-Bam is derived from plasmid pG7 (Example 2). pG7 is cut with restriction endonucleases EcoRI and BamHI. The resulting DNA fragments are separated by preparative gel electrophoresis. A 2.1 kb EcoRI-BamHI fragment is isolated which represents flanking sequences upstream of the PHO5 BamHI site. The 2.1 kb fragment is ligated to a 4 kb EcoRI-BamHI fragment of vector pBR322. The resulting plasmid is referred to as pBR322/PHO5 Eco-Bam.

Plasmid pBR322/PHO5 Eco-Bam contains a 2.1 kb EcoRI-BamHI fragment which represents flanking sequences upstream of the PHO5 BamHI site. 15 µg of plasmid DNA are digested with BamHI and XbaI. The restriction fragments are separated on a 0.6% low melting agarose gel in Tris-borate-EDTA pH 8.3. The 4.25 kb BamHI-XbaI fragment is cut out from the gel.

Gel blocks containing the DNA fragments are liquified at 65°C and diluted with $H_2O$ to lower the agarose concentration to 0.3%. Equimolar amounts of the 637 bp BamHI-XbaI fragment and the 4.25 kb BamHI-XbaI vector fragment are mixed and ligated in 125 µl of 60 mM Tris · HCl pH 7.5, 10 mM $MgCl_2$, 5 mM DTT, 1 mM ATP and 600 units of T4 DNA ligase at 15°C for 16 hours.

10 µl of the ligation mixture are added to 100 µl of transformation competent *E. coli* HB 101 cells as described in Example 4a. 5 $amp^R$ colonies are grown individually in LB medium containing 100 µg/ml ampicillin. The plasmid DNA is analysed for the size of the insert by cleavage with restriction endonucleases EcoRI and BamHI. A single clone with the correct insert is referred to as pBR322/PHO5Bam-Rsa 637.

The 637 bp BamHI-XbaI fragment can be replaced by BamHI-XbaI fragments of 595 bp, 811 bp or 1312 bp in size. Single clones with an insert of expected size are referred to as pBR322/PHO5Bam-Rsa 595, pBR322/PHO5Bam-Rsa 811 and pBR322/PHO5Bam-Rsa 1312.

b) Addition of synthetic oligodeoxynucleotides:

The 637 bp BamHI-XbaI fragment contains the PHO5 promoter and the PHO5 signal sequence with an extension into the coding sequence of mature acid phosphatase up to the RsaI site at position 637. The DNA fragment is joined in frame to the coding sequence of mature TPA via a synthetic oligodeoxynucleotide linker which codes for the amino acid sequence Leu-Glu-Gly-Val-Ser-Leu-Asp-Lys-Arg. This sequence is also found upstream of the coding sequence for yeast α factor (54).

5 µg of plasmid pBR322/PHO5Bam-Rsa 637 are digested with restriction endonuclease XbaI. After complete digestion the DNA is extracted with phenol/chloroform and precipitated with ethanol. The DNA is redissolved in 50 mM Tris · HCl pH 8.0 at a concentration of 50 µg/ml and is digested with 11 units of alkaline phosphatase from calf intestine (Boehringer) for 1 hour at 37°C. The enzyme is inactivated at 65°C for 1 hour. The DNA is purified by DE52 (Whatman) ion exchange chromatography and ethanol precipitation as described in Example 9Aa.

Two synthetic oligodeoxynucleotides of the formula

5'- CTAGAAGGGGTATCTTTGGATAAGA -3'
3'- TTCCCCATAGAAACCTATTCTCTAG -5'

are individually phosphorylated at their 5' ends as described in Example 9Ab. The phosphorylated oligodeoxynucleotides are mixed in equimolar amounts. The mixture is heated for 10 min at 75°C, is then allowed to cool slowly to room temperature and is stored at −20°C. This linker is referred to as linker C.

1 μg (120 pmoles) of phosphorylated, annealed linker C and 5 μg (1.5 pmoles) of XbaI cleaved, dephosphorylated pBR322/PHO5Bam-Rsa 637 are ligated in 40 μl of 60 mM Tris · HCl pH 7.5, 10 mM $MgCl_2$, 5 mM DTT, 1 mM ATP, 1600 units of T4 DNA ligase at 15°C for 20 hours. DNA ligase is inactivated for 10 min at 85°C and excess linkers are removed by isopropanol precipitation. The DNA is redissolved in $H_2O$ at a concentration of 0.25 mg/ml. Multiple linkers are removed by digestion with restriction endonuclease BglII. The DNA is precipitated with ethanol, redissolved and then further digested with BamHI. The restriction fragments are separated on a 0.6% low melting agarose gel in Tris-borate-EDTA pH 8.3. The 662 bp BamHI-BglII fragment (derived from the 637 bp BamHI-RsaI fragment) is localized and cut out from the gel.

c) Isolation of a vector fragment containing the coding sequence for mature TPA (Figure 23)

5 μg of plasmid p31R/SS-TPAΔ2 are digested with restriction endonucleases BamHI and BglII. After complete digestion the DNA is precipitated with ethanol and resuspended in 50 mM Tris · HCl pH 8.0 at a concentration of 75 μg/ml. The DNA fragments are dephosphorylated at their 5' ends by digestion with 1 unit of alkaline phosphatase from calf intestine (Boehringer) in 60 μl of 50 mM Tris · HCl pH 8.0 for 1 hour at 37°C. The phosphatase is inactivated by incubation at 65°C for 1.5 hours. The DNA is purified by DE52 (Whatman) ion exchange chromatography as described in Example 9Aa. The DNA is precipitated with ethanol, redissolved in 10 mM Tris-HCl pH 8.0 and applied to a 0.6% low melting agarose gel. The 5.2 kb large BamHI-BglII fragment is cut out from the gel.

d) Ligation of isolated DNA fragments and transformation of *E. coli* HB 101 (cf. Figures 23 and 24)

Two low melting agarose gel blocks containing 0.1 pmoles of the 5.2 kb BamHI-BglII fragment of p31R/SS-TPAΔ2 and 0.2 pmoles of the 662 bp BamHI-BglII fragment from pBR322/PHO5Bam-Rsa 637, respectively, are mixed, liquified at 65°C and diluted to lower the agarose concentration to 0.3%. Ligation is carried out in 150 μl of 60 mM Tris · HCl pH 7.5, 10 mM $MgCl_2$, 5 mM DTT, 1 mM ATP and 800 units of T4 DNA ligase (Biolabs) at 15°C for 16 hours. 10 μl and 30 μl aliquots are mixed with 100 μl of transformation competent *E. coli* HB101 cells as described in Example 4a.

6 $amp^R$, transformed colonies are grown individually in LB medium containing 100 μg/ml ampicillin. Plasmid DNA is analyzed for size and orientation of the insert by cleavage with restriction endonuclease BamHI. A single clone with the correct orientation of the insert is referred to as p31/PHO5637C-TPA (cf. Figure 23)

Plasmid pBR322/PHO5Bam-Rsa 637 is also replaced by pBR322/PHO5Bam-Rsa 595, pBR322/PHO5 Bam-Rsa 811 and pBR322/PHO5Bam-Rsa 1312. Following the same procedure as described above single clones with inserts of expected size and orientation are isolated and referred to as p31/PHO5 595C-TPA, p31/PHO5 811C-TPA and p31/PHO5 1312C-TPA.

Linker C is also replaced by linker B of the following formula:

5'- CTAGATAAGAGATCTGC -3'
3'- TATTCTCTAGACG -5'

Phosphorylation of the synthetic oligodeoxynucleotides, annealment and ligation to the corresponding DNA fragments is as described above for linker C. The resulting clones are marked by the letter B and are referred to as p31/PHO5 595B-TPA, p31/PHO5 637B-TPA, p31/PHO5 811B-TPA and p31/PHO5 1312B-TPA. In these constructions linker B codes for the amino acid sequence Leu-Asp-Lys-Arg.

Linker C is also replaced by linker A of the following formula:

5'- AAGAGATCTGC -3'
3'- TTCTCTAGACG -5'

which codes for Lys-Arg in the final construction (Figure 24). Phosphorylation of the synthetic oligodeoxynucleotides and annealment are as described for linker C.

For linker addition 5 μg of XbaI cleaved pBR322/PHO5Bam-Rsa 637 are digested with 2 units of nuclease $S_1$ (sigma) in 50 μl of 30 mM sodium acetate pH 4.6, 200 mM NaCl and 1 mM $ZnSO_4$ for 40 min at 37°C to create blunt ends (see Figure 23). After phenol/chloroform extraction the DNA is precipitated with ethanol and is redissolved in $H_2O$ at a concentration of 0.25 mg/ml. 0.4 μg (120 pmoles) of phosphorylated, annealed linker A and 5 μg (1.5 pmoles) of XbaI/$S_1$ cleaved pBR322/PHO5Bam-Rsa 637 are blunt end ligated as described for linker C except for using 3.5 mM ATP. Further constructions are as described above. The resulting clones are identified by the letter A and referred to as p31/PHO5 595A-TPA, p31/PHO5 637A-TPA, p31/PHO5 811A-TPA and p31/PHO5 1312A-TPA.

Example 16

Subcloning of gene constructions in the high copy number yeast vector pJDB207 and transformation of *S. cerevisiae* GRF18

The constructions described in Examples 13—15 contain the PHO5 promoter with or without extensions into the coding region of the PHO5 gene, the TPA coding region with or without the prepro-sequence and the PHO5 transcription termination signals in a tandem array, all inserted in a pBR322 derived vector. BamHI-HindIII fragments containing the whole array are ligated to the 6.4 kb BamHI-HindIII fragment of pJDB207 as described for an analogous reaction in Example 10. Competent *E. coli* HB101 cells are transformed and several amp$^R$ colonies from each experiment are grown individually in LB medium with 100 µg/ml of ampicillin. Plasmid DNA is analyzed by cleavage with restriction endonucleases HindIII, BamHI and PstI.

Starting from plasmids p31/PHO5-TPA18, p31/PHO5-TPA42, p31R/SS-TPAΔ2, p31R/SS-TPAΔ3, p31/PHO5 595C-TPA, p31/PHO5 637C-TPA, p31/PHO5 811C-TPA, p31/PHO5 1312C-TPA, p31/PHO5 595B-TPA, p31/PHO5 637B-TPA, p31/P8O5 811B-TPA, p31/PHO5 1312B-TPA, p31/PHO5 595A-TPA, p31/PHO5 637A-TPA, p31/PHO5 811A-TPA and p31/PHO5 1312A-TPA the following clones with the correct inserts are obtained:

pJDB207/PHO5-TPA18
pJDB207/PHO5-TPA42
pJDB207R/PHO5-SS-TPAΔ2
pJDB207R/PHO5-SS-TPAΔ3
pJDB207/PHO5 595C-TPA
pJDB207/PHO5 637C-TPA
pJDB207/PHO5 811C-TPA
pJDB207/PHO5 1312C-TPA
pJDB207/PHO5 595B-TPA
pJDB207/PHO5 637B-TPA
pJDB207/PHO5 811B-TPA
pJDB207/PHO5 1312B-TPA
pJDB207/PHO5 595A-TPA
pJDB207/PHO5 637A-TPA
pJDB207/PHO5 811A-TPA
pJDB207/PHO5 1312A-TPA.

These plasmids are each introduced into *Saccharomyces cerevisiae* strain GRF18 as described in Example 11.

A single yeast colony from each of the yeast transformations is picked. The obtained clones are referred to as

*Saccharomyces cerevisiae* GRF18/pJDB207/PHO5-TPA18
*Saccharomyces cerevisiae* GRF18/pJDB207/PHO5-TPA42
*Saccharomyces cerevisiae* GRF18/pJDB207R/PHO5-SS-TPAΔ2
*Saccharomyces cerevisiae* GRF18/pJDB207R/PHO5-SS-TPAΔ3
*Saccharomyces cerevisiae* GRF18/pJDB207/PHO5 595C-TPA
*Saccharomyces cerevisiae* GRF18/pJDB207/PHO5 637C-TPA
*Saccharomyces cerevisiae* GRF18/pJDB207/PHO5 811C-TPA
*Saccharomyces cerevisiae* GRF18/pJDB207/PHO5 1312C-TPA
*Saccharomyces cerevisiae* GRF18/pJDB207/PHO5 595B-TPA
*Saccharomyces cerevisiae* GRF18/pJDB207/PHO5 637B-TPA
*Saccharomyces cerevisiae* GRF18/pJDB207/PHO5 811B-TPA
*Saccharomyces cerevisiae* GRF18/pJDB207/PHO5 1312B-TPA
*Saccharomyces cerevisiae* GRF18/pJDB207/PHO5 595A-TPA
*Saccharomyces cerevisiae* GRF18/pJDB207/PHO5 637A-TPA
*Saccharomyces cerevisiae* GRF18/pJDB207/PHO5 811A-TPA
*Saccharomyces cerevisiae* GRF18/pJDB207/PHO5 1312A-TPA

Yeast cells are grown, harvested and extracted as described in Examples 11 and 12. TPA activity in cell extracts is assayed as described in Example 12. The results are shown in Table 3.

TABLE 3

TPA activity of yeast strain *Saccharomyces cerevisiae* GRF18 transformed with different plasmids and assayed under derepressed conditions (low $P_i$):

| Plasmid | TPA activity (units/1 yeast cell culture/$OD_{600}$) |
|---|---|
| pJDB207/PHO5-TPA18 | 750 |
| pJDB207/PHO5-TPA42 | 700 |
| pJDB207R/PHO5-SS-TPAΔ2 | 600 |
| pJDB207R/PHO5-SS-TPAΔ3 | 650 |
| pJDB207/PHO5 595C-TPA | 900 |

Example 17

Chromosomal replacement of the PHO5 gene by the gene of TPA (see Figure 25)

The TPA protein coding region is placed into yeast chromosome II at the position of the protein coding region of PHO5. Experimentally, this is accomplished by cotransformation.

*Saccharomyces cerevisiae* strain GRF18 is cotransformed with 25 μg of plasmid p31/PHO5-TPA18, linearized by BamHI and HindIII restriction cuts, and 3 μg of yeast plasmid Yep13 (51). From 24 $Leu^+$ colonies obtained, one is $Pho5^-$ and at the same time produces approximately 35 units of TPA/1 yeast cell culture/$OD_{600}$. By growing this transformant on a leucine containing medium (YPD, 10 g/l Bacto Yeast Extract, 20 g/l Bacto Peptone, 20 g/l glucose) for 10 generations, $Leu^-$ segregants are obtained at a frequency of 10—20% which presumably have lost plasmid Yep13. By Southern analysis of total yeast DNA isolated from $Leu^-$ segregants (52) it is verified on the DNA level that the TPA protein coding sequences have replaced the PHO5 protein coding sequences leaving the flanking sequences on chromosome II unchaged, and that the strains are free of Yep13 plasmid DNA. One strain is selected and referred to as *Saccharomyces cerevisiae* GRF18(pho5-TPA).

Example 18

Strain improvement by mutation

The strain *Saccharomyces cerevisiae* GRF18/pJDB207/PHO5-TPA(1A) is further developed for TPA production by mutation and selection for low acid phosphatase activity.

*Saccharomyces cerevisiae* H243/pJDB207/PHO5-TPA(1A) a temperature-sensitive mutant of *Saccharomyces cerevisiae* GRF 18/pJDB207/PHO5-TPA(1A) is obtained by UV-irradiation (0.08 $\mu Wcm^{-2}$, 15 seconds, kill 99.2%). Colonies are grown on 1.2 M sorbitol osmotically stabilised YPD-medium (10 g/l Bacto Yeast Extract, 20 g/l Bacto Peptone, 20 g/l glucose, 20 g/l Bacto agar) and replica-plated onto YPD-medium without sorbitol. *Saccharomyces cerevisiae* H243 is isolated as a slow-growing colony.

Approximately $10^5$ cells of *Saccharomyces cerevisiae* H243/pJDB207/PHO5-TPA(1A) are plated onto YNB-agar (6.8 l Yeast Nitrogen Base without amino acids; 20 g/l glucose, 20 g/l Bacto Agar, supplemented with 20 mg/l histidine) and are directly UV-irradiated (0.08 $\mu Wcm^{-2}$ for 12 seconds). Kill is 98%. Colonies obtained from the surviving cells are replica-plated onto low $P_i$-medium and after 2 days stained for acid phosphatase activity by a soft-agar overlayer-assay (48). Acid phosphatase negative mutants are obtained with a mutation frequency of $3.2 \times 10^{-4}$. The strain with the highest TPA titer is selected and is referred to as *Saccharomyces cerevisiae* H423/pJDB207/PHO5-TPA(1A).

TPA activities of three different yeast strains transformed with pJDB207/PHO5-TPA(1A) are shown in Table 4.

TABLE 4

TPA activity of different transformed *Saccharomyces cerevisiae* strains under derepressed conditions (low $P_i$)

| | TPA activity (units/1 yeast cell culture/$OD_{600}$) |
|---|---|
| GRF18/pJDB207/PHO5-TPA(1A) | 625 |
| H243/pJDB207/PHO5-TPA(1A) | 1560 |
| H423/pJDB207/PHO5-TPA(1A) | 7200 |

Example 19

Production of TPA by a recombinant strain of the yeast *Saccharomyces cerevisiae* on a 30 l scale

*Saccharomyces cerevisiae* strain GRF18/pJDB207/PHO5-TPA(1A) carries a plasmid which includes a leucine marker allowing selective maintenance of the plasmid in the host organism, a structural gene for human TPA and the acid phosphatase PHO5 promoter which allows expression of the TPA gene in media with limiting amounts of inorganic phosphate (derepressed conditions).

The strain is maintained on agar slant cultures prepared with a defined medium lacking the amino acid leucine to ensure retention of the plasmid. Freshly inoculated slants are incubated for 24 hours at 30°C. The surface culture of one slant is resuspended in 3 ml pre-culture medium which is then transferred to the first shake flask pre-culture. The 500 ml flask has a single baffle and contains 100 ml pre-culture medium I having the following composition (values in g/l): L-asparagine, 2.0; L-histidine, 0.02; glucose, 10; $KH_2PO_4$, 1.0; $MgSO_4 \cdot 7\,H_2O$, 0.5; NaCl, 0.1; $CaCl_2 \cdot 2\,H_2O$, 0.1; vitamin solution, 5 ml/l and trace element solution, 5 ml/l.

The medium is adjusted to pH 7.2 using NaOH before sterilisation. The glucose, vitamins and trace elements are sterilised separately and added to the medium. The stock solutions for vitamins and trace elements have the following compositions (in g/l): Vitamins—biotin, 0.0002; calcium-D-panthothenat, 0.04; folic acid, 0.0002; nicotinic acid, 0.04; p-aminobenzoic acid, 0.02; pyridoxine hydrochloride, 0.04; riboflavin, 0.02; thiamine hydrochloride, 0.04; and inositol, 0.2 in 1 l of deionised water; trace elements—boric acid, 0.05; $CuSO_4 \cdot 5H_2O$, 0.004; KI, 0.01; $FeCl_3 \cdot 6H_2O$, 0.02; $MnSO_4 \cdot 4H_2O$, 0.04; $Na_2MoO_4 \cdot 2H_2O$, 0.02 and $ZnSO_4 \cdot 7H_2O$, 0.04 in 1 l of deionised water. The medium is prepared using deionised water.

The first pre-culture is incubated for 24 hours at 30°C on an orbital shaker with 5 cm throw at a speed of 250 rev/min.

The first pre-culture flask provides the inoculum for the second pre-culture flasks. These flasks receive an inoculum level of 1% v/v. They contain 100 ml pre-culture medium II having the following composition (in g/l): Yeast extract (Difco), 10.0; L-asparagine, 6.6; $KH_2PO_4$, 1.0; $MgSO_4 \cdot 7H_2O$, 1.0; L-histidine, 0.02 and D-glucose (monohydrate), 33.0.

The medium which is prepared using deionised water, has a pH value of approximately 6.0. The glucose is sterilised separately. Incubation conditions are identical with those for the first pre-culture. The culture broth from 3 such flasks are combined to provide a 1% v/v inoculum for the main production fermenter.

The production fermenter has a total volume of approximately 45 l, contains 4 baffles and a six-bladed disc turbine agitator with a diameter of 115 mm. The agitation rate is 600 rev/min, the overpressure 0.3 bar and the aeration rate is 0.5 vol/vol/min. The fermenter contains 30 l of a medium with the following composition (values in g/l): L-asparagine, 2.0; L-histidine, 0.02; $KH_2PO_4$, 0.03; $MgSO_4 \cdot 7H_2O$, 0.5; NaCl, 0.1; $CaCl_2 \cdot 2H_2O$, 0.1; KCl, 1.0; D-glucose (monohydrate), 20.0; vitamin solution, 5 ml/l and trace element solution, 5 ml/l. The medium is adjusted to pH 7.2 using NaOH before sterilization. The glucose, vitamins and trace elements are sterilised separately and added to the medium. Stock solutions of vitamins and trace elements have the same composition as those described for the pre-culture medium I.

The fermentation temperature is 30°C. The pH value falls to a value of 5.0 where it is controlled using NaOH. After fermenting for about 20 hours the maximum yield of TPA is reached. The optical density, which reaches 2—3 units, and the acid phosphatase activity are useful indications of the progress of the fermentation. The fermentation broth is cooled to 10°C prior to harvesting of the yeast cells.

Example 20

Recovery and purification of TPA and pro-TPA by monoclonal anti-TPA antibody chromatography

a) Cell extraction

The culture fluid (pH 4) of the 30 l fermentation (cf. Example 19) is cooled to 10°C and centrifuged using an Alfa-Laval BRPX-207 desludger centrifuge. The separated cells are resuspended in 2 l lysis buffer A (20 mM $NaH_2PO_4$, 80 mM $K_2HPO_4$, 150 mM NaCl, 0.01% Tween, 10 mM Benzamidine and 10 mM EDTA). The solution is cooled to 5°C and passed through a Dyno® Mill (type KDL-Pilot) at a feed rate of 10 l/h. The mill is equipped with 500 ml of glass beads of 0.5—0.75 m in diameter and is operated at 3000 rev/min. Prior to the application of the suspended cells the glass beads are washed with 300 ml lysis buffer A containing 30 mg BSA. The ruptured cell suspension is centrifuged (Sorvall rotor 653, 40 min. 9000 rpm) and the pellet is discarded.

b) DNA digestion and ammonium sulfate fractionation

5 mg DNAse is added to 2 l of cleared suspension and the mixture is stirred for 30 min at 4°C. Subsequently the solution is brought to 35% saturation with respect to $(NH_4)_2SO_4$ (w/v) and is stirred for 1 hour at 4°C. The solution is centrifuged as described above and the pellet containing all the TPA activity is resuspended in 1 l of buffer A containing 1% Tween and 0.1% SDS. The suspension is stirred for at least 1 h at 4°C.

c) Immunoaffinity chromatography

I) Purification of anti-TPA antibody

5 ml of Rabbit anti-TPA antiserum (provided by Dr. E. Reich, Friedrich-Miescher-Institut, Basel) are passed over a lysine sepharose column to remove the plasminogen from the serum.

The column is washed with 1—2 volumes of PBS pH 7.4 containing 100 mM NaCl, 2.7 mM KCl, 8.1 mM $Na_2HPO_4$ and 1.5 mM $KH_2PO_4$. The flow through and wash are pooled. The plasminogen depleted antiserum is precipitated by adding solid $(NH_4)_2SO_4$ to a final concentration of 50% (w/v). The solution is kept over night at 4°C and is then centrifuged (Sorvall GSA rotor 12000 rpm 20 min). The precipitate is dissolved in a small volume of PBS and dialysed against PBS containing 0.1% $NaN_3$. The IgG from this dialysed solution is purified on a protein A sepharose column (55).

II) Preparation of anti-TPA antibody column

13 mg of purified anti-TPA antibody are dialysed against 0.1 M MOPS pH 7.0. The antibody is coupled to 5 ml of activated Affigel 10 (Biorad) according to the manufacturer's instruction. The gel matrix is equilibrated with PBS containing 1% Tween 80, 0.1% SDS, 0.2 M NaCl, 10 mM Benzamidine and 0.1% $NaN_3$. The matrix is packed into a 5 ml column.

III) Purification of TPA by immunoaffinity chromatography

The redissolved pellet of the ammonium sulfate fractionation is centrifuged to remove particulate material (Sorvall GSA rotor, 30 min. 12,000 rpm) and subsequently applied to the affinity column at a flow rate of about 10 ml/hr at 4°C. After the total volume has passed through, the column is washed with 10 column volumes of PBS containing 0.05% Tween 80. The column is eluted using 0.1 M glycine—HCl pH 2.5 containing 0.1% Tween. Fractions of 2 ml are taken and assayed for TPA activity according to the method of Rånby (49). D-Val-Leu-Lys-pNA (Kabi S-2251) is used as substrate. Fractions having the highest activities are pooled, neutralized using 1 M Tris and concentrated by ultrafiltration using an Amicon flat bed membrane YM10. The TPA obtained in this way is about 90% pure and is predominantly in the one-chain form (pro-TPA) as evidenced by SDS-PAGE under reducing conditions. The apparent molecular weight under non-reducing conditions is about 60,000 on SDS-PAGE (see Figure A).

Figures A and B: SDS polyacrylamide gel electrophoresis of the yeast TPA gene product (Figure A) and its enzymatic activity on activity gel (casein plates) (Figure B).

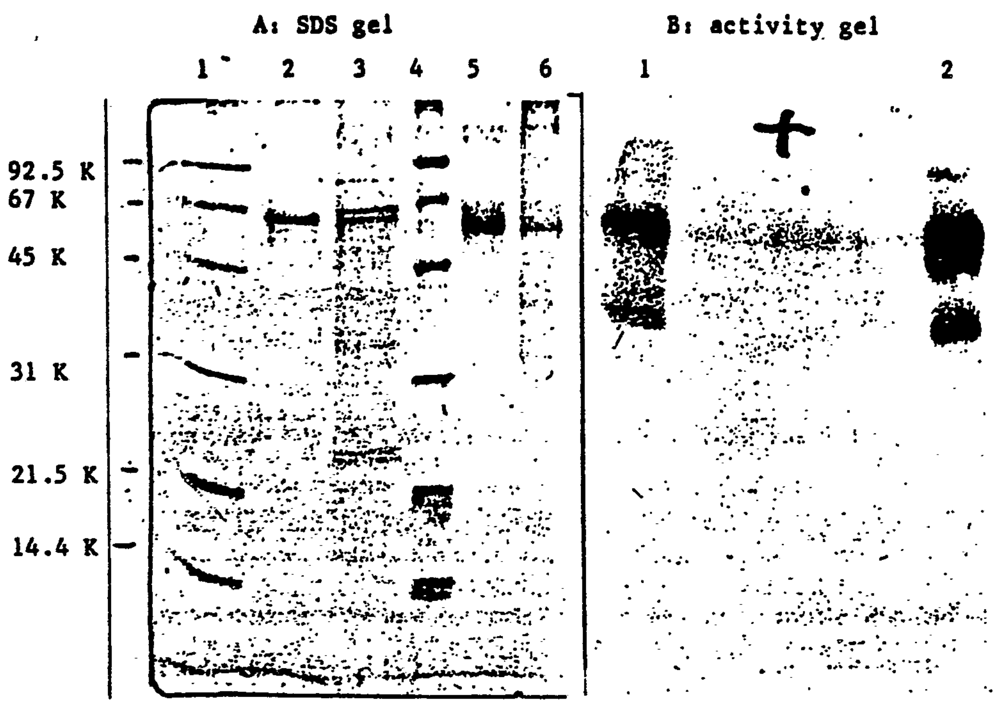


ad Figure A (SDS-PAGE):

lanes 1 and 4: standard proteins (size markers)

lanes 2 and 5: melanoma TPA

lanes 3 and 6: yeast TPA

lanes 1—3 under reducing conditions

lanes 4—6 under non-reducing conditions

The samples were run on a 12.5% gel and stained for protein using Coomassie brilliant blue.

ad Figure B (activity gel, c.f. Example 23):
lane 1: melanoma TPA
lane 2: yeast TPA
Casein-agar-plasminogen overlay (reference 56).

Example 21
Recovery and purification of TPA and pro-TPA by DE-3 affinity chromatography
a. Preparation of a DE-3 Sepharose® column
26 mg of purified DE-3 inhibitor from *Erythrina latissima* (16) are coupled to 5 ml of cyanogen bromide activated Sepharose 4b® (Pharmacia) according to the manufacturer's instructions. The matrix is equilibrated with phosphate buffered saline ("PBS") pH 7.4 containing 0.4 M NaCl, 0.1% Triton X-100® and 0.02% sodium azide. The matrix is then packed into a 5 ml column.

b. Chromatographical purification of TPA and pro-TPA on DE-3 Sepharose 4b®
Cell extracts (cf. Example 20a) are made 0.4 M with respect to NaCl and 0.1% with respect to Triton X-100® and filtered through a 0.45 µm membrane (Millipore). The solution is then applied to the DE-3 Sepharose® column (see above) at a flow rate of 45 ml/hr at room temperature and the effluent is discarded. After the total volume of cell extract has passed through, the column is washed with approximately 50 ml of PBS containing 0.4 M NaCl and 0.1% Triton X-100®, and 2 ml fractions are collected at 4°C. The protein content of each fraction is determined by measuring the UV absorbance at 280 nm. The adsorbed protein is found to be eluted as a sharp peak. Fractions containing the highest UV absorbance and highest fibrinolytic activity as determined in the $^{125}$I-fibrin assay (47) are pooled to give 8 ml of solution which is stored at −20°C. This represents aproximately 70—80% of the total activity applied to the column. Fractions containing lower activity are pooled separately. The total recovery of activity in both pools usually amounts to 90—100%.

c. Chromatographical purification of pro-TPA on DE-3 Sepharose 4b® in the presence of a proteinase inhibitor
Chromatography of yeast cell extracts is carried out in a similar manner as described in Example 21b, except that basic pancreatic trypsin inhibitor (BPTI) at 0.1 KIU/ml is included in the procedure. Using the fluorometric assay with Cbz-Gly-Gly-Arg-AMC as substrate (37), the TPA and pro-TPA contents of the purified solution are determined. 90% of the TPA is in the pro-enzyme form and 10% is in the active form. Thus, the conversion of pro-TPA to TPA suring the purification procedure is inhibited by BPTI.

Example 22
Separation of pro-TPA from TPA
The solution of TPA and pro-TPA obtained as described in Example 21c is adjusted to pH 8.0 with 0.1 M Tris · HCl containing 0.1% Triton X-100® and rendered 1 mM with respect to diisopropylfluorophosphate. After incubation at 37°C for 4 hours, the mixture is passed through a 5 ml DE-3 Sepharose® column (cf. Example 21a). The effluent containing the irreversibly inhibited TPA is discarded. The column is washed with 6 column volumes of PBS containing 0.4 M NaCl and 0.1% Triton X-100® and subsequently eluted with PBS containing 1.6 M KSCN, 0.4 M NaCl and 0.1% Triton X-100® as described in Example 21b. Fractions showing the highest UV absorbance are pooled. The pool contains pro-TPA in substantially pure form as no amidolytic activity is detectable in the fluorometric assay using Cbz-Gly-Gly-Arg-AMC (37) as substrate. The amidolytic as well as the fibrinolytic activity can be re-established by treatment with plasmin which converts pro-TPA into the active enzyme.

Example 23
Properties of the yeast TPA gene product
a. Enzymatic properties
The yeast TPA gene product obtained according to Example 19 and purified by DE-3 affinity chromatography (cf. Example 21b) is enzymatically active as shown by the cleavage of zymogen plasminogen to plasmin in the absence and in the presence of fibrin. Enzymatic activity can be shown by the colorimetric assays described by Rânby (49) (cf. Example 12) and by Verheijen et al. (60), on casein plates and fibrin plates (56) and in a $^{125}$J-fibrinogen solid phase assay (47). The enzymatic activity on casein plates is shown in Figure B (cf. Example 20cIII).
The enzymatic activity of the obtained TPA is greatly stimulated by fibrin. The TPA which has been partially purified by DE-3 affinity chromatography is assayed in a parabolic rate assay (cf. Rânby (49)). Taking the reaction rate ΔE versus $\Delta t^2$ between 3 to 5 minutes as a measure of enzyme activity the stimulation by optimal concentrations of fibrin (ca. 100 µg/ml) is 10—20 fold. The effect can be seen with fibrin fragments obtained by CNBr digestion (57) or with bathroxobin treated fibrinogen (58). When testing urokinase under the same conditions no stimulation of its activity by fibrin fragments occurs.

b. Binding of TPA of fibrin plates

Applying the fibrin binding test described by Rijken et al. (2) the obtained TPA which has been purified by DE-3 affinity chromatography is found to be bound to a great extent to fibrin clots. In contrast thereto urokinase does not bind significantly to fibrin. Therefore a striking difference between the yeast TPA gene product obtained and urokinase is that the former absorbs to fibrin which results in a marked enhancement of the activation of plasminogen (59).

Example 24

Glycosylation state of the yeast TPA gene product

In order to see whether or not the TPA produced by yeast is glycosylated, its behaviour on a concanavalin-A Sepharose® column (2) is analysed.

A TPA fraction partially purified by affinity chromatography on the DE-3 Sepharose® column (see Example 21b) is used. This fraction has an activity of 11.5 FU/ml which corresponds to 2.1 µg/ml of TPA protein.

The active fraction (3 ml) is applied to a concanavalin-A Sepharose® column (0.5 ml bed volume), equilibrated with 0.1 M Tris·HCl, pH 7.5, containing 1 M NaCl and 0.01% (v/v) Tween 80®.

The proteins in the flow-through are collected in only one fraction (≃3 ml). After washing the column with 6 ml of equilibration buffer, the elution of the proteins bound to the matrix is performed in four steps with α-methyl mannoside and KSCN added to the equilibration buffer. The four steps are carried out successively with 0.5 ml each of the following solutions:

1. 50 mM α-methyl mannoside+0.25 M KSCN in equilibration buffer
2. 100 mM α-methyl mannoside+0.50 M KSCN in equilibration buffer
3. 200 mM α-methyl mannoside+1 M KSCN in equilibration buffer
4. 300 mM α-methyl mannoside+2 M KSCN in equilibration buffer

A significant TPA activity is recovered at steps 2 and 3 but no activity is released with low concentrations of salt and sugar (step 1).

Also some TPA activity is found in the flow-through fraction (see above).

These results show that TPA is glycosylated in yeast. However, activity is also found in the flow-through fraction which indicates that not all of the TPA synthesized in yeast is fully glycosylated which might be due to the overproduction situation in low $P_i$ medium.

Example 25

Pharmaceutical preparation for parenteral administration

A TPA and/or pro-TPA containing solution obtained as described in Examples 20 to 22, is dialysed against 0.3 molar sodium chloride containing 0.01% Tween 80® and stored at −80°C. Prior to administration the concentration is adjusted to 75 µg/ml of total TPA (i.e. TPA or pro-TPA or TPA plus pro-TPA) and 0.3 M NaCl. The solution is sterilised by filtration through a 0.22 µm membrane filter.

This procedure is suitable for the preparation of solutions of TPA, pro-TPA or TPA plus pro-TPA for parenteral, such as intravenous, administration.

References

1. S. Thorsen et al., Thromb. Diath. Haemorrh. *28*, 65 (1972)
2. D. C. Rijken and D. Collen, J. Biol. Chem. *256*, 7035 (1981)
3. D. C. Rijken et al., J. Biol. Chem. *257*, 2920 (1982)
4. P. Wallén et al., Progr. Fibrin. *5*, 16 (1982)
5. E. L. Wilson et al., Cancer Res. *40*, 933 (1980)
6. E. L. Wilson et al., Blood *61*, 568 (1983)
7. D. Pennica et al., Nature *301*, 214 (1983)
8. T. Edlund et al., Proc. Natl. Acad. Sci. USA *80*, 349 (1983)
9. Perlman et al., Proc. Natl. Acad. Sci. USA *79*, 781 (1982)
10. A. M. Maxam et al., in "Methods in Enzymology", vol. 65, p. 499, New York 1980
11. J. Lodder, "The Yeasts", Amsterdam 1971
12. A. Hinnen et al., Proc. Natl. Acad. Sci. USA *75*, 1929 (1978)
13. A. Hinnen et al., Curr. Top. Microbiol. Immun. *96*, 101 (1982)
14. A. Jimenez et al., Nature *287*, 869 (1980)
15. European Patent Application No. 23860
16. F. J. Joubert et al., Hoppe-Seyler's Zeitschr. Physiol. Chem. *302*, 531 (1981)
17. M. V. Olson et al., J. Mol. Biol. *132*, 387 (1979)
18. H. Meyer, FEBS Lett. *90*, 341 (1978)
19. B. Hohn et al. in "Genetic Engineering, Vol. 2, p. 169, New York 1980
20. B. Hohn in "Methods in Enzymology", Vol. 68, p, 299, New York 1979
21. A. Hinnen et al. in "Eukaryotic Gene Regulation", Vol. *14*, p. 43, New York 1979
22. J. D. Beggs in "Genetic Engineering, Vol. *2*, p. 175, New York 1981

23. J. Messing, in the 3rd Cleveland Symposium on Macromolecules: Recombinant DNA (ed. A. Walton), Elsevier, Amsterdam 1981, p. 143
24. M. Mandrel et al., J. Mol. Biol. *53*, 159 (1970)
25. A. J. Berk et al., Cell *12*, 721 (1977)
26. D. S. Holmes et al., Anal. Biochem. *144*, 193 (1981)
27. P. M. Lizardi et al., Anal. Biochem. *96*, 116 (1979)
28. Y. Nagamine et al., Cell *32*, 1181 (1983)
29. J. B. Gurdon et al., J. Embryol. Exp. Morphol. *36*, 523 (1976)
30. M. W. McDonnel et al., J. Mol. Biol. *110*, 119 (1977)
31. G. Deng et al., Nucl. Acids Res. *9*, 4173 (1981)
32. L. Villa-Komaroff et al., Proc. Natl. Acad. Sci. USA *75*, 3727 (1978)
33. D. A. Morrison in "Methods in Enzymology", Vol. *68*, 326 (1979)
34. T. Edlund et al., Proc. Natl. Acad. Sci. USA *80*, 349 (1983)
35. H. Ito et al., Nucl. Acids Res. *10*, 1755 (1982)
36. H. C. Birnboim et al., Nucl. Acids Res. *7*, 1513 (1979)
37. M. Zimmerman et al., Proc. Natl. Acad. Sci. USA *75*, 750 (1978)
38. J. Messing in "M13 cloning/Dideoxy Sequencing" (EMBO-course manual), 1981
39. in manual "M13 cloning and DNA sequencing system", New England Biolabs
40. K. Itakura et al., J. Am. Chem. Soc. *97*, 7327 (1975)
41. J. F. M. de Rooij et al., Recl. Trav. Chim. Pays-Bas *98*, 537 (1979)
42. Molecular cloning. A laboratory Manual (eds. T. Maniatis, E. F. Fritsch, J. Sambrook), Cold Spring Harbor Lab. 1982, p. 177
43. G. F. Hong, Bioscience Reports *1*, 243 (1981)
44. A. Toh-e et al., J. Bacteriol. *113*, 727 (1973)
45. S. V. Suggs et al., in "Developmental biology using purified genes", ICNUCLA symposium on molecular and cellular biology, vol. 23 (ed. D. D. Brown and D. F. Fox), p. 683 (1981)
46. M. Smith, in "Methods of RNA and DNA sequencing" (ed. S. M. Weissmann), Praeger Scientific, New York 1983
47. E. L. Wilson et al., Int. J. Cancer *22*, 390 (1978)
48. B. Meyhack et al., EMBO Journal *1*, 675 (1982)
49. M. Rânby. Biochim. Biophys. Acta *704*, 461 (1982)
50. Hicks et al., Cold Spring Harbor Symposium on Quantitative Biology, Vol. XLIII, p. 1305 (1979)
51. Broach et al., Gene *8*, 121 (1979)
52. Struhl et al., Proc. Natl. Acad. Sci. USA *76*, 1035 (1979)
53. F. Sanger et al., Proc. Natl. Acad. Sci. USA *74*, 5463 (1977)
54. J. Kurjan et al., Cell *30*, 933 (1982)
55. P. L. Ey et al., Immunochem. *15*, 429 (1978)
56. J. Jespersen et al., Haemostasis *18*, 301 (1983)
57. C. Zamarrron et al., J. Biol. Chem. *259*, 2080 (1984)
58. J. Chmielewska et al., Thrombosis Research *31*, 427 (1983)
59. P. Wallén, Progr. Chem. Fibrinolysis Thrombolysis, *3*, 167 (1978)
60. J. H. Verheijen et al., Thromb. Haemost. *48*, 266 (1982)


IN THE FIGURES OF THE ACCOMPANYING DRAWINGS, THE SYMBOLS USED HAVE THE FOLLOWING MEANINGS:

| | | | |
|---|---|---|---|
| | pBR 322 SEQUENCES | t | TRANSCRIPTION TERMINATION REGION |
| | YEAST CHROMOSOMAL DNA DERIVED FROM PH03, PH05 REGION | | DELETION OF A RESTRICTION SITE |
| | JEAST 2μ PLASMID DNA | | TPA GENE |
| | JEAST CHROMOSOMAL DNA DERIVED FROM LEU 2 REGION | | DELETION IN pBR 322 |
| | | | RESTRICTION SITE |
| $amp^R$ | AMPICILLIN RESISTANCE GENE | | |
| $tet^R$ | TETRACYCLINE RESISTANCE GENE | | DIRECTION OF TRANSCRIPTION |
| p | PROMOTER REGION | | LINKER DNA STRETCH |

**Claims for the Contracting States: BE, CH, DE, FR, GB, IT, LI, LU, NL, SE**

1. A yeast hybrid vector comprising the yeast PHO5 promoter operably linked to a DNA sequence consisting of a signal sequence linked in frame to the human tissue plasminogen activator (TPA) coding region, and a DNA sequence containing transcription termination signals of a yeast gene.

2. A yeast hybrid vector according to Claim 1, comprising the PHO5 signal sequence linked in frame to the human TPA coding region.

3. A yeast hybrid vector according to Claim 1, comprising the TPA prepro-sequence linked in frame to the human TPA coding region.

4. A yeast hybrid vector according to Claim 1 wherein the yeast PHO5 promoter is extended into the PHO5 coding region which is linked to the human TPA coding region via a synthetic linker containing processing sites for a yeast endopeptidase.

5. A DNA segment comprising the yeast PHO5 promoter operably linked to a DNA sequence consisting of the PHO5 signal sequence linked in frame to the gene for human tissue plasminogen activator, and a DNA sequence containing transcription termination signals of the yeast PHO5 gene.

6. A transformed yeast strain selected from the group consisting of a yeast strain transformed with a hybrid vector comprising the yeast PHO5 promoter operably linked to a DNA sequence consisting of a signal sequence linked in frame to the human tissue plasminogen activator coding region, and a DNA sequence containing transcription termination signals of a yeast gene, and a yeast strain with the gene for human tissue plasminogen activator stably integrated in the yeast chromosome and being under the control of the chromosomal yeast PHO5 promoter, and mutants thereof.

7. A yeast transformed with a hybrid vector according to any one of Claims 1 to 4.

8. A transformed yeast according to Claim 6, wherein the yeast is *Saccharomyces cerevisiae*.

9. A method for producing TPA, pro-TPA or mixtures thereof wherein the TPA and pro-TPA is present in glycosylated or unglycosylated form or in a mixture of these forms, comprising the steps of culturing under appropriate nutrient conditions a yeast strain transformed with a hybrid vector comprising the yeast PHO5 promoter operably linked to a DNA sequence consisting of a signal sequence linked in frame to the human tissue plasminogen activator coding region, and a DNA sequence containing transcription termination signals of a yeast gene, or a yeast strain with the gene for human tissue plasminogen activator stably integrated in the yeast chromosome and being under the control of the chromosomal yeast PHO5 promoter, or a mutant of such a yeast strain, and isolating and purifying said proteins, and, if desired, separating a mixture of TPA and pro-TPA obtained into the individual components, and, for the production of TPA which is free of pro-TPA, converting the produced pro-TPA into TPA, and, if desired, separating glycosylated from unglycosylated products obtained in a mixture and, for the production of unglycosylated protein which is free of glycosylated protein, removing glycosyl residues in the obtained proteins.

10. A method according to Claim 9 for producing TPA, pro-TPA or mixtures thereof wherein the TPA and pro-TPA is present in glycosylated or unglycosylated form or in a mixture of these forms, comprising the steps of culturing under appropriate nutrient condition a yeast strain transformed with a hybrid vector comprising the yeast PHO5 promoter operably linked to a DNA sequence consisting of a signal sequence linked in frame to the human TPA coding region and a DNA sequence containing transcription termination signals of a yeast gene, and isolating and purifying said proteins, and, if desired, separating a mixture of TPA and pro-TPA obtained into the individual components, and, for the production of TPA which is free of pro-TPA, converting the produced pro-TPA into TPA, and, if desired, separating glycosylated from unglycosylated products obtained in a mixture and, for the production of unglycosylated protein which is free of glycosylated protein, removing glycosyl residues in the obtained proteins.

11. A method according to any one of Claims 9 or 10, wherein the yeast is *Saccharomyces cerevisiae*.

12. A method according to Claim 10, wherein TPA and pro-TPA and mixtures thereof are isolated from culture fluids by selective adsorption on a DE-3 Sepharose column.

13. A method according to Claim 10, wherein TPA and pro-TPA and mixtures thereof are isolated from culture fluids by affinity chromatography using monoclonal antibodies.

14. A method according to Claim 10, wherein a protease inhibitor is included during the purification procedure, and chromatography on a DE-3 Sepharose column is performed in the presence of an inhibitor which selectively binds only TPA so as to prepare pro-TPA which is substantially free of TPA.

15. A method according to Claim 10, wherein glycosylated and unglycosylated products obtained are separated by chromatography on a concanavalin-A Sepharose column.

**Claims for the Contracting State: AT**

1. A method for producing a yeast hybrid vector comprising the yeast PHO5 promoter operably linked to a DNA sequence consisting of a signal sequence linked in frame to the human tissue plasminogen activator (TPA) coding region, and a DNA sequence containing transcription termination signals of a yeast gene, which method comprises introducing into a vector DNA said promoter, said signal sequence, said TPA coding region and said terminator sequence in the predetermined order.

2. A method according to Claim 1 wherein the hybrid vector comprises the PHO5 signal sequence linked in frame to the human TPA coding region.

3. A method according to Claim 1, wherein the hybrid vector comprises the TPA prepro-sequence linked in frame to the human TPA coding region.

4. A method according to Claim 1 wherein the yeast PHO5 promoter is extended into the PHO5 coding region which is linked to the human TPA coding region via a synthetic linker containing processing sites for a yeast endopeptidase.

5. A method for producing a transformed yeast strain selected from the group consisting of a yeast strain transformed with a hybrid vector comprising the yeast PHO5 promoter operably linked to a DNA sequence consisting of a signal sequence linked in frame to the human tissue plasminogen activator coding region, and a DNA sequence containing transcription termination signals of a yeast gene, and a yeast strain with the gene for human tissue plasminogen activator stably integrated in the yeast chromosome and being under the control of the chromosomal yeast PHO5 promoter, which method comprises transforming yeast with said hybrid vector or with a DNA segment comprising the yeast PHO5 promoter operably linked to a DNA sequence consisting of the PHO5 signal sequence linked in frame to the gene for human tissue plasminogen activator, and a DNA sequence containing transcription termination signals of the yeast PHO5 gene.

6. A method according to Claim 5 comprising transforming yeast with a hybrid vector obtainable according to any one of Claims 1 to 4.

7. A method according to Claim 5 wherein the yeast is *Saccharomyces cerevisiae.*

8. A method for producing TPA, pro-TPA or mixtures thereof wherein the TPA and pro-TPA is present in glycosylated or unglycosylated form or in a mixture of these forms, comprising the steps of culturing under appropriate nutrient conditions a yeast strain transformed with a hybrid vector comprising the yeast PHO5 promoter operably linked to a DNA sequence consisting of a signal sequence linked in frame to the human tissue plasminogen activator coding region, and a DNA sequence containing transcription termination signals of a yeast gene, or a yeast strain with the gene for human tissue plasminogen activator stably integrated in the yeast chromosome and being under the control of the chromosomal yeast PHO5 promoter, or a mutant of such a yeast strain, and isolating and purifying said proteins, and, if desired, separating a mixture of TPA and pro-TPA obtained into the individual components, and, for the production of TPA which is free of pro-TPA, converting the produced pro-TPA into TPA, and, if desired, separating glycosylated from unglycosylated products obtained in a mixture and, for the production of unglycosylated protein which is free of glycosylated protein, removing glycosyl residues in the obtained proteins.

9. A method according to Claim 8 for producing TPA, pro-TPA or mixtures thereof wherein the TPA and pro-TPA is present in glycosylated or unglycosylated form or in a mixture of these forms, comprising the steps of culturing under appropriate nutrient conditions a yeast strain transformed with a hybrid vector comprising the yeast PHO5 promoter operably linked to a DNA sequence consisting of a signal sequence linked in frame to the human TPA coding region and a DNA sequence containing transcription termination signals of a yeast gene, and isolating and purifying said proteins, and, if desired, separating a mixture of TPA and pro-TPA obtained into the individual components, and, for the production of TPA which is free of pro-TPA, converting the produced pro-TPA into TPA, and, if desired, separating glycosylated from unglycosylated products obtained in a mixture and, for the production of unglycosylated protein which is free of glycosylated protein, removing glycosyl residues in the obtained proteins.

10. A method according to any one of Claims 8 or 9, wherein the yeast is *Saccharomyces cerevisiae.*

11. A method according to Claim 9, wherein TPA and pro-TPA and mixtures thereof are isolated from culture fluids by selective adsorption on a DE-3 Sepharose column.

12. A method according to Claim 9, wherein TPA and pro-TPA and mixtures thereof are isolated from culture fluids by affinity chromatography using monoclonal antibodies.

13. A method according to Claim 9, wherein a protease inhibitor is included during the purification procedure, and chromatography on a DE-3 Sepharose column is performed in the presence of an inhibitor which selectively binds only TPA so as to prepare pro-TPA which is substantially free of TPA.

14. A method according to Claim 9, wherein glycosylated and unglycosylated products obtained are separated by chromatography on a concanavalin-A Sepharose column.

**Patentansprüche für die Vertragsstaaten: BE, CH, DE, FR, GB, IT, LI, LU, NL, SE**

1. Hefe-Hybridvektor umfassend den Hefe-PHO5-Promotor, der operabel verknüpft ist mit einer DNA-Sequenz bestehend aus einer Signalsequenz, die im Rahmen mit der humanen Gewebsplasminogenaktivator (GPA) codierenden Region verknüpft ist, und eine DNA-Sequenz, die Transkriptionsterminationssignale eines Hefe-Gens enthält.

2. Hefe-Hybridvektor nach Anspruch 1, umfassend die PHO5-Signalsequenz, die im Rahmen mit der humanen GPA codierenden Region verknüpft ist.

3. Hefe-Hybridvektor nach Anspruch 1, umfassend die GPA-Präpro-Sequenz, die im Rahmen mit der humanen GPA codierenden Region verknüpft ist.

4. Hefe-Hybridvektor nach Anspruch 1, worin sich der Hefe-PHO5-Promotor in die PHO5-codierende Region erstreckt, die mit der humanen GPA codierenden Region über einen synthetischen Linker verknüpft ist, der Erkennungsstellen für eine Hefe-Endopeptidase enthält.

5. DNA-Segment, umfassend den Hefe-PHO5-Promotor, der operabel verknüpft ist mit einer DNA-Sequenz bestehend aus der PHO5-Signalsequenz, die im Rahmen mit dem Gen für humanen Gewebsplasminogenaktivator verknüpft ist, und eine DNA-Sequenz, die Transkriptionsterminationssignale der Hefe-PHO5-Gens enthält.

6. Transformierter Hefestamm ausgewählt aus der Gruppe bestehend aus einem Hefestamm, der transformiert wurde mit einem Hybridvektor umfassend den Hefe-PHO5-Promotor, der operabel verknüpft ist mit einer DNA-Sequenz bestehend aus einer Signalsequenz, die im Rahmen mit der humanen Gewebsplasminogenaktivator codierenden Region verknüpft ist, und eine DNA-Sequenz, die Transkriptionsterminationssignale eines Hefe-Gens enthält, und einem Hefestamm mit dem Gen für humanen Gewebsplasminogenaktivator, das im Hefe-Chromosom stabil integriert und unter der Kontrolle des chromosomalen Hefe-PHO5-Promotors ist, und Mutanten davon.

7. Hefe, die mit einem Hybridvektor nach einem der Ansprüche 1 bis 4 transformiert ist.

8. Transformierte Hefe nach Anspruch 6, wobei die Hefe *Saccharomyces cerevisiae* ist.

9. Verfahren zur Herstellung von GPA, Pro-GPA oder deren Mischungen, wobei der GPA und Pro-GPA in glycosylierter oder unglycosylierter Form oder in einer Mischung dieser Formen vorliegt, umfassend die Schritte der unter geeigneten Nährstoffbedingungen erfolgenden Kultivierung eines Hefestammes, der transformiert wurde mit einem Hybridvektor umfassend den Hefe-PHO5-Promotor, der operabel verknüpft ist mit einer DNA-Sequenz bestehend aus einer Signalsequenz, die im Rahmen mit der humanen Gewebsplasminogenaktivator codierenden Region verknüpft ist, und eine DNA-Sequenz, die Transkriptionsterminationssignale eines Hefe-Gens enthält, oder eines Hefestammes mit dem Gen für humanen Gewebsplasminogenaktivator, das in das Hefe-Chromosom stabil integriert und unter Kontrolle des chromosomalen Hefe-PHO5-Promotors ist, oder einer Mutante eines solchen Hefestammes, Isolierung und Reinigung der genannten Proteine und, falls gewünscht, Separieren eines erhaltenen Mischung von GPA und Pro-GPA in die einzelnen Komponenten, und zur Herstellung von GPA, der frei ist von Pro-GPA, Umwandlung des gebildeten Pro-GPA in GPA und, falls gewünscht, Separieren glycosylierter von unglycosylierten Produkten, die in einer Mischung erhalten wurden, und Entfernung der Glycosylreste in den erhaltenen Proteinen zur Herstellung von unglycosyliertem Protein, das frei ist von glycosyliertem Protein.

10. Verfahren nach Anspruch 9 zur Herstellung von GPA, Pro-GPA und deren Mischungen, worin der GPA und Pro-GPA in glycosylierter oder unglycosylierter Form oder einer Mischung dieser Formen vorliegt, umfassend die Schritte der unter geeigneten Nährstoffbedingungen erfolgenden Kultivierung eines Hefestammes, der transformiert wurde mit einem Hybridvektor umfassend den Hefe-PHO5-Promotor, der operabel verknüpft ist mit einer DNA-Sequenz bestehd aus einer Signalsequenz, die im Rahmen verknüpft ist mit der humanen GPA codierenden Region, und eine DNA-Sequenz, die Transkriptionsterminationssignale eines Hefe-Gens enthält, und Isolierung und Reinigung der genannten Proteine und, falls gewünscht, Separierung einer erhaltenen Mischung von GPA und Pro-GPA in die einzelnen Komponenten, und zur Herstellung von GPA, der frei ist von Pro-GPA, Umwandlung des gebildeten Pro-GPA in GPA, falls gewünscht, Separieren der glycosylierten von den unglycosylierten Produkte, die in einer Mischung erhalten wurden, und zur Herstellung von unglycosyliertem Protein, das frei ist von glycosyliertem Protein, Entfernung der Glycosylreste in den erhaltenen Proteinen.

11. Verfahren nach einem der Ansprüche 9 oder 10, worin die Hefe *Saccharomyces cerevisiae* ist.

12. Verfahren nach Anspruch 10, worin GPA und Pro-GPA und deren Mischungen aus Kulturflüssigkeiten durch selektive Adsorption an einer DE-3 Sepharose-Säule isoliert werden.

13. Verfahren nach Anspruch 10, worin GPA und Pro-GPA und deren Mischungen aus Kulturflüssigkeiten durch Affinitätschromatographie unter Verwendung monoklonaler Antikörper isoliert werden.

14. Verfahren nach Anspruch 10, worin ein Proteaseinhibitor während der Reinigungsprozedur einbezogen wird und Chromatographie auf einer DE-3 Sepharose-Säule in Gegenwart eines Inhibitors durchgeführt wird, der selektiv nur GPA bindet, um Pro-GPA herzustellen, der im wesentlichen frei von GPA ist.

15. Verfahren nach Anspruch 10, worin erhaltene glycosylierte und unglycosylierte Produkte durch Chromatographie auf einer Concanavalin-A Sepharose-Säule getrennt werden.

**Patentansprüche für den Vertragsstaat: AT**

1. Verfahren zur Herstellung eines Hefe-Hybridvektors umfassend den Hefe-PHO5-Promotor, der operabel verknüpft ist mit einer DNA-Sequenz bestehend aus einer Signalsequenz, die im Rahmen mit der humanen Gewebsplasminogenaktivator (GPA) codierenden Region verknüupft ist, und eine DNA-Sequenz, die Transkriptionsterminationssignale eines Hefe-Gens enthält, welches Verfahren umfaßt das Einführen des genannten Promotors, der genannten Signalsequenz, der genannten GPA codierenden region und der genannten Terminatorsequenz in eine Vektor-DNA in der festgesetzten Reihenfolge.

2. Verfahren nach Anspruch 1, worin der Hybridvektor die PHO5-Signalsequenz umfaßt, die im Rahmen mit der humanen GPA codierenden Region verknüpft ist.

3. Verfahren nach Anspruch 1, worin der Hybridvektor die GPA-Präpro-Sequenz umfaßt, die im Rahmen mit der humanen GPA codierenden Region verknüpft ist.

4. Verfahren nach Anspruch 1, worin sich der Hefe-PHO5-Promotor in die PHO5 codierende Region

erstreckt, die mit der humanen GPA codierenden Region über einen synthetischen Linker verknüpft ist, der Erkennungsstellen für eine Hefe-Endopeptidase enthält.

5. Verfahren zur Herstellung eines transformierten Hefestammes ausgewählt aus der Gruppe bestehend aus einem Hefestamm, der transformiert wurde mit einem Hybridvektor umfassend den Hefe-PHO5-Promotor, der operabel verknüpft ist mit einer DNA-Sequenz bestehend aus einer Signalsequenz, die im Rahmen mit der humanen Gewebsplasminogenaktivator codierenden Region verknüpft ist, und eine DNA-Sequenz, die Transkriptionsterminationssignale eines Hefe-Gens enthält, und einem Hefestamm mit dem Gen für humanen Gewebsplasminogenaktivator, das im Hefe-Chromsom stabil integriert und unter der Kontrolle des chromosomalen Hefe-PHO5-Promotors ist, welches Verfahren umfaßt das Transformieren von Hefe mit dem genannten Hybridvektor oder mit einem DNA-Segment umfassend den Hefe-PHO5-Promotor, der operabel verknüpft ist mit einer DNA-Sequenz bestehend aus der PHO5-Signalsequenz, die im Rahmen mit dem Gen für humanen Gewebsplasminogenaktivator verknüpft ist, und eine DNA-Sequenz, die Transkriptionsterminationssignale des Hefe-PHO5-Gens enthält.

6. Verfahren nach Anspruch 5, umfassend das Transformieren von Hefe mit einem Hybridvektor, der nach einem der Ansprüche 1 bis 4 erhalten werden kann.

7. Verfahren nach Anspruch 5, bei dem die Hefe *Saccharomyces cerevisiae* ist.

8. Verfahren zur Herstellung von GPA, Pro-GPA oder deren Mischungen, wobei der GPA und Pro-GPA in glycosylierter oder unglycosylierter Form oder in einer Mischung dieser Formen vorliegt, umfaßend die Schritte der unter geeigneten Nährstoffbedingungen erfolgenden Kultivierung eines Hefestammes, der transformiert wurde mit einem Hybridvektor umfassend den Hefe-PHO5-Promotor, der operabel verknüpft ist mit einer DNA-Sequenz bestehend aus einer Signalsequenz, die im Rahmen mit der humanen Gewebsplasminogenaktivator codierenden Region verknüpft ist, und eine DNA-Sequenz, die Transkriptionsterminationssignale eines Hefe-Gens enthält, oder eines Hefestammes mit dem Gen für humanen Gewebsplasminogenaktivator, das in das Hefe-Chromosom stabil integriert und unter Kontrolle des chromosomalen Hefe-PHO5-Promotors ist, oder einer Mutante eines solchen Hefestammes, Isolierung und Reinigung der genannten Proteine und, falls gewünscht, Separieren einer erhaltenen Mischung von GPA und Pro-GPA in die einzelnen Komponenten, und zur Herstellung von GPA, der frei ist von Pro-GPA, Umwandlung des gebildeten Pro-GPA in GPA und, falls gewünscht, Separieren glycosylierter von unglycosylierten Produkten, die in einer Mischung erhalten wurden, und Entfernung der Glycosylreste in den erhaltenen Proteinen zur Herstellung von unglycosyliertem Protein, das frei ist von glycosyliertem Protein.

9. Verfahren nach Anspruch 8 zur Herstellung von GPA, Pro-GPA und deren Mischungen, worin der GPA und Pro-GPA in glycosylierter oder unglycosylierter Form oder einer Mischung dieser Formen vorliegt, umfassend die Schritte der unter geeigneten Nährstoffbedingungen erfolgenden Kultivierung eines Hefestammes, der transformiert wurde mit einem Hybridvektor umfassend den Hefe-PHO5-Promotor, der operabel verknüpft ist mit einer DNA-Sequenz bestehend aus einer Signalsequenz, die im Rahmen verknüpft ist, mit der humanen GPA codierenden Region, und eine DNA-Sequenz, die Transkriptionsterminationssignale eines Hefe-gens enthält, und Isolierung und Reinigung der genannten Proteine und, falls gewünscht, Separierung einer erhaltenen Mischung von GPA und Pro-GPA in die einzelnen Komponenten, und zur Herstellung von GPA, der frei ist von Pro-GPA, Umwandlung des gebildeten Pro-GPA in GPA und, falls gewünscht, Separieren der glycosylierten von den unglycosylierten Produkten, die in einer Mischung erhalten wurden, und Entfernung der Glycosylreste in den erhaltenen Proteinen zur Herstellung von unglycosyliertem Protein, das frei ist von glycosyliertem Protein.

10. Verfahren nach einem der Ansprüche 8 oder 9, worin die Hefe *Saccharomyces cerevisiae* ist.

11. Verfahren nach Anspruch 9, worin GPA und Pro-GPA und deren Mischungen aus Kulturflüssigkeiten durch selektive Adsorption an einer DE-3 Sepharose-Säule isoliert werden.

12. Verfahren nach Anspruch 9, worin GPA und Pro-GPA und deren Mischungen aus Kulturflüssigkeiten durch Affinitätschromatographie unter Verwendung monoklonaler Antikörper isoliert werden.

13. Verfahren nach Anspruch 9, worin ein Proteaseinhibitor während des Reinigungsvorganges einbezogen wird und Chromatographie auf einer DE-3 Sepharose-Säule in Gegenwart eines Inhibitors durchgeführt wird, der selektiv nur GPA bindet, um Pro-GPA herzustellen, der im wesentlichen frei von GPA ist.

14. Verfahren nach Anspruch 9, worin erhaltene glycosylierte und unglycosylierte Produkte durch Chromatographie auf einer Concanavalin-A Sepharose-Säule getrennt werden.

**Revendications pour les Etats Contractants: BE, CH, DE, FR, GB, IT, LT, LU, NL, SE**

1. Vecteur hybride de levure comprenant le promoteur PHO5 de levure lié de façon opérationnelle à une séquence d'ADN consistant en une séquence signal liée en un cadre à la région codant pour l'activateur du plasminogène tissulaire humain (TPA), et une séquence d'ADN contenant les signaux de terminaison de la transcription d'un gène de levure.

2. Vecteur hybride de levure selon la revendication 1, comprenant la séquence signal de PHO5 liée en un cadre à la région codant pour le TPA humain.

3. Vecteur hybride de levure selon la revendication 1, comprenant la séquence prépro de TPA liée en un cadre à la région codant pour le TPA humain.

4. Vecteur hybride de levure selon la revendication 1 dans lequel le promoteur PHO5 de levure se prolonge dans la région codant pour PHO5 qui est liée à la région codant pour le TPA humain par un lieur contenant des sites de maturation pour une endopeptidase de levure.

5. Un segment d'ADN comprenant le promoteur PHO5 de levure lié de façon opérationnelle à une séquence d'ADN consistant en séquence signal de PHO5 liée en un cadre au gène de l'activateur du plasminogène tissulaire humain, et une séquence d'ADN contenant les signaux de terminaison de la transcription du gène de PHO5 de levure.

6. Souche de levure transformée choisie dans le groupe consistant en une souche de levure transformée par un vecteur hybride comprenant le promoteur PHO5 de levure lié de façon opérationnelle à une séquence d'ADN consistant en une séquence signal liée en un cadre à la région codant pour l'activateur du plasminogène tissulaire humain, et une séquence d'ADN contenant les signaux de terminaison de la transcription d'une gène de levure, et une souche de levure avec le gène pour l'activateur du plasminogène tissulaire human intégré de façon stable dans le chromosome de levure et étant sous le contrôle du promoteur PHO5 de levure chromosomique, et ses mutants.

7. Levure transformée par un vecteur hybride selon l'une quelconque des revendications 1 à 4.

8. Levure transformée selon la revendication 6 dans laquelle la levure est *Saccharomyces cerevisiae*.

9. Méthode pour la production de TPA, de pro-TPA ou de leurs mélanges où TPA et pro-TPA sont présents sous une forme glycosylées ou non glycosylée ou en un mélange de ces formes, comprenant les étapes de culture dans des conditions nutritionnelles appropriées d'une souche de levure transformée par un vecteur hybride comprenant le promoteur PHO5 de levure lié de façon opérationnelle à une séquence d'ADN consistant en une séquence signal liée en un cadre à la région codant pour l'activateur du plasminogène tissulaire humain, et une séquence d'ADN contenant les signaux de terminaison de transcription d'un gène de levure, ou une souche de levure avec le gène pour l'activateur du plasminogène tissulaire humain intégré de façon stable dans le chromosome de levure et étant sous le contrôle du promoteur PHO5 de levure chromosomique, ou un mutant d'une telle souche de levure, et l'isolement et la purification desdites protéines et, si cela est souhaitable, la séparation d'un mélange de TPA et de pro-TPA obtenu en composants individuels, et, pour la production de TPA qui est exempt de pro-TPA, la conversion du pro-TPA produit en TPA, et, si cela est souhaitable, la séparation des produits glycosylés des produits non glycosylés obtenus en mélange et, pour la production d'une protéine non glycosylée qui est exempte de protéine glycosylée, l'élimination des résidus glycosyles des protéines obtenues.

10. Méthode selon la revendication 9 pour la production de TPA, de pro-TPA ou de leurs mélanges où le TPA et le pro-TPA sont présents sous une forme glycosylée ou non glycosylée ou en un mélange de ces formes, comprenant les étapes de culture dans des conditions nutritionnelles appropriées d'une souche de levure transformée par un vecteur hybride comprenant le promoteur PHO5 de levure lié de façon opérationnelle à une séquence d'ADN consistant en une séquence signal liée en un cadre à la région codant pour la TPA humain et une séquence d'ADN contenant les signaux de terminaison de la transcription d'un gène de levure, et l'isolement et la purification desdites protéines et, si cela est souhaitable, la séparation d'un mélange de TPA et de pro-TPA obtenu en composants individuels, et, pour la production de TPA qui est exempt de pro-TPA, la conversion du pro-TPA produit en TPA et, si cela est souhaitable, la séparation des produits glycosylés des produits non glycosylés obtenus en mélange et, pour la production d'une protéine non glycosylée qui est exempte de protéine glycosylée, l'élimination des résidues glycosyles des protéines obtenues.

11. Méthode selon l'une quelconque des revendications 9 ou 10, dans laquelle la levure est *Saccharomyces cerevisiae*.

12. Méthode selon la revendication 9, dans laquelle le TPA et la pro-TPA et leurs mélanges sont isolés des liquides de culture par adsorption sélective sur une colonne de DE-3 Sepharose.

13. Méthode selon la revendication 10 oùle TPA et le pro-TPA et leurs mélanges sont isolées des liquides de culture par chromatographie d'affinité en utilisant des anticorps monoclonaux.

14. Méthode selon la revendication 10 dans laquelle un inhibiteur de la protéase est inclus pendant le processus de purification, et une chromatographie sur une colonne de De-3 Sepharose est effectuée en présence d'un inhibiteur qui ne fixe sélectivement que le TPA de façon à préparer le pro-TPA qui est pratiquement exempt de TPA.

15. Méthode selon la revendication 10, dans laquelle les produits glycosylés et non glycosylés obtenus sont séparés par chromatographie sur une colonne de Sepharose avec la concanavaline-A.

**Revendications pour l'Etat Contractant: AT**

1. Méthode pour la production d'un vecteur hybride de levure comprenant le promoteur PHO5 de levure lié de façon opérationelle à une séquence d'ADN consistant en une séquence signal liée en un cadre à la région codant pour l'activateur du plasminogène tissulaire humain (TPA), et une séquence d'ADN contenant les signaux de terminaison de la transcription d'un gène de levure, méthode qui comprend l'introduction dans un ADN de vecteur ledit promoteur, ladite séquence signal, ladite région codant pour la TPA et ladite séquence terminateur dans l'ordre prédéterminé.

2. Méthode selon la revendication 1, dans laquelle le vecteur hybride comprend la séquence signal de PHO5 liée en un cadre à la région codant pour le TPA humain.

3. Méthode selon la revendication 1, dans laquelle le vecteur hybride comprend la séquence du prépro-TPA liée en un cadre à la région codant pour le TPA humain.

4. Méthode selon la revendication 1, dans laquelle le promoteur PHO5 de levure se prolonge dans la région codant PHO5 qui est liée à la région codant pour le TPA humain par un lieur de synthèse contenant des dites de maturation pour une endopeptidase de levure.

5. Méthode pour la production d'une souche de levure transformée, choisie dans le groupe consistant en une souche de levure transformée par un vecteur hybride comprenant le promoteur PHO5 de levure lié de façon opérationnelle à une séquence d'ADN consistant en une séquence signal liée en un cadre à la région codant pour l'activateur du plasminogène tissulaire humain, et une séquence d'ADN contenant les signaux de terminaison de transcription d'un gène de levure, et une souche de levure avec le gène pour l'activateur du plasminogène tissulaire humain intégré de façon stable dans le chromosome de levure et étant sous le contrôle du promoteur PHO5 de levure chromosomique, méthode qui comprend la transformation de la levure par ledit vecteur hybride ou par un segment d'ADN comprenant le promoteur PHO5 de levure lié de façon opérationnelle à une séquence d'ADN consistant en séquence signal de PHO5 liée en un cadre au gène pour l'activateur du plasminogène tissulaire humain, et une séquence d'ADN contenant les signaux de terminaison de la transcription du gène de PHO5 de levure.

6. Méthode selon la revendication 5 comprenant la transformation de la levure par un vecteur hybride pouvant être obtenu par l'une quelconque des revendications 1 à 4.

7. Méthode selon la revendication 5 dans laquelle le levure est *Saccharomyces cerevisiae*.

8. Méthode pour la production de TPA, de pro-TPA ou de leurs mélanges dans laquelle le TPA et le pro-TPA sont présents sous une forme glycosylée ou non glycosylée ou en un mélange de ces formes, comprenant les étapes de culture dans des conditions nutritionnelles appropriées d'une souche de levure transformée par un vecteur hybride comprenant le promoteur PHO5 de levure lié de façon opérationnelle à la région codant pour l'activateur du plasminogène tissulaire humain, et une séquence d'ADN contenant les signaux de terminaison de transcription d'un gène de levure, ou une souche de levure avec le gène pour l'activateur du plasminogène tissulaire humain intégré de façon stable dans le chromosome de levure et étant sous le contrôle du promoteur PHO5 de levure, ou un mutant d'une telle souche de levure, et l'isolement et la purification desdites protéines et, si cela est souhaitable, la séparation d'un mélange de TPA et de pro-TPA obtenu en composants individuels et, pour la production de TPA qui est exempt de pro-TPA, la conversion du pro-TPA produit en TPA et, si cela est souhaitable, la séparation des produits glycosylés des produits non glycosylés obtenus en mélange et, pour la production d'une protéine non glycosylée qui est exempte de protéine glycosylée, l'élimination des résidus glycosyles des protéines obtenues.

9. Méthode selon la revendication 8 pour la production de TPA, de pro-TPA ou de leurs mélanges, dans laquelle le TPA et le pro-TPA sont présents sous une forme glycosylée ou non glycosylée ou en un mélange de ces formes, comprenant les étapes de culture dans des conditions nutritionnelles appropriées d'une souche de levure transformée par un vecteur hybride comprenant le promoteur PHO5 de levure lié à une séquence d'ADN consistant en une séquence signal liée en un cadre à la région codant pour le TPA humain et une séquence d'ADN contenant les signaux de terminaison de transcription d'un gène de levure, et l'isolement et la purification desdites protéines et, si cela est souhaitable, la séparation d'un mélange de TPA et de pro-TPA obtenu en composants individuels et, pour la production de TPA qui est exempte de pro-TPA, la conversion du pro-TPA conduit en TPA et, si cela est souhaitable, la séparation des produits glycosylés des produits non glycosylés obtenus en mélange et, pour la production d'une protéine non glycosylée qui est exempte de protéine glycosylée, l'élimination des résidus glycosyles des protéines obtenues.

10. Méthode selon l'une quelconque des revendications 8 ou 9, dans laquelle la levure est *Saccharomyces cerevisiae*.

11. Méthode selon la revendication 9, dans laquelle le TPA et le pro-TPA et leurs mélanges sont isolés des liquides de culture par adsorption sélective sur une colonne de DE-3 Sepharose.

12. Méthode selon la revendication 9, dans laquelle le TPA et le pro-TPA et leurs mélanges sont isolés des liquides de culture par chromatographie d'affinité en utilisant des anticorps monoclonaux.

13. Méthode selon la revendication 9, dans laquelle un inhibiteur de la protéase est inclus pendant le processus de purification, et une chromatographie sur une colonne de DE-3 Sepharose est effectuée en présence d'un inhibiteur qui ne fixe sélectivement que le TPA de façon à préparer le pro-TPA qui est pratiquement exempt de TPA.

14. Méthode selon la revendication 9, dans laquelle les produits glycosylés et non glycosylés obtenus sont séparés par chromatographie sur une colonne de Sepharose avec la concanavaline-A.

Fig. 1 STARTING PLASMIDS FOR DNA SEQUENCING AND EXPRESSION PLASMID CONSTRUCTION

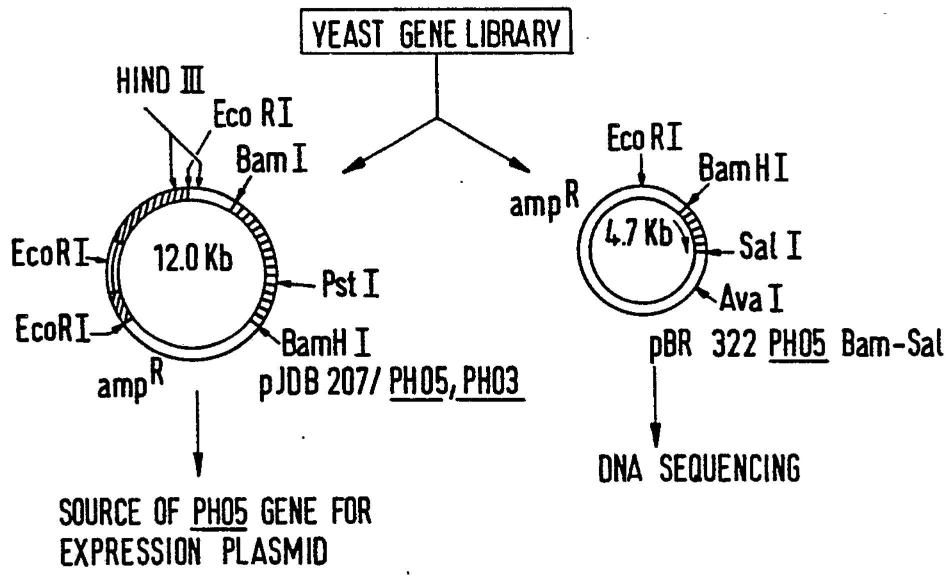

Fig. 2 LOCALIZATION OF THE REPRESSIBLE (PHO5) AND THE CONSTITUTIVE (PHO3) ACID PHOSPHATASE GENE

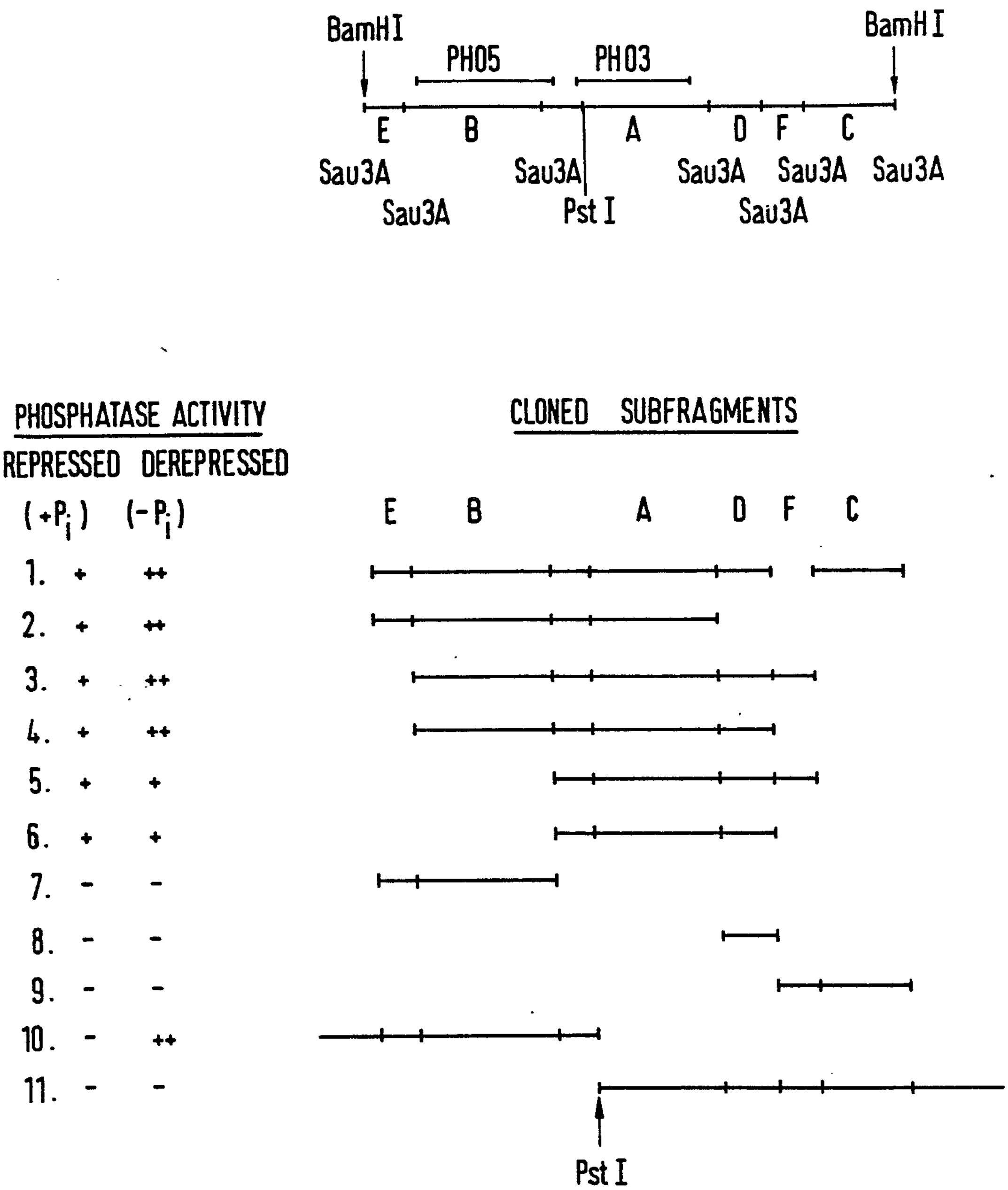

## Fig. 3a

Nucleotide sequence of the BamHI-SalI restriction fragment of PH05 including the PH05 promoter region

```
                                                                      G
-540
    ATCCGAAAGTTGTATTCAACAAGAATGCGCAAATATGTCAACGTATTTGGAAGTCATCTT

-480
    ATGTGCGCTGCTTTAATGTTTTCTCATGTAAGCGGACGTCGTCTATAAACTTCAAACGAA

-420
    GGTAAAAGGTTCATAGCGCTTTTTCTTTGTCTGCACAAAGAAATATATATTAAATTAGCA

-360
    CGTTTTCGCATAGAACGCAACTGCACAATGCCAAAAAAAGTAAAAGTGATTAAAAGAGTT

-300
    AATTGAATAGGCAATCTCTAAATGAATCGATACAACCTTGGCACTCACACGTGGGACTAG

-240
    CACAGACTAAATTTATGATTCTGGTCCCTGTTTTCGAAGAGATCGCACATGCCAAATTAT

-180
    CAAATTGGTCACCTTACTTGGCAAGGCATATACCCATTTGGGATAAGGGTAAACATCTTT

-120
    GAATTGTCGAAATGAAACGTATATAAGCGCTGATGTTTTGCTAAGTCGAGGTTAGTATGG

-60
    CTTCATCTCTCATGAGAATAAGAACAACAACAAATAGAGCAAGCAAATTCGAGATTACCA

 +1
    ATG TTT AAA TCT GTT GTT TAT TCA ATT TTA GCC GCT TCT TTG GCC +45
    MET PHE LYS SER VAL VAL TYR SER ILE LEU ALA ALA SER LEU ALA

                                                    +82
    AAT GCA GGT ACC ATT CCC TTA GGC AAA CTA GCC GAT G
    ASN ALA GLY THR ILE PRO LEU GLY LYS LEU ALA ASP
```

**Fig. 3b**

Nucleotide sequence of the PstI-RsaI restriction fragment of PHO3 including the PHO3 promoter region

```
                                                                  GA
-360
   GATATCCGAAACAGGTAAATGGATGTTTCAATCCCTGTAGTCAGTCAGGAACCCATATTA

-300
   TATTACAGTATTAGTCGCCGCTTAGGCACGCCTTTAATTAGCAAAATCAAACCTTAAGTG

-240
   CATATGCCGTATAAGGGAAACTCAAAGAACTGGCATCGCAAAAATGAAAAAAAGGAAGAG

-180
   TGAAAAAAAAAAAATTCAAAAGAAATTTACTAAATAATACCAGTTTGGGAAATAGTAAAC

-120
   AGCTTTGAGTAGTCCTATGCAACATATATAAGTGCTTAAATTTGCTGGATGGAAGTCAAT

- 60
   TATGCCTTGATTATCATAAAAAAAATACTACAGTAAAGAAAGGGCCATTCCAAATTACCT

  +1                                                            +45
   ATG TTT AAG TCT GTT GTT TAT TCG GTT CTA GCC GCT GCT TTA GTT
   MET PHE LYS SER VAL VAL TYR SER VAL LEU ALA ALA ALA LEU VAL

            +54
   AAT GCA GGT
   ASN ALA GLY
```

Fig. 4 CONSTRUCTION OF PLASMID p30

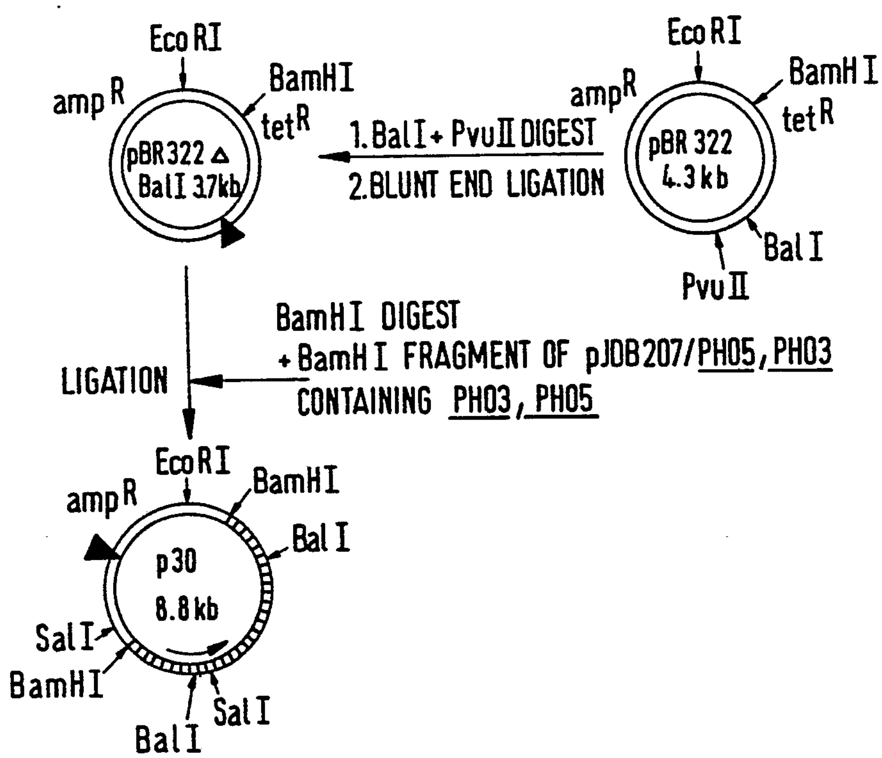

Fig. 5 CONSTRUCTION OF EXPRESSION PLASMID p31

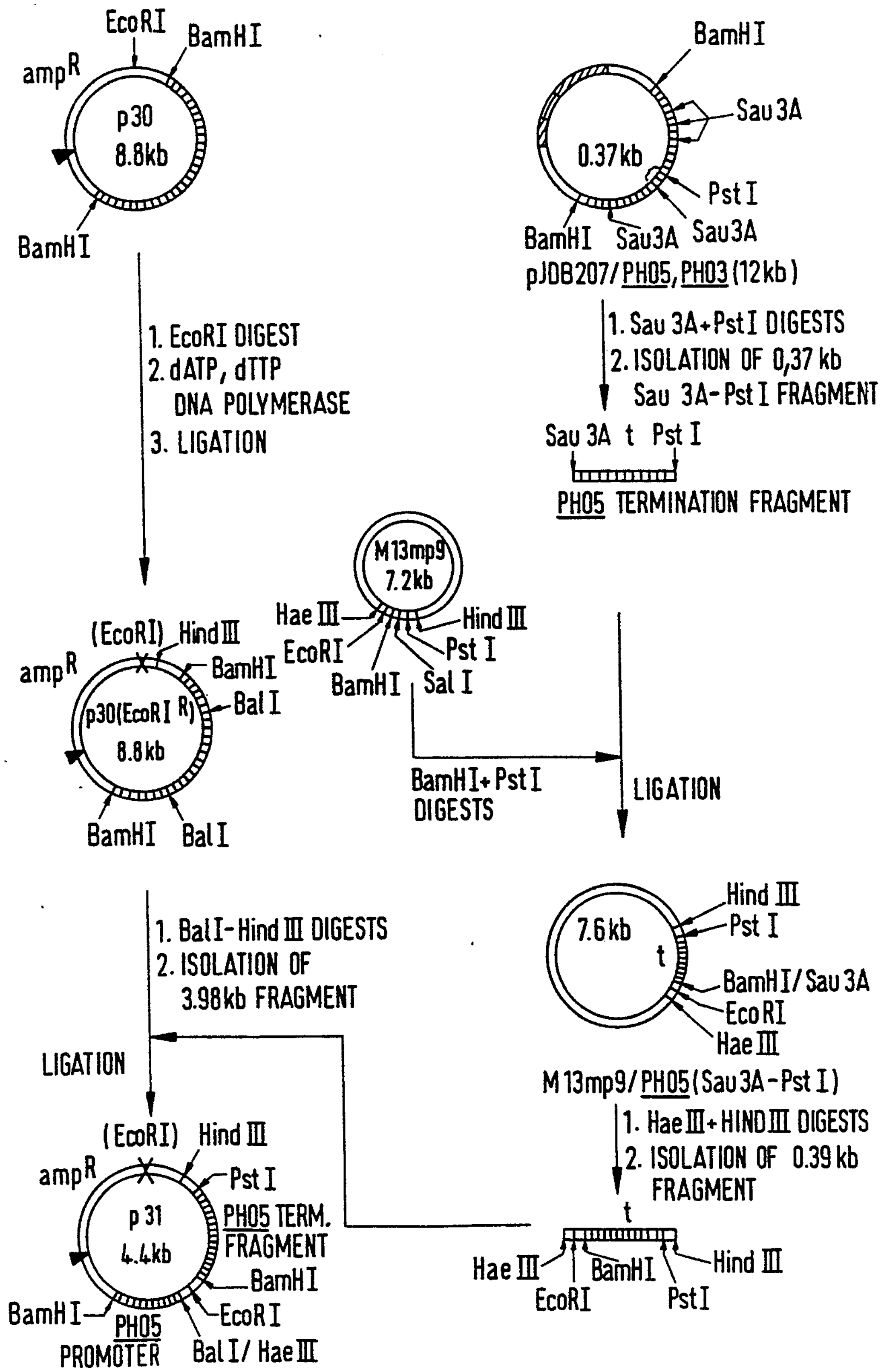

**Fig. 6**

Nucleotide sequence of the Sau3A-PstI PHO5 transcription termination fragment

GATCCTGGTACGTTCCTCAAGGTGCTCGTGTCTACACCGAAAAATTCCAATGTTCTAACG

ACACCTACCTCAGATACGTCATTAACGATGCTGTTGTTCCAATTGAAACCTGTTCCACTG

GTCCAGGGTTCTCTTGTGAAATCAATGACTTCTACGACTATGCTGAAAAGAGAGTAGCCG

GTACTGACTTCCTAAAGGTCTGTAACGTCAGCAGCGTCAGTAACTCTACTGAATTGACCT

TCTACTGGGACTGGAACACTACTCATTACAACGCCAGTCTATTGAGACAATAGTTTTGTA

TAACTAAATAATATTGGAAACTAAATACGAATACCCAAATTTTTTATCTAAATTTTGCCG

AAAGATTAAAATCTGCA

**Fig. 7** DELETION OF THE PHO5 SIGNAL SEQUENCE IN EXPRESSION PLASMID p31

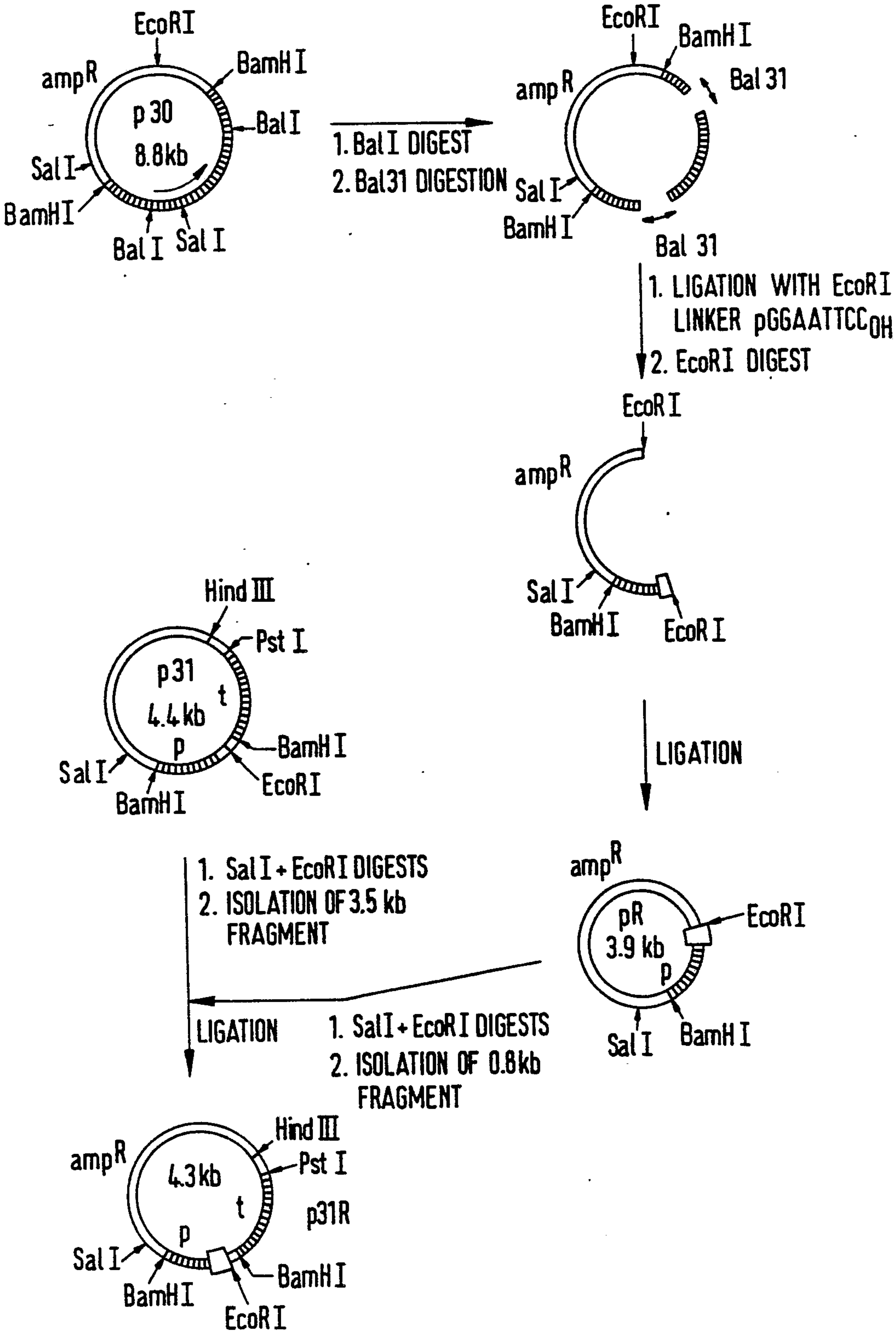

**Fig. 8**

Collection of Bal31 deletions in the PHO5 region with EcoRI linkers at the arrows

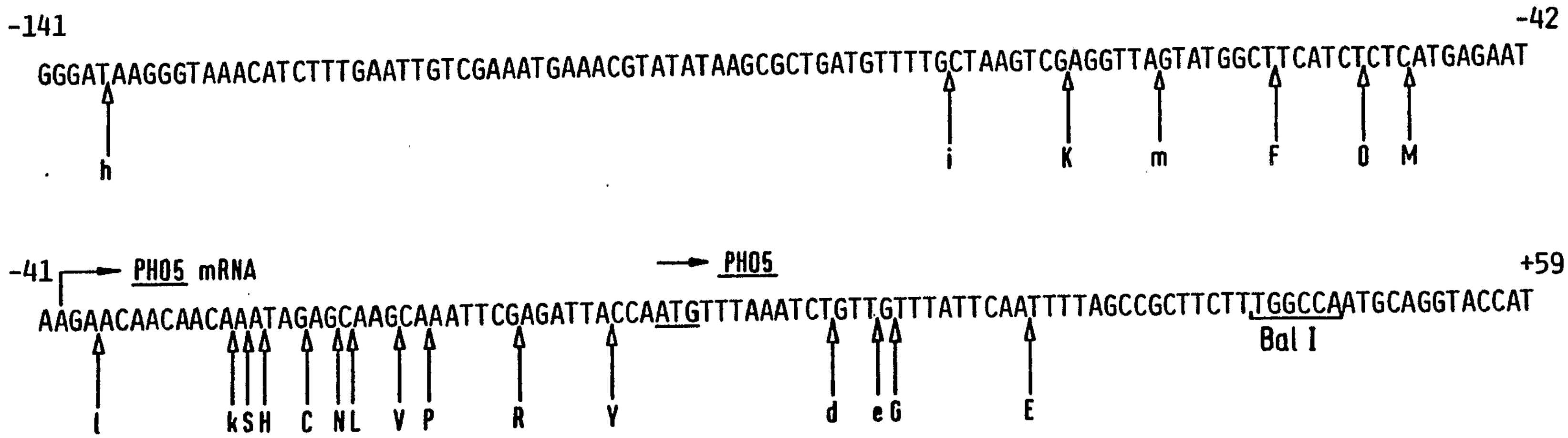


PHO5 — startpoint of PHO5 protein coding region

PHO5 mRNA — major PHO5 mRNA initiation site

endpoint of Bal31 deletion (from BalI restriction site) and position of EcoRI linker addition

## Fig. 9

Nucleotide sequence of the BamHI-EcoRI restriction fragment containing the PHO5R promoter region

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGGG

**Fig. 10**

Nucleotide sequence of the BamHI-EcoRI restriction fragment containing the PHO5Y promoter region

G

ATCCGAAAGTTGTATTCAACAAGAATGCGCAAATATGTCAACGTATTTGGAAGTCATCTT

ATGTGCGCTGCTTTAATGTTTTCTCATGTAAGCGGACGTCGTCTATAAACTTCAAACGAA

GGTAAAAGGTTCATAGCGCTTTTTCTTTGTCTGCACAAAGAAATATATATTAAATTAGCA

CGTTTTCGCATAGAACGCAACTGCACAATGCCAAAAAAAGTAAAAGTGATTAAAAGAGTT

AATTGAATAGGCAATCTCTAAATGAATCGATACAACCTTGGCACTCACACGTGGGACTAG

CACAGACTAAATTTATGATTCTGGTCCCTGTTTTCGAAGAGATCGCACATGCCAAATTAT

CAAATTGGTCACCTTACTTGGCAAGGCATATACCCATTTGGGATAAGGGTAAACATCTTT

GAATTGTCGAAATGAAACGTATATAAGCGCTGATGTTTTGCTAAGTCGAGGTTAGTATGG

CTTCATCTCTCATGAGAATAAGAACAACAACAAATAGAGCAAGCAAATTCGAGATTAGG

## Fig. 11

Nucleotide sequence and deduced amino acid sequence of TPA cDNA insert prepared from HeLa cells

| | | |
|---|---|---|
| 1 | AGGGCTGGAGAGAAAACCTCTGCGAGGAAAGGGAAGGAGCAAGCCGTGAATTTAAGGGACGCTGTGA | |
| | MET ASP ALA MET LYS ARG GLY LEU CYS CYS VAL LEU LEU LEU CYS | 15 |
| 68 | AGCAATC ATG GAT GCA ATG AAG AGA GGG CTC TGC TGT GTG CTG CTA CTG TGT | |
| | GLY ALA VAL PHE VAL SER PRO SER GLN GLU ILE HIS ALA ARG PHE ARG ARG | 32 |
| 120 | GGA GCA GTC TTC GTT TCG CCC AGC CAG GAA ATC CAT GCC CGA TTC AGA AGA | |
| | GLY ALA ARG SER TYR GLN VAL ILE CYS ARG ASP GLU LYS THR GLN MET ILE | 49 |
| 171 | GGA GCC AGA TCT TAC CAA GTG ATC TGC AGA GAT GAA AAA ACG CAG ATG ATA | |
| | TYR GLN GLN HIS GLN SER TRP LEU ARG PRO VAL LEU ARG SER ASN ARG VAL | 66 |
| 222 | TAC CAG CAA CAT CAG TCA TGG CTG CGC CCT GTG CTC AGA AGC AAC CGG GTG | |
| | GLU TYR CYS TRP CYS ASN SER GLY ARG ALA GLN CYS HIS SER VAL PRO VAL | 83 |
| 273 | GAA TAT TGC TGG TGC AAC AGT GGC AGG GCA CAG TGC CAC TCA GTG CCT GTC | |
| | LYS SER CYS SER GLU PRO ARG CYS PHE ASN GLY GLY THR CYS GLN GLN ALA | 100 |
| 324 | AAA AGT TGC AGC GAG CCA AGG TGT TTC AAC GGG GGC ACC TGC CAG CAG GCC | |
| | LEU TYR PHE SER ASP PHE VAL CYS GLN CYS PRO GLU GLY PHE ALA GLY LYS | 117 |
| 375 | CTG TAC TTC TCA GAT TTC GTG TGC CAG TGC CCC GAA GGA TTT GCT GGG AAG | |
| | CYS CYS GLU ILE ASP THR ARG ALA THR CYS TYR GLU ASP GLN GLY ILE SER | 134 |
| 426 | TGC TGT GAA ATA GAT ACC AGG GCC ACG TGC TAC GAG GAC CAG GGC ATC AGC | |
| | TYR ARG GLY THR TRP SER THR ALA GLU SER GLY ALA GLU CYS THR ASN TRP | 151 |
| 477 | TAC AGG GGC ACG TGG AGC ACA GCG GAG AGT GGC GCC GAG TGC ACC AAC TGG | |

**Fig. 11** (Continued)

ASN SER SER ALA LEU ALA GLN LYS PRO TYR SER GLY ARG ARG PRO ASP ALA 168
528 AAC AGC AGC GCG TTG GCC CAG AAG CCC TAC AGC GGG CGG AGG CCA GAC GCC

ILE ARG LEU GLY LEU GLY ASN HIS ASN TYR CYS ARG ASN PRO ASP ARG ASP 185
579 ATC AGG CTG GGC CTG GGG AAC CAC AAC TAC TGC AGA AAC CCA GAT CGA GAC

SER LYS PRO TRP CYS TYR VAL PHE LYS ALA GLY LYS TYR SER SER GLU PHE 202
630 TCA AAG CCC TGG TGC TAC GTC TTT AAG GCG GGG AAG TAC AGC TCA GAG TTC

CYS SER THR PRO ALA CYS SER GLU GLY ASN SER ASP CYS TYR PHE GLY ASN 219
681 TGC AGC ACC CCT GCC TGC TCT GAG GGA AAC AGT GAC TGC TAC TTT GGG AAT

GLY SER ALA TYR ARG GLY THR HIS SER LEU THR GLU SER GLY ALA SER CYS 236
732 GGG TCA GCC TAC CGT GGC ACG CAC AGC CTC ACC GAG TCG GGT GCC TCC TGC

LEU PRO TRP ASN SER MET ILE LEU ILE GLY LYS VAL TYR THR ALA GLN ASN 253
783 CTC CCG TGG AAT TCC ATG ATC CTG ATA GGC AAG GTT TAC ACA GCA CAG AAC

PRO SER ALA GLN ALA LEU GLY LEU GLY LYS HIS ASN TYR CYS ARG ASN PRO 270
834 CCC AGT GCC CAG GCA CTG GGC CTG GGC AAA CAT AAT TAC TGC CGG AAT CCT

ASP GLY ASP ALA LYS PRO TRP CYS HIS VAL LEU LYS ASN ARG ARG LEU THR 287
885 GAT GGG GAT GCC AAG CCC TGG TGC CAC GTG CTG AAG AAC CGC AGG CTG ACG

TRP GLU TYR CYS ASP VAL PRO SER CYS SER THR CYS GLY LEU ARG GLI
936 TGG GAG TAC TGT GAT GTG CCC TCC TGC TCC ACC TGC GGC CTG AGA CA

SER GLN PRO GLN PHE ARG ILE LYS GLY GLY LEU PHE ALA ASP ILE AL
987 AGC CAG CCT CAG TTT CGC ATC AAA GGA GGG CTC TTC GCC GAC ATC GC

**Fig. 11** (Continued)

| | | |
|---|---|---|
| | HIS PRO TRP GLN ALA ALA ILE PHE ALA LYS HIS ARG ARG SER PRO GLY GLU | 338 |
| 1038 | CAC CCC TGG CAG GCT GCC ATC TTT GCC AAG CAC AGG AGG TCG CCC GGA GAG | |
| | ARG PHE LEU CYS GLY GLY ILE LEU ILE SER SER CYS TRP ILE LEU SER ALA | 355 |
| 1089 | CGG TTC CTG TGC GGG GGC ATA CTC ATC AGC TCC TGC TGG ATT CTC TCT GCC | |
| | ALA HIS CYS PHE GLN GLU ARG PHE PRO PRO HIS HIS LEU THR VAL ILE LEU | 372 |
| 1140 | GCC CAC TGC TTC CAG GAG AGG TTT CCG CCC CAC CAC CTG ACG GTG ATC TTG | |
| | GLY ARG THR TYR ARG VAL VAL PRO GLY GLU GLU GLU GLN LYS PHE GLU VAL | 389 |
| 1191 | GGC AGA ACA TAC CGG GTG GTC CCT GGC GAG GAG GAG CAG AAA TTT GAA GTC | |
| | GLU LYS TYR ILE VAL HIS LYS GLU PHE ASP ASP ASP THR TYR ASP ASN ASP | 406 |
| 1242 | GAA AAA TAC ATT GTC CAT AAG GAA TTC GAT GAT GAC ACT TAC GAC AAT GAC | |
| | ILE ALA LEU LEU GLN LEU LYS SER ASP SER SER ARG CYS ALA GLN GLU SER | 423 |
| 1293 | ATT GCG CTG CTG CAG CTG AAA TCG GAT TCG TCC CGC TGT GCC CAG GAG AGC | |
| | SER VAL VAL ARG THR VAL CYS LEU PRO PRO ALA ASP LEU GLN LEU PRO ASP | 440 |
| 1344 | AGC GTG GTC CGC ACT GTG TGC CTT CCC CCG GCG GAC CTG CAG CTG CCG GAC | |
| | TRP THR GLU CYS GLU LEU SER GLY TYR GLY LYS HIS GLU ALA LEU SER PRO | 457 |
| 1395 | TGG ACG GAG TGT GAG CTC TCC GGC TAC GGC AAG CAT GAG GCC TTG TCT CCT | |
| | PHE TYR SER GLU ARG LEU LYS GLU ALA HIS VAL ARG LEU TYR PRO SER SER | 474 |
| 1446 | TTC TAT TCG GAG CGG CTG AAG GAG GCT CAT GTC AGA CTG TAC CCA TCC AGC | |
| | ARG CYS THR SER GLN HIS LEU LEU ASN ARG THR VAL THR ASP ASN MET LEU | 491 |
| 1497 | CGC TGC ACA TCA CAA CAT TTA CTT AAC AGA ACA GTC ACC GAC AAC ATG CTG | |

**Fig. 11** (Continued)

```
      CYS ALA GLY ASP THR ARG SER GLY GLY PRO GLN ALA ASN LEU HIS ASP ALA   508
1548  TGT GCT GGA GAC ACT CGG AGC GGC GGG CCC CAG GCA AAC TTG CAC GAC GCC

      CYS GLN GLY ASP SER GLY GLY PRO LEU VAL CYS LEU ASN ASP GLY ARG MET   525
1599  TGC CAG GGC GAT TCG GGA GGC CCC CTG GTG TGT CTG AAC GAT GGC CGC ATG

      THR LEU VAL GLY ILE ILE SER TRP GLY LEU GLY CYS GLY GLN LYS ASP VAL   542
1650  ACT TTG GTG GGC ATC ATC AGC TGG GGC CTG GGC TGT GGA CAG AAG GAT GTC

      PRO GLY VAL TYR THR LYS VAL THR ASN TYR LEU ASP TRP ILE ARG ASP ASN   559
1701  CCG GGT GTG TAC ACA AAG GTT ACC AAC TAC CTA GAC TGG ATT CGT GAC AAC

      MET ARG PRO
1752  ATG CGA CCG TGA CCAGGAACACCCGACTCCTCAAAAGCAAATGAGATCCCGCCTCTTCTTCT

1814  TCAGAAGACACTGCAAAGGCGCAGTGCTTCTCTACAGACTTCTCCAGACCCACCACACCGCAGAAG

1880  CGGGACGAGACCCTACAGGAGAGGGAAGAGTGCATTTTCCCAGATACTTCCCATTTTGGAAGTTTT

1946  CAGGACTTGGTCTGATTTCAGGATACTCTGTCAGATGGGAAGACATGAATGCACACTAGCCTCTCC

2012  AGGAATGCCTCCTCCCTGGGCAGAAGTGGCCATGCCACCCTGTTTTCGCTAAAGCCCAACCTCCTG

2078  ACCTGTCACCGTGAGCAGCTTTGGAAACAGGACCACAAAAATGAAAGCATGTCTCAATAGTAAAAG

2144  AAACAAGA
```

Fig. 12 CONSTRUCTION OF M13mp9 / PHO5-TPA

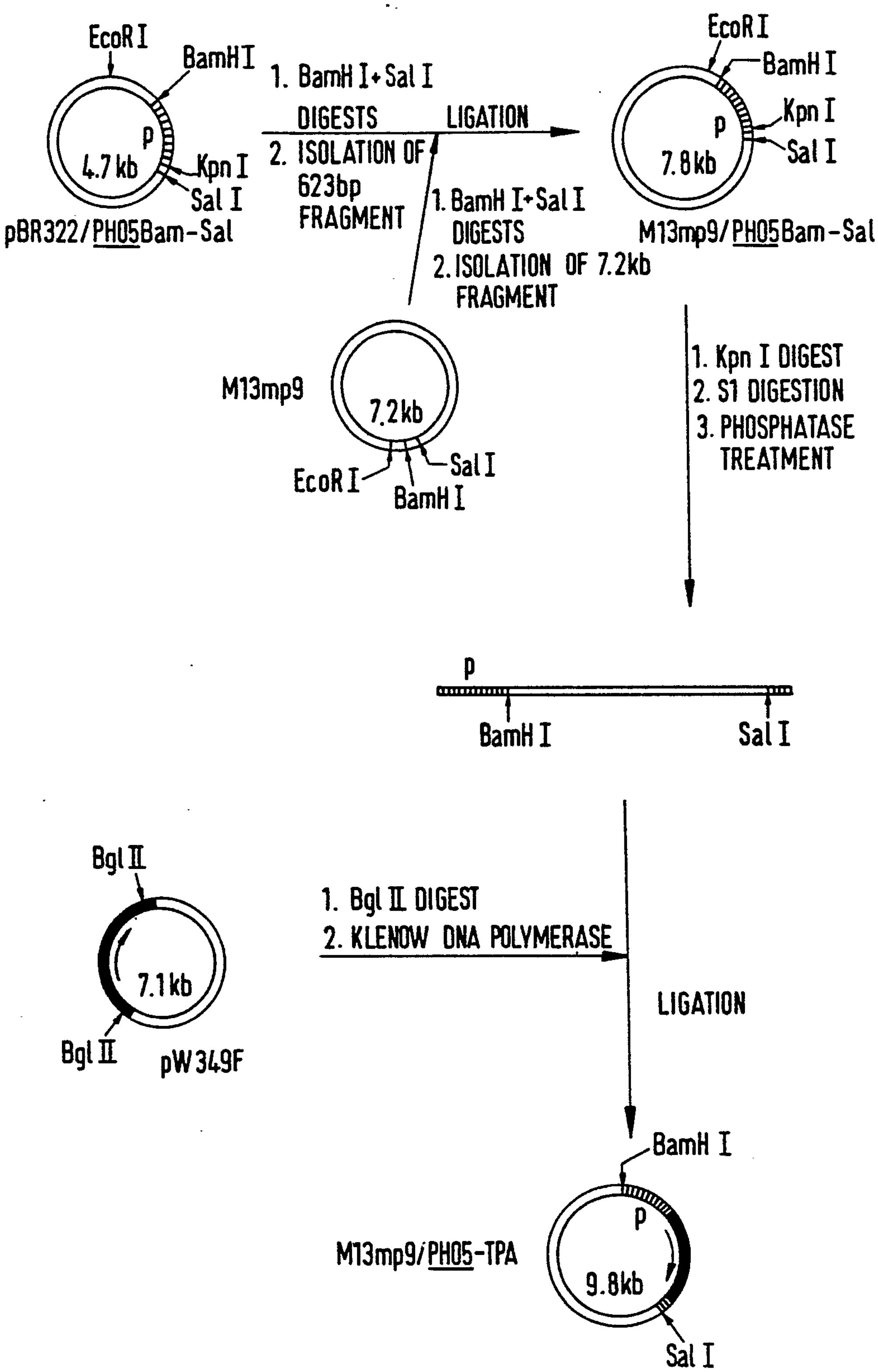

Fig. 13 CONSTRUCTION OF pJDB 207 / PHO5 - TPA (1A)

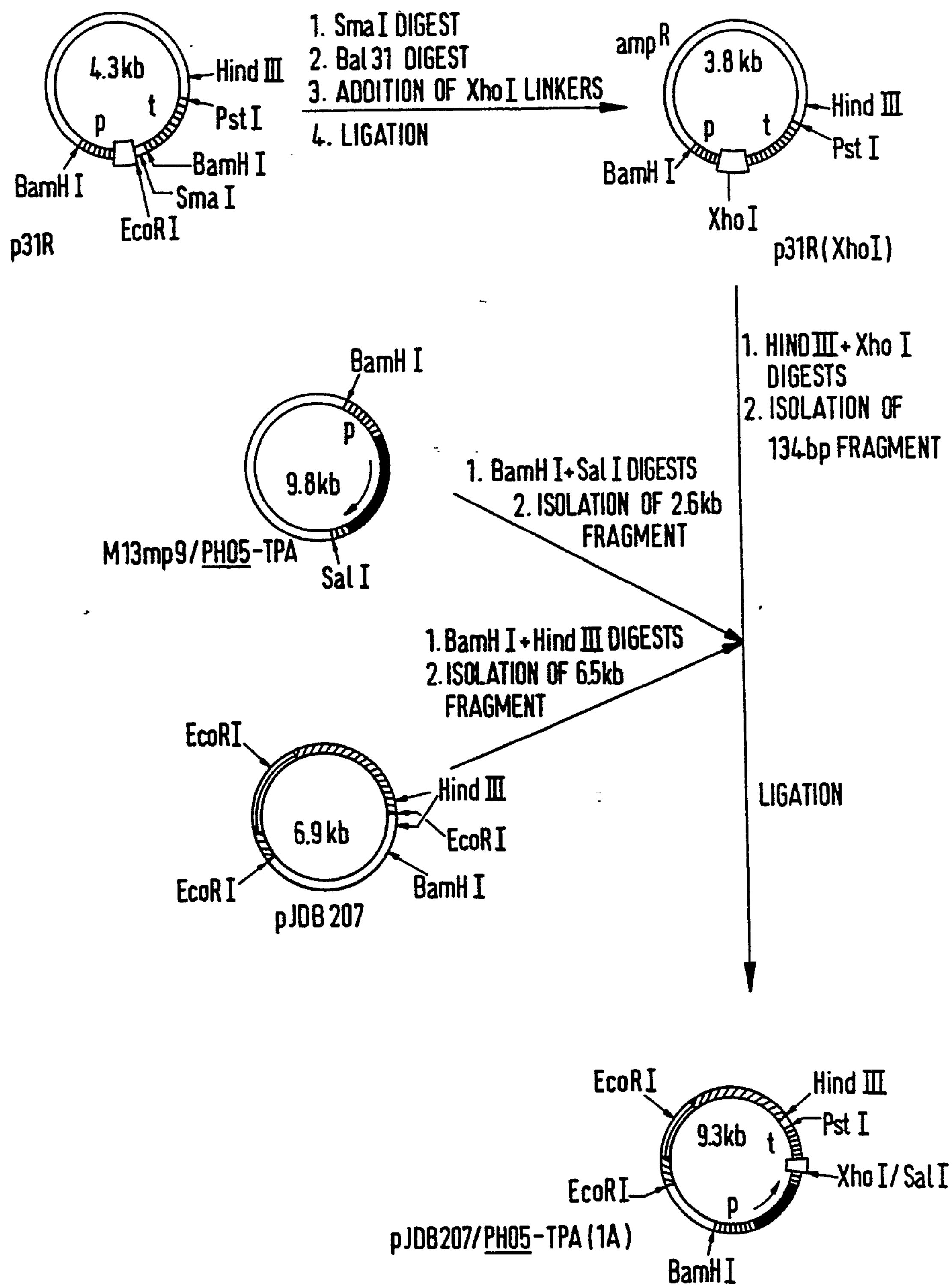

Fig. 14 CONSTRUCTION OF pJDB207/PH05-TPA△

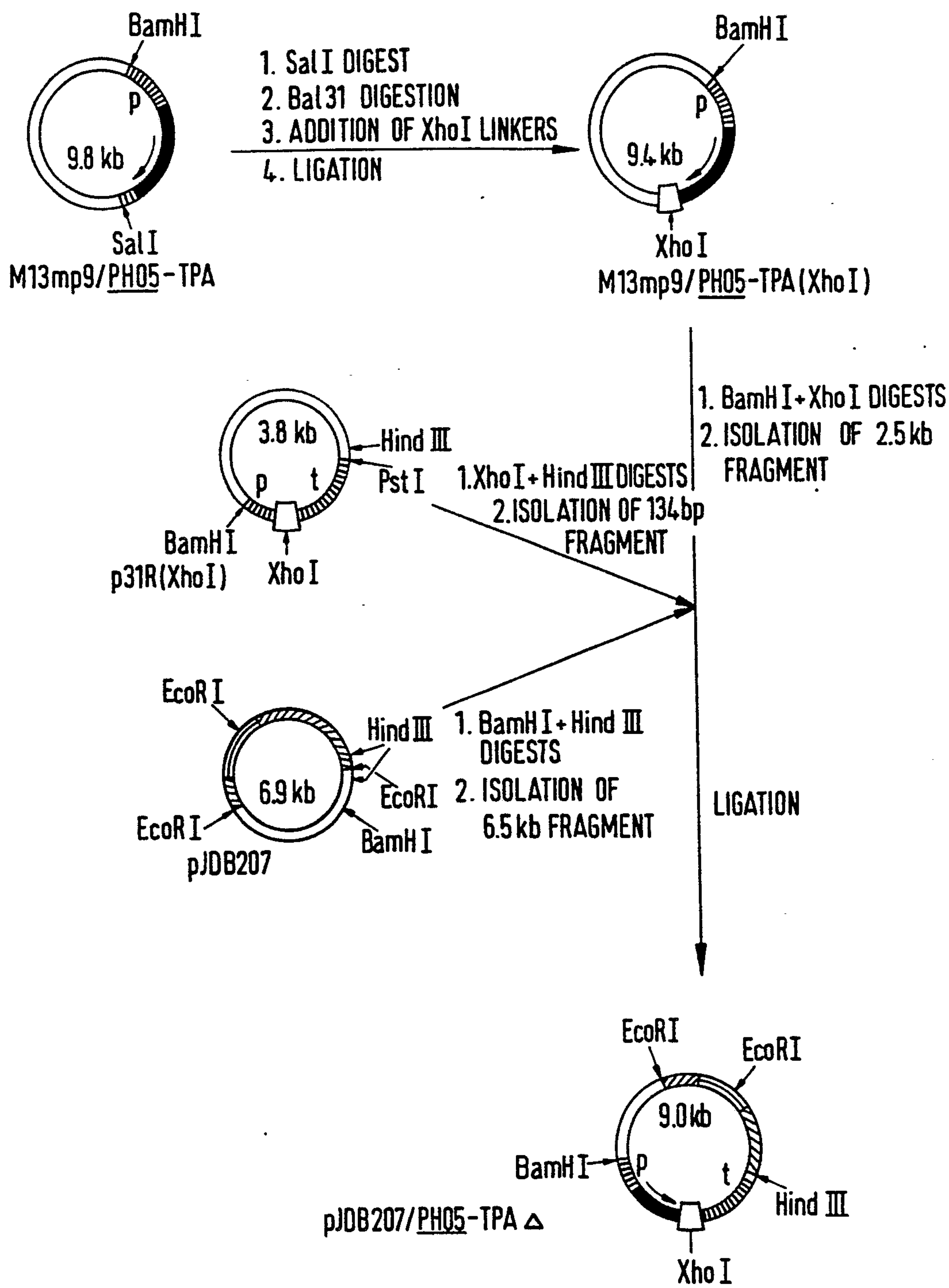

**Fig. 15**

Sequence of the shortened PHO5 transcription termination fragment

p31R sequence — CCTCGAGG GGAACACTACTCATTACAACGCCAGTCTATTGAGACAATAGTTTTGTATAACTAAATAATATTGGA

PHO5 TRANSCRIPTION TERMINATION FRAGMENT

AACTAAATACGAATACCCAAATTTTTTATCTAAATTT TGCCGAAAGATTAAAATCTGCA — p31R sequence

mRNA

PstI

Fig. 16 CONSTRUCTION OF PLASMID p31RL/TPA (12-2)

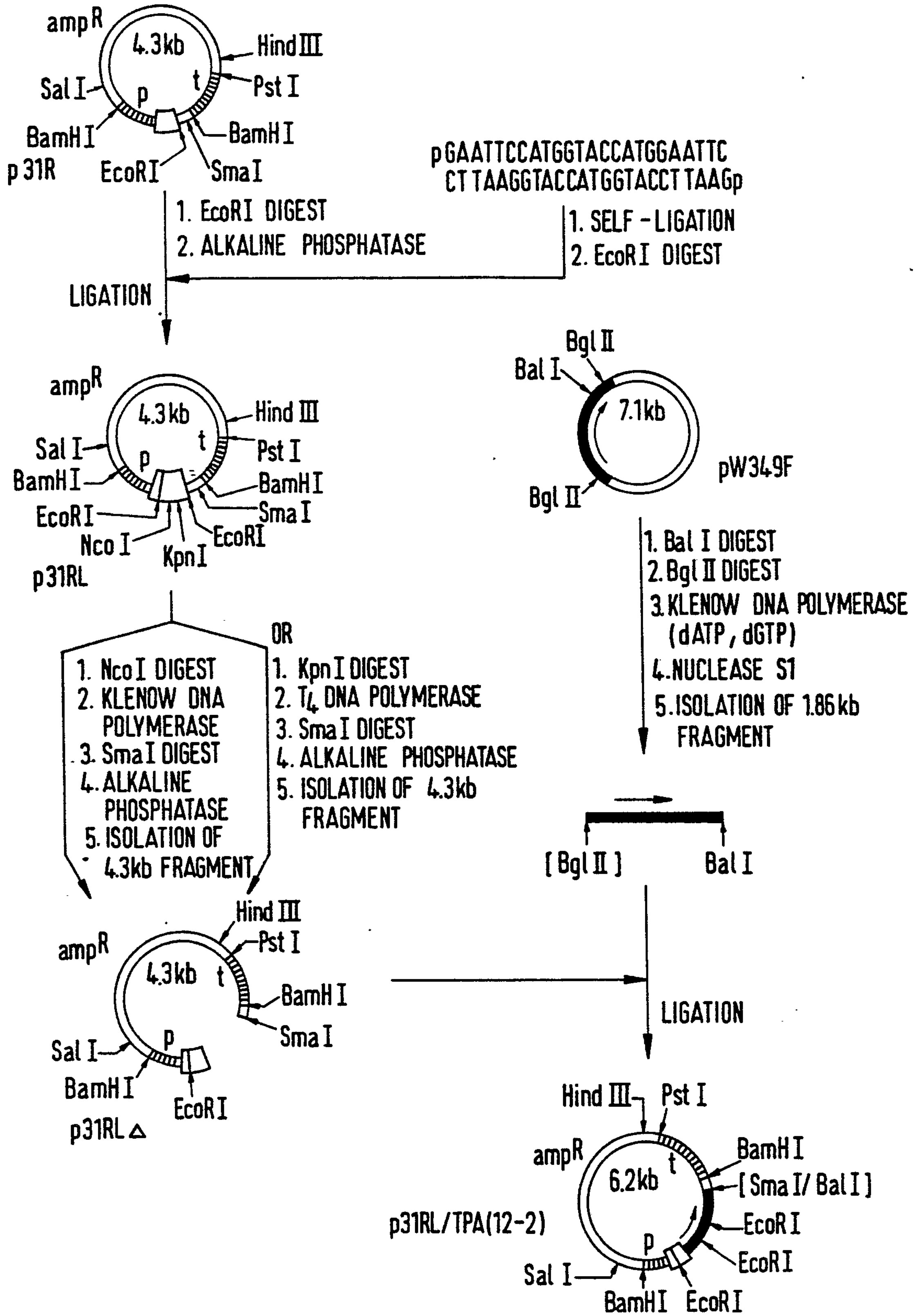

Fig. 17 CONSTRUCTION OF PLASMID pJDB207R/PHO5-TPA(12-2)

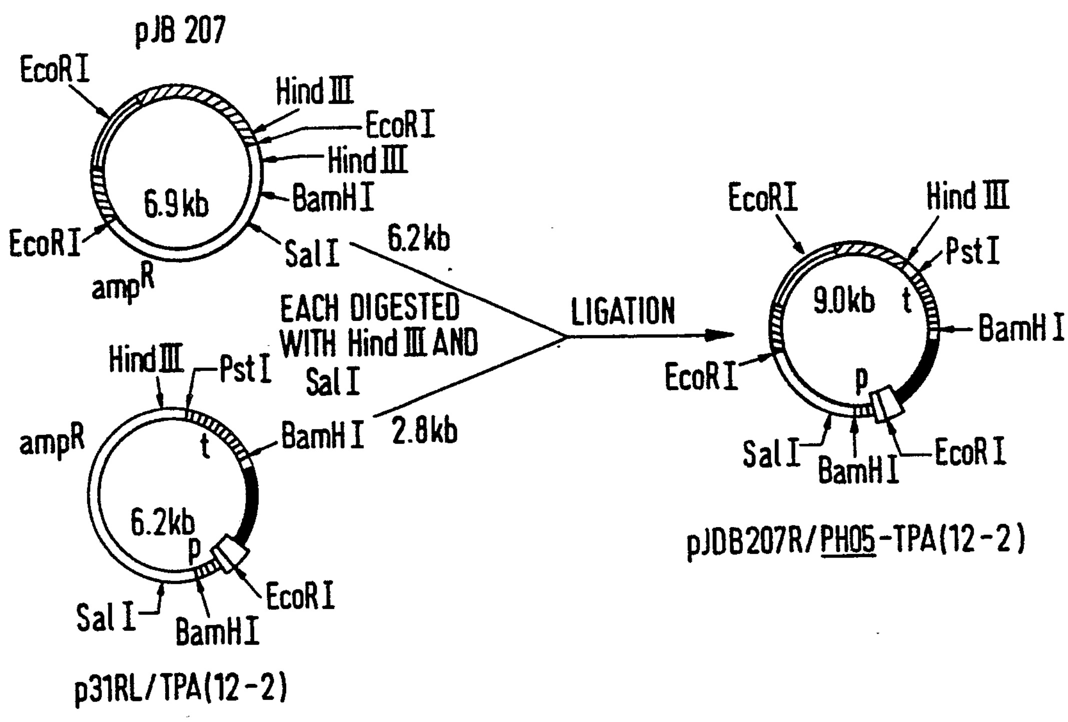

Fig. 18 CONSTRUCTION OF PLASMID p31/PH05-TPA18

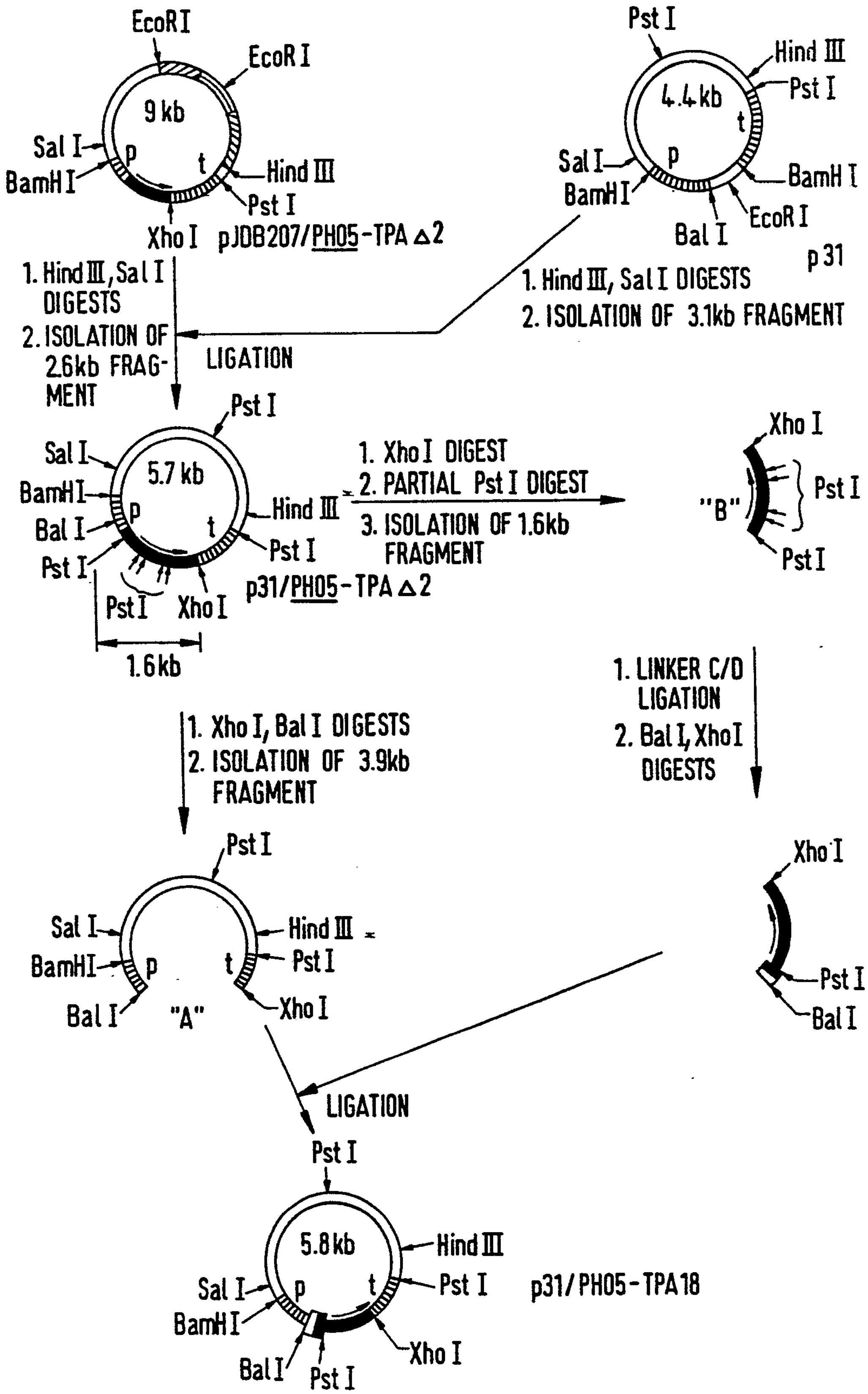

**Fig. 19** Junction between the PHO5 signal sequence and the coding region of mature TPA

Ball  Linker  PstI

5'-ATG TTT AAA TCT GTT GTT TAT TCA ATT TTA GCC GCT TCT TTG GCC AAT GCA TCT TAC CAA GTG ATC TGC AGA GAT GAA ...-3'
3'-TAC AAA TTT AGA CAA CAA ATA AGT TAA AAT CGG CGA AGA AAC CGG TTA CGT AGA ATG GTT CAC TAG ACG TCT CTA CTT ...-5'

MET PHE LYS SER VAL VAL TYR SER ILE LEU ALA ALA SER LEU ALA ASN ALA SER TYR GLN VAL ILE CYS ARG ASP GLU ...

← PHO5 signal peptide → ← mature TPA →

**Fig. 20** ISOLATION OF DNA FRAGMENTS CONTAINING PARTS OF THE PREPRO-TPA CODING REGION

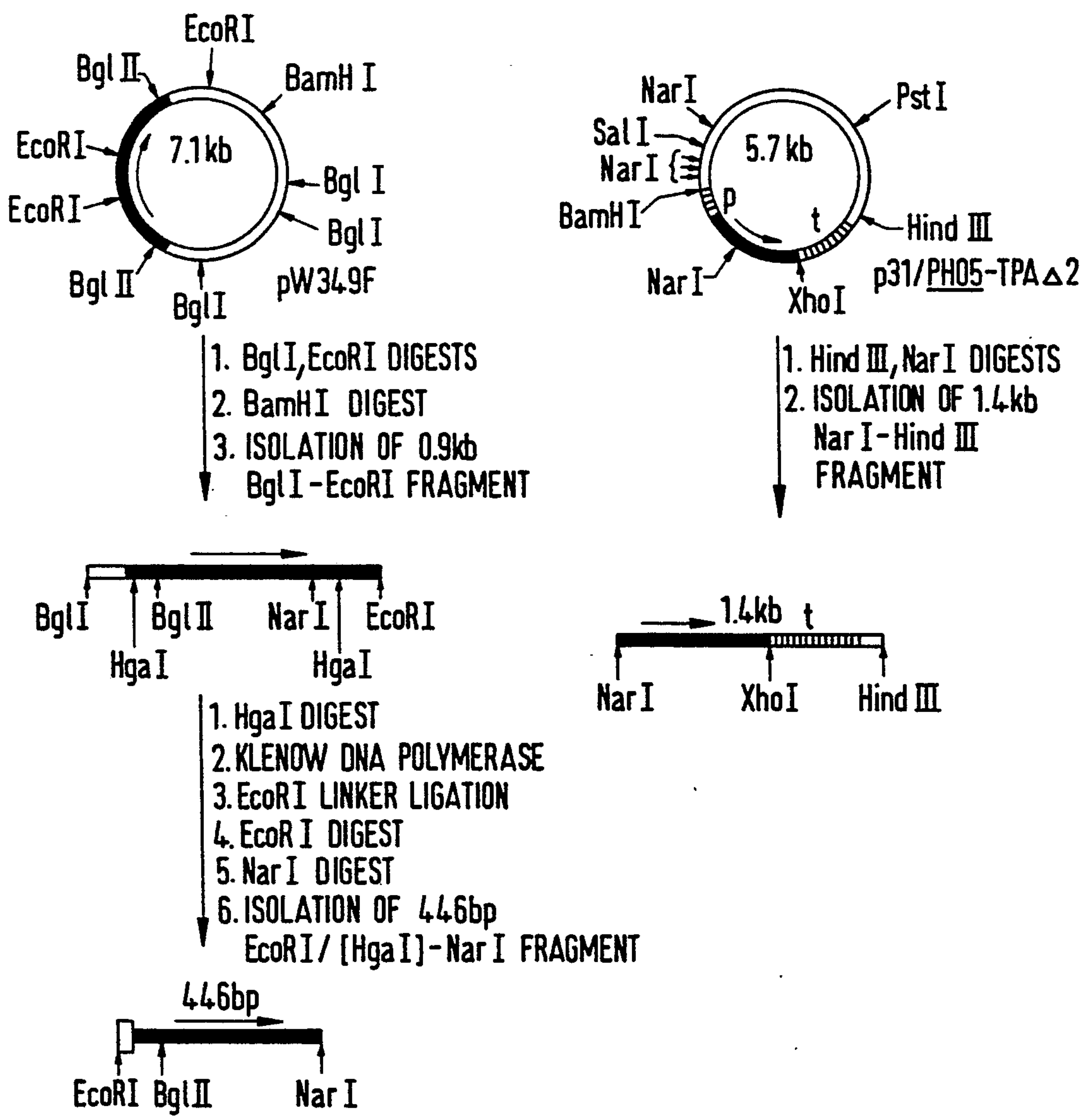

Fig. 21 PLASMID CONSTRUCTION INCLUDING THE PREPRO-SEQUENCE OF TPA

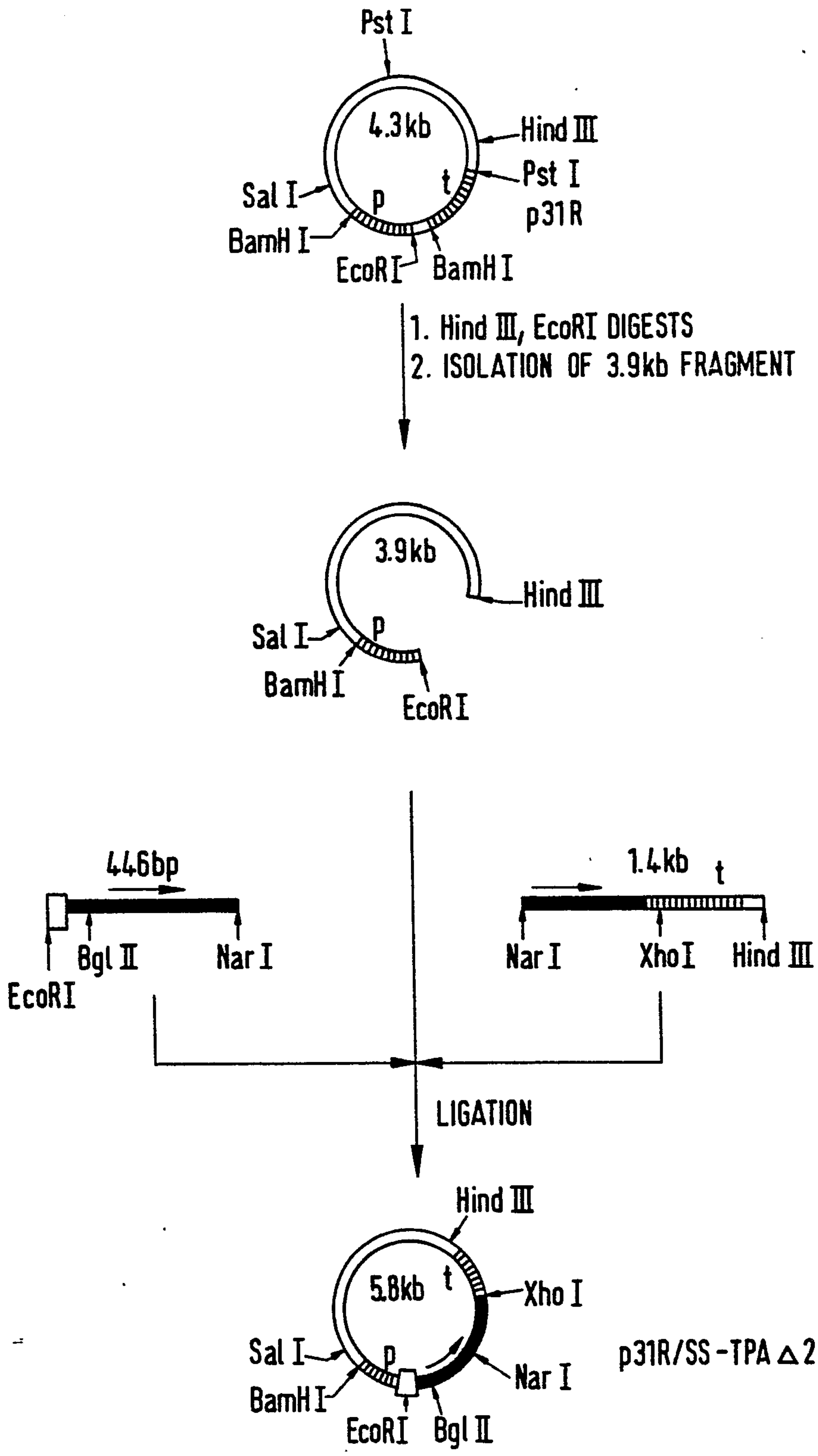

Fig. 22 CONSTRUCTION OF PLASMID pBR322/PHO5 Bam-Rsa 637

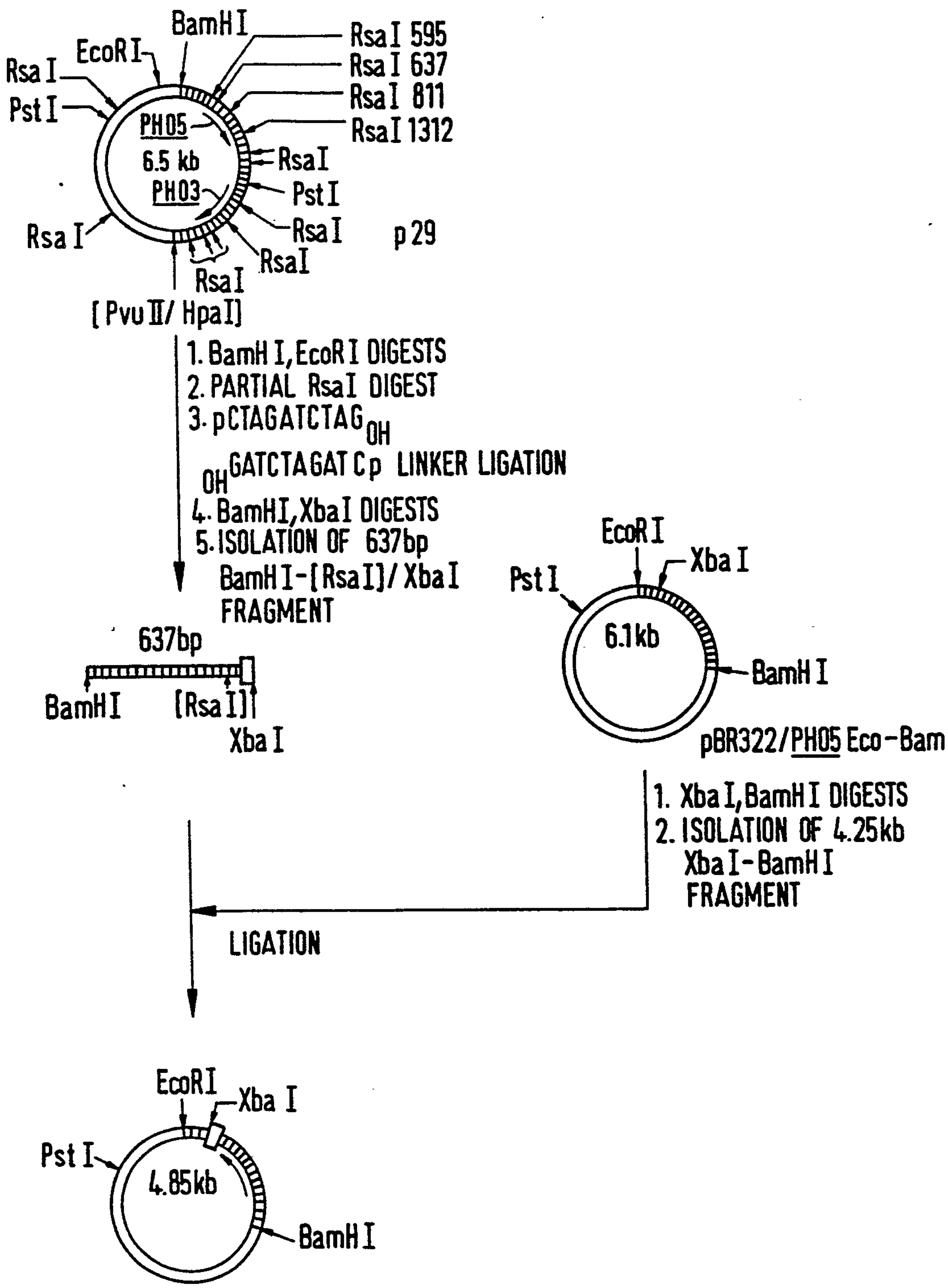


pBR322/PHO5 Bam-Rsa 637

Fig. 23 PLASMID CONSTRUCTIONS WITH THE PHO5 SIGNAL SEQUENCE EXTENDED INTO THE PHO5 CODING REGION

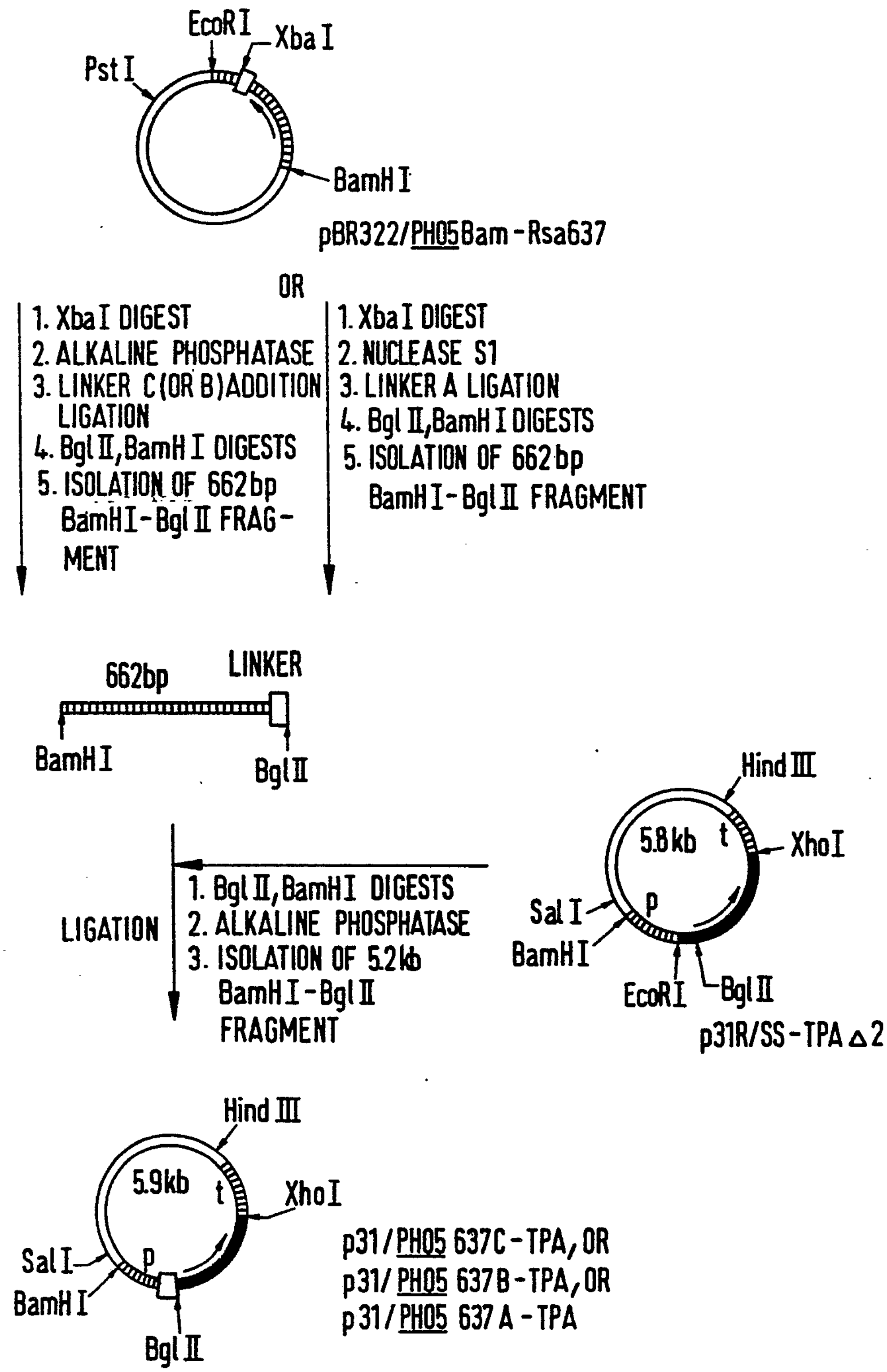

**Fig. 24** LINKAGE OF THE PHO5 PROMOTER AND PART OF THE PHO5 CODING SEQUENCE TO THE CODING SEQUENCE OF MATURE TPA

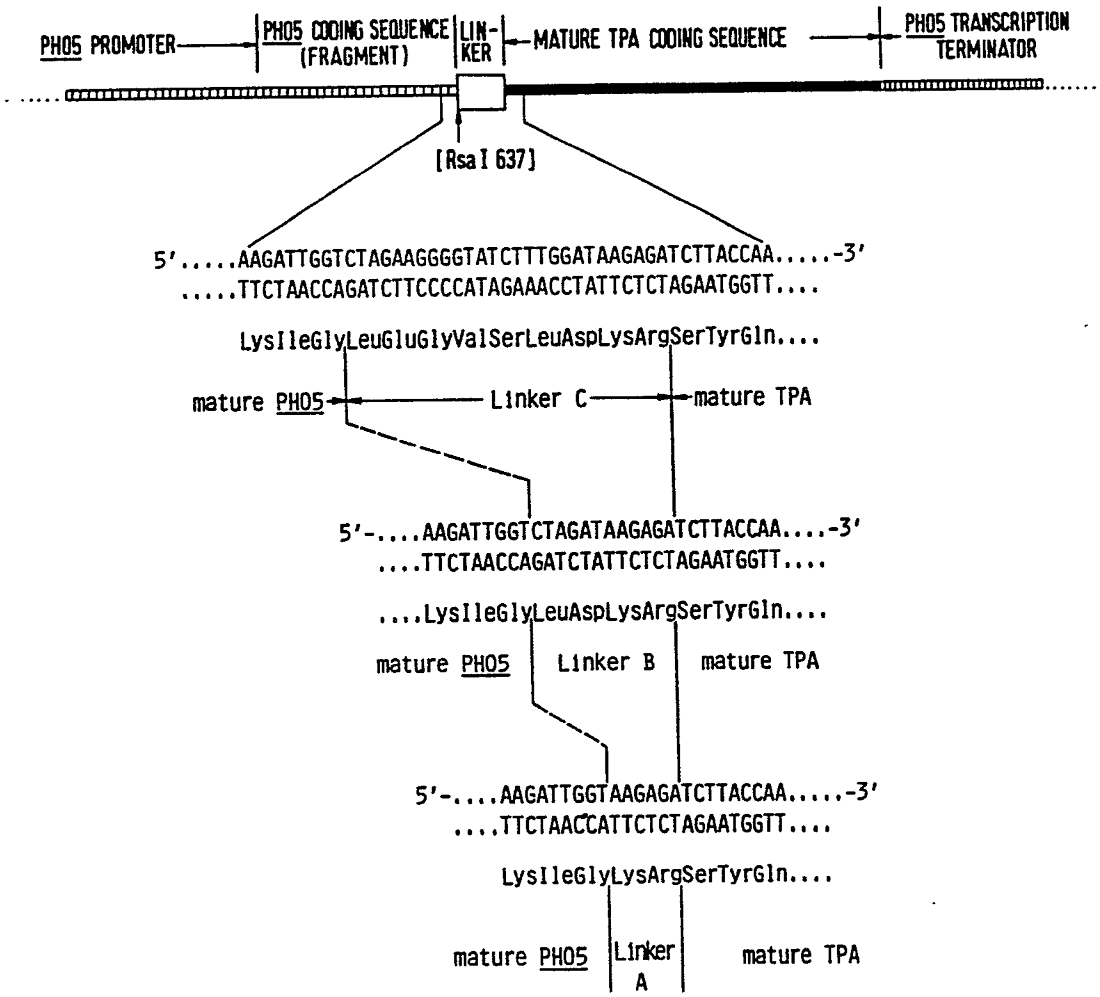

Fig. 25 CHROMOSOMAL REPLACEMENT OF THE PHO5 GENE BY THE GENE FOR TPA

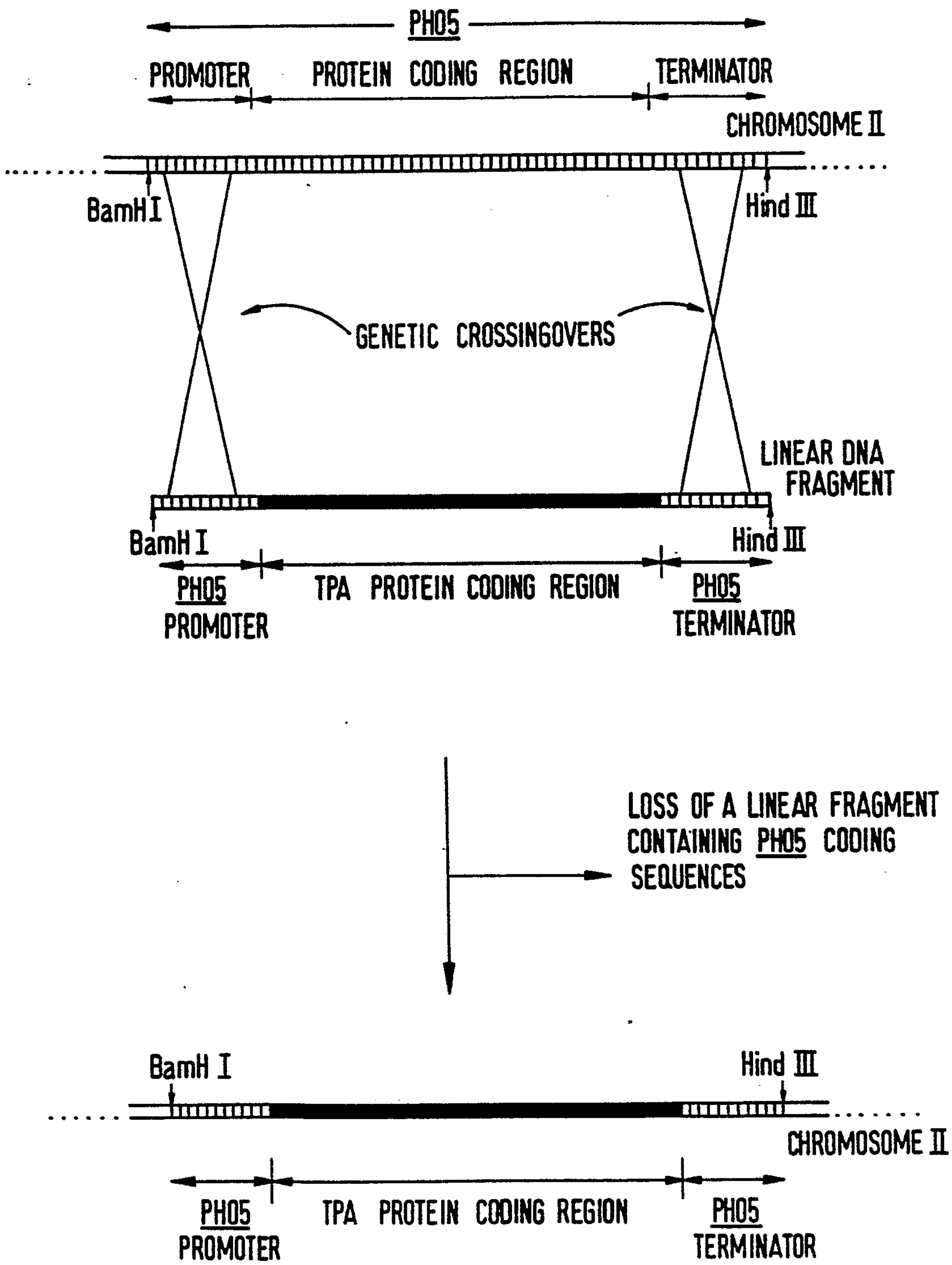

IN THE FIGURES OF THE ACCOMPANYING DRAWINGS, THE SYMBOLS USED HAVE THE FOLLOWING MEANINGS:

| Symbol | Meaning |
|---|---|
| | pBR 322 SEQUENCES |
| | YEAST CHROMOSOMAL DNA DERIVED FROM PHO3, PHO5 REGION |
| | JEAST 2μ PLASMID DNA |
| | JEAST CHROMOSOMAL DNA DERIVED FROM LEU 2 REGION |
| $amp^R$ | AMPICILLIN RESISTANCE GENE |
| $tet^R$ | TETRACYCLINE RESISTANCE GENE |
| p | PROMOTER REGION |
| t | TRANSCRIPTION TERMINATION REGION |
| | DELETION OF A RESTRICTION SITE |
| | TPA GENE |
| | DELETION IN pBR 322 |
| | RESTRICTION SITE |
| | DIRECTION OF TRANSCRIPTION |
| | LINKER DNA STRETCH |